(19) Europäisches Patentamt
European Patent Office
Office européen des brevets

(11) EP 4 079 738 A1

(12) EUROPEAN PATENT APPLICATION
published in accordance with Art. 153(4) EPC

(43) Date of publication:
**26.10.2022 Bulletin 2022/43**

(21) Application number: **20903427.1**

(22) Date of filing: **10.09.2020**

(51) International Patent Classification (IPC):
***C07F 9/564*** *(2006.01)* ***A61K 31/06*** *(2006.01)*
***A61K 31/664*** *(2006.01)* ***A61P 35/00*** *(2006.01)*

(52) Cooperative Patent Classification (CPC):
**A61K 31/06; A61K 31/664; A61P 35/00; C07F 9/564**

(86) International application number:
**PCT/CN2020/114519**

(87) International publication number:
**WO 2021/120717 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2019 CN 201911324466**

(71) Applicant: **Ascentawits Pharmaceuticals, Ltd.**
**Shenzhen, Guangdong 518118 (CN)**

(72) Inventors:
- **LI, Anrong**
  **Shenzhen, Guangdong 518118 (CN)**
- **DUAN, Jianxin**
  **Shenzhen, Guangdong 518118 (CN)**
- **MENG, Fanying**
  **Shenzhen, Guangdong 518118 (CN)**
- **CAI, Xiaohong**
  **Shenzhen, Guangdong 518118 (CN)**

(74) Representative: **Michalski Hüttermann & Partner Patentanwälte mbB**
**Kaistraße 16A**
**40221 Düsseldorf (DE)**

# (54) ANTICANCER COMPOUND AND MEDICAL USE THEREOF

(57) A compound of formula (I), or pharmaceutically acceptable salts, solvates, isotopic variants, or isomers thereof, and anticancer medical use are provided.

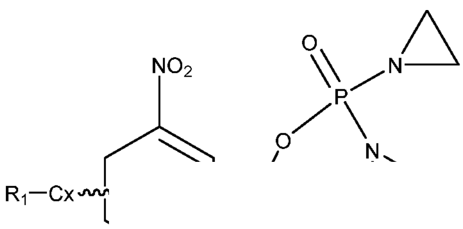

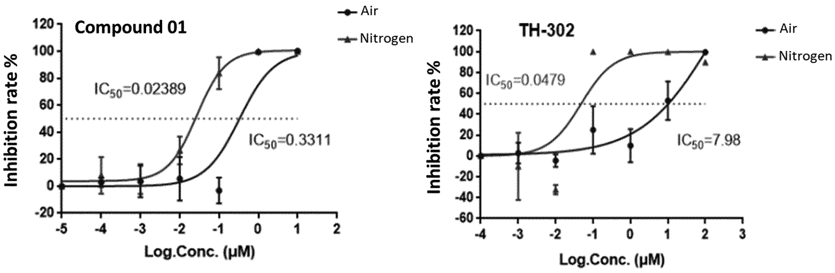


FIG. 1

EP 4 079 738 A1

## Description

## BACKGROUND OF THE INVENTION

### 1. Field of the Invention

The present disclosure relates to an anticancer compound and belongs to the field of anticancer drug research and development.

### 2. Description of the Related Art

With rapid growth of tumors, some tumor tissues become farther and farther away from the nearest blood vessels or produce abnormal tumor vessel structures, causing insufficient oxygen supply, and resulting in tumor hypoxia (referred to Nature review cancer, 2002, 2: 38-47). Traditional antitumor drugs have good killing effects on tumors near blood vessels, but have limited or no effect on tumors in hypoxic regions.

Hypoxia activation prodrug (HAP) can specifically release antitumor active compounds in the hypoxia regions of the tumor, so as to kill tumor cells in the hypoxia regions (referred to Chinese Journal of Cancer, 2014, 33: 80-86). Hypoxia activation prodrug has tumor-targeting properties, resulting in better safety. When used in combination with traditional anti-tumor chemotherapeutics or immunotherapy, the antitumor effect of hypoxia activation prodrug is even better.

TH-302 (Evofosfamide, CAS No. 918633-87-1) is a 2-nitroimidazole-induced hypoxia activation prodrug of which metabolite is bromoisophosphoramide (Br-IPM). Under hypoxia condition, an inactive TH-302 (shown in structural formula 1 below) prodrug can release highly toxic Br-IPM (shown in structural formula 2 below). TH-302 has a broad spectrum of in vitro and in vivo biological activities, specific hypoxia-selective priming activities, and activities of inducing histone H2AX phosphorylation and DNA crossing-linking, and thus cell cycle arrest. Therefore, the compound is used by many pharmaceutical companies and scientific research institutes for the development of anticancer drugs.

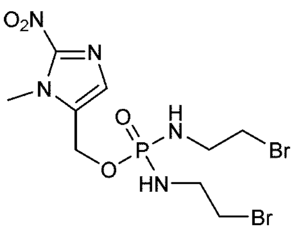

TH-302

Structural formula 1

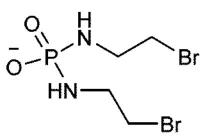

Br-IPM

Structural formula 2

The research article published by Fanying Meng et al of Threshold Pharmaceuticals Inc. (referred to Meng et al, Molecular and Cellular Pharmacology of the Hypoxia-Activated Prodrug TH-302, MCT, 2012(11): 740; DOI:10.1158/1535-7163.MCT-11-0634) pointed out that TH-302 has broad-spectrum activity against various tumors, and has a hypoxia-selective activity-enhancing effect. The enhanced cytotoxicity of TH-302 under hypoxia condition was observed in 32 human cancer cell lines, showing that the compound has selective enhance activity for cancer cells under hypoxia. Using cells overexpressing human NADPH confirmed the principle of enhanced activity of TH-302 under hypoxia condition, that is, single electron reductase dependence, as shown in the following reaction formula 1.

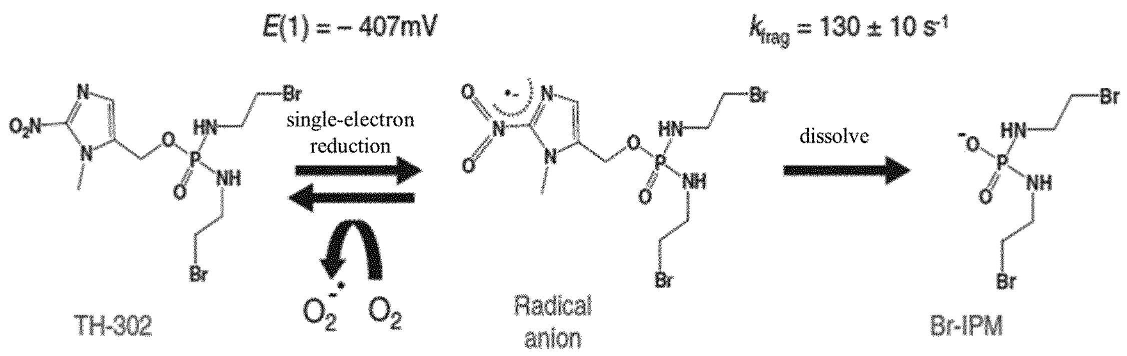


Reaction formula 1

After Merck Inc. purchased TH-302 related research projects from Threshold Pharmaceuticals Inc., Merck Inc. conducted a phase III clinical trial of the antitumor drug TH-302. The trial was to examine the drug's effects on soft tissue sarcoma and pancreatic cancer. However, in 2015, Merck Inc. announced that the trial had failed. Also, Threshold Pharmaceuticals Inc. indicated that the failure of the TH-302 clinical trial showed that the drug could not effectively target tumors in the long-term and become a stable antitumor drug.

Further, a team of the inventors of this application, Dr. Jian-Xin Duan and others, further improved TH-302. An amino phosphate moiety was modified to aziridine structure (referred to WO2016210175A1, CN108024974A, main compound structures disclosed below). After testing, it was found that this kind of compounds has identical hypoxia activation mechanism as TH-302. This kind of compounds has stronger cytotoxicity to cancer cells under hypoxia condition than under normoxic condition.

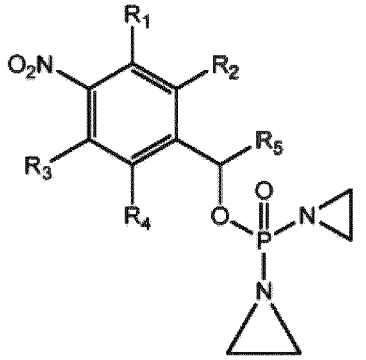

(I)

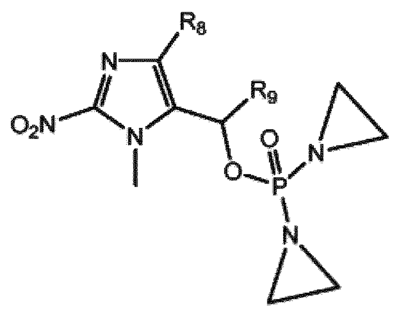

(II)

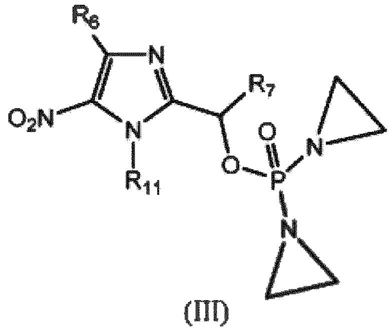

(III)

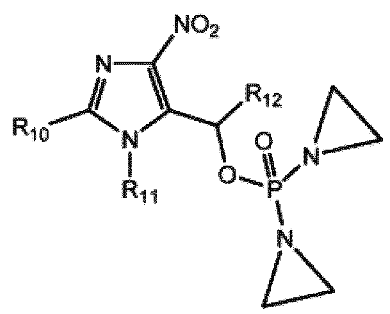

(IV)

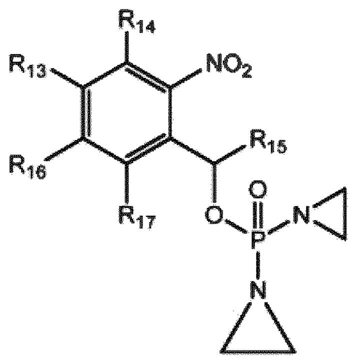

(V)

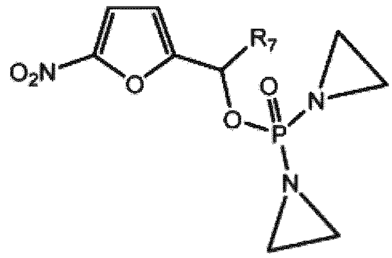

(VI)

The aziridine structure compounds obtained after structural modification of TH-302

With the same research and development idea, CN107513057A, CN107383136B both disclosed that compounds with similar structures of TH-302 may have hypoxia-activated anticancer activity, but they have not been further developed.

Although the above patents (WO2016210175A1, CN108024974A, CN107513057A, and CN107383136B) all disclosed a series of hypoxia-activated antitumor compounds, none of them have entered human clinical trials, indicating that these compounds are still insufficient as effective anticancer drugs in some aspects.

## SUMMARY

The purpose of the present disclosure is to provide candidate anticancer compounds with novel structures.

A first aspect of the present disclosure relates to a compound of formula (I), or pharmaceutically acceptable salts, solvates, isotopic variants, or isomers thereof:

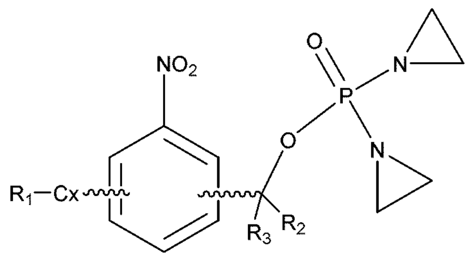

(I)

wherein

Cx is a 5- to 10-membered aryl ring or heteroaryl ring, a heteroaliphatic ring or a cycloalkane, and the Cx shares two carbons with the nitrobenzene ring to form a fused ring structure;

$R_1$ is attached to any skeleton atom of the Cx ring, and the $R_1$ is selected from a hydrogen, a halogen atom, a cyano or isocyano group, an hydroxyl group, an thiol group, an amine, an OTs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4- to 15-membered heterocycle or a Z-substituted heterocycle, a 5- to 15-membered heteroaryl group or a Z-substituted heteroaryl group, an alkoxyl group with 1-6 carbon atoms or a Z-substituted alkoxyl group with 1-6 carbon atoms, $-CONR^6R^7$, $-SO_2NR^6R^7$, $-SO_2R^6$, $-OCOO-R^6$, $-COOR^6$, $-NR^6COR^7$, $-OCOR^6$, $-NR^6SO_2R^7$, $-NR^6SO_2NR^6R^7$;

$R_2$, $R_3$ are each respectively a hydrogen, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4- to 15-membered heterocycle or a Z-substituted heterocycle, a 5- to 15-membered heteroaryl group or a Z-substituted heteroaryl group, or a 3- to 6-membered ring formed by $R_2$, $R_3$, and the bonded carbon atom at the benzylic position;

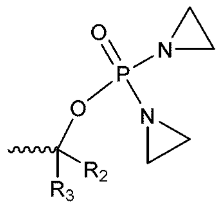

group replaces a hydrogen atom at any position on a carbon atom of the fused ring, and a number of substitutions is 1;

The Z-substituted group is a halogen atom, a cyano or isocyano group, a hydroxyl group, a thiol group, an amine group, a $C_1$-$C_3$ alkyl group or a substituted alkyl group, a $C_1$-$C_3$ alkoxyl group or a substituted alkoxyl group, a $C_2$-$C_3$ alkenyl group or a substituted alkenyl group, $C_2$-$C_3$ alkynyl group or a substituted alkynyl group, a $C_3$-$C_8$ cycloalkyl group or a substituted cycloalkyl group;

$R_6$, $R_7$ are each respectively a hydrogen, a $C_1$-$C_6$ alkyl group or a Z-substituted alkyl group, a $C_2$-$C_6$ alkenyl group or a $C_2$-$C_6$ Z-substituted alkenyl group, a $C_2$-$C_6$ alkynyl group or a Z-substituted alkynyl group, $C_3$-$C_8$ cycloalkyl group or a Z-substituted cycloalkyl group, a $C_6$-$C_{10}$ aryl group or a $C_6$-$C_{10}$ Z-substituted aryl group, a 4- to 15-membered heterocylic group or a Z-substituted 4- to 15-membered heterocyclic group, 5- to 15-membered heteroaryl group or a Z-substituted 5- to 15-membered heteroaryl group, or a 5- to 7-membered heterocyclic group or a Z-substituted 5- to 7-membered heterocyclic group formed by $R_6$, $R_7$, and an atom bonded thereto.

The pharmaceutically acceptable salts of the compound of the present disclosure are basic or acid salts. The salts may be the basic salts, comprising salts of the compound with inorganic bases (such as alkali hydroxide, alkali-earth hydroxide, etc.) or with organic bases (such as monoethanolamine, diethanolamine, triethanolamine, etc.). Alternatively, the salts may be the acid salts, comprising salts of the compound with inorganic acids (such as hydrochloric acid, hyhrobromic acid, hydroiodic acid, nitric acid, perchloric acid, sulfuric acid, or phosphoric acid, etc.) or with organic acids (such as methanesulfonic acid, trifluoromethanesulfonic acid, ethanesulfonic acid, benzenesulfonic acid, para-toluenesulfonic acid, fumaric acid, oxalic acid, maleic acid, or citric acid, etc.).

In a preferred embodiment, the solvates of the compound of the present disclosure are hydrates or alcoholates. Besides, the alcoholates comprises ethanolates.

A typical substitution mode of the isotopic variants of the compound of the present disclosure is that a hydrogen atom (H) is substituted by a heavy hydrogen atom deuterium (D).

Specially, a position substituted by deuterium is located on Ph-C*- of the compound of formula (I), as shown in the following formula.

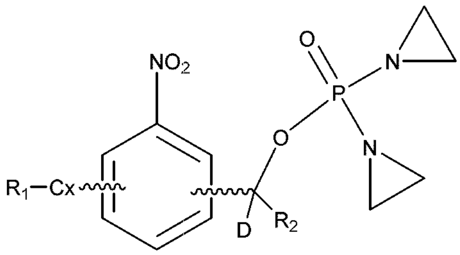

The compound of the present disclosure may also comprise enantiomers, non-enantiomers, and geometric isomers except the enantiomers of the compound, as well as a mixture of enantiomers, non-enantiomers, and geometric isomers except the enantiomers.

Methods of selecting and preparing the pharmaceutically acceptably salts, the solvates, the isotopic variants, or the isomers of the compound are well known in the art.

In a preferred embodiment, in the compound of formula (I):

The Cx is a 5-, 6-, 8-membered aryl ring. Alternatively, the Cx is a 5-, 6-, 7-, 8-membered heteroaryl ring or aliphatic heterocycle containing N, O, S atoms. Alternatively, the Cx is a 5-, 6-, 7-, or 8-membered aliphatic ring.

In a further preferred embodiment, it is a compound of formula (II):

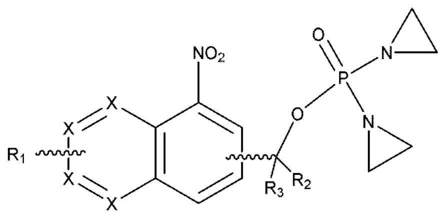

(II)

wherein
$R_1$ substitute a hydrogen atom at any position on carbon atom of a fusedring, and a member of the substituent $R_1$ is 1, 2, 3, 4, 5, or 6. Additionally, X is C or N.

In further preferred embodiments, only one or two of the four X atoms are N atoms in the compound of formula (II).

In further preferred embodiments, in the compound of formula (II), $R_1$ is a hydrogen, a halogen atom, the $C_1$-$C_6$ alkyl group or the Z-substituted alkyl group, the $C_3$-$C_8$ cycloalkyl group or the Z-substituted cycloalkyl group, the $C_6$-$C_{10}$ aryl group or the Z-substituted aryl group, the 4- to 15-membered heterocycle or the Z-substituted heterocycle, the 5- to 15-membered heteroaryl group or the Z-substituted heteroaryl; or

$R_2$, $R_3$ are each respectively the hydrogen, the $C_1$-$C_6$ alkyl group or the Z-substituted alkyl group, the $C_3$-$C_8$ cycloalkyl group or the Z-substituted cycloalkyl group, the $C_6$-$C_{10}$ aryl group or the Z-substituted aryl group, the 4- to 15-membered heterocycle or the Z-substituted heterocycle, the 5- to 15-membered heteroaryl group or the Z-substituted heteroaryl group.

In further preferred embodiments, in the compound of formula (II), $R_1$ is the hydrogen, the $C_1$-$C_6$ alkyl group or a halogen substituted $C_1$-$C_6$ alkyl group, the $C_3$-$C_8$ cycloalkyl group or a halogen substituted $C_3$-$C_8$ cycloalkyl group, the $C_6$-$C_{10}$ aryl group or a halogen substituted $C_6$-$C_{10}$ aryl group; or

$R_2$, $R_3$ are each respectively the hydrogen, the $C_1$-$C_6$ alkyl group or a halogen substituted $C_1$-$C_6$ alkyl group, the $C_3$-$C_8$ cycloalkyl group or a halogen substituted $C_3$-$C_8$ cycloalkyl group, the $C_6$-$C_{10}$ aryl group or a halogen substituted $C_6$-$C_{10}$ aryl group.

In further preferred embodiments, in the compound of formula (II), $R_1$ is H, - $CH_3$, $-CF_3$. Also, $R_2$, $R_3$ are respectively H, D, $-CH_3$, $-CF_3$. More preferably, X is C, $R_1$, $R_2$ are H, and $R_3$ is H, D or $-CF_3$.

In a preferred embodiment, Z-substituted group is the halogen in the compound of formula (I).

In further preferred embodiments, the compound is a compound with the following structures:

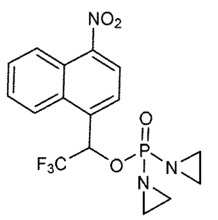,
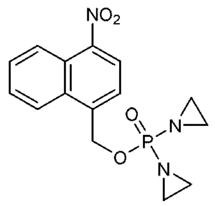,
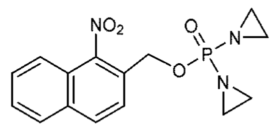,
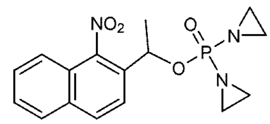,

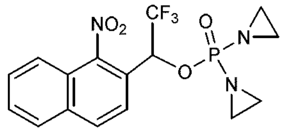,
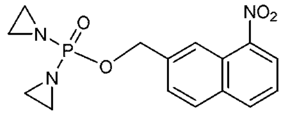,
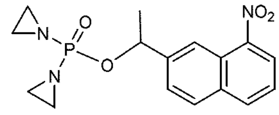,

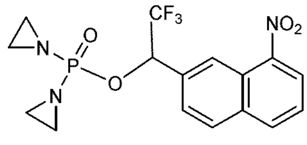,
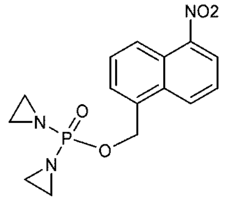,
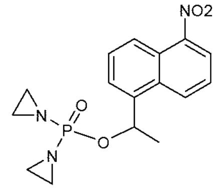,
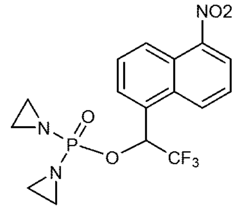,

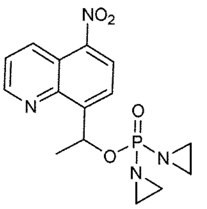,
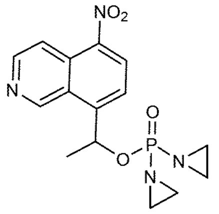,
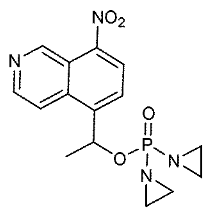,
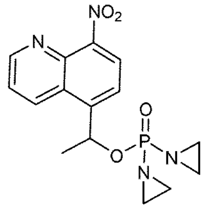,
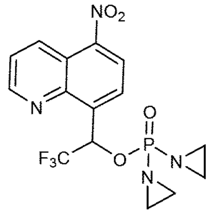,
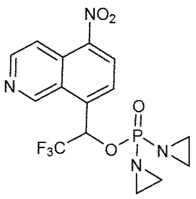,
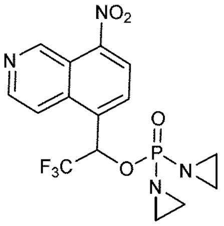,
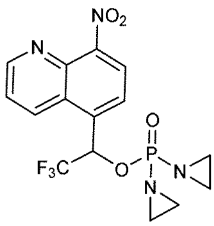,
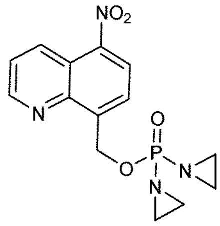,
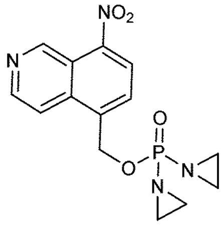,
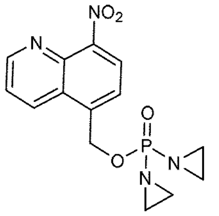,
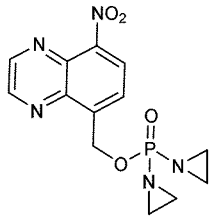,
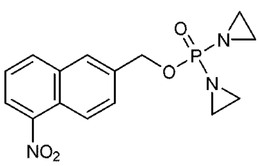,
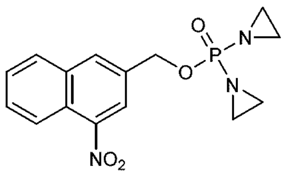,
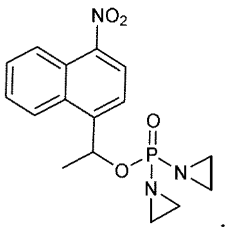.

A second aspect of the present disclosure relates to a medical use of the compound, or the pharmaceutically acceptable salts, the solvates, the isotopic variants, or the isomer thereof according to the first aspect of the present disclosure.

The present disclosure provides a drug or a formulation containing the preceding compound, or the pharmaceutical acceptable salts, the solvates, the isotopic variants, or the isomers thereof.

The present disclosure also provides a use of the preceding drug and preparation for treating a tumor, a cancer, or a cell proliferative disease.

The present disclosure also provides a use of the preceding compound, or the pharmaceutical acceptable salts, the solvates, the isotopic variants, or the isomers thereof in a preparation of a drug for treating of tumors, cancers or cell proliferative diseases.

In preferred embodiments, the tumor or the cancer comprise:

lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous-cell cancer , basal cell carcinoma, adenocarcinoma, squamous-cell carcinoma, sebaceous carcinoma, papillary carcinoma, papillary adenocarcinoma, cystadenocarcinoma, cystic carcinoma, medullary carcinoma, bronchial carcinoma, bone cell carcinoma, epithelial carcinoma, cholangiocarcinoma, choriocarcinoma, embryonic carcinoma, seminoma, Wilms' carcinoma, glioblastoma, astrocytoma, medulloblastoma, craniopharyngioma, ependymoma, pineal tumor, hemoblastoma, neurogenic tumor of the larynx, meningiomas, neuroblastoma of optic nerve, neuroblastomas, retinoblastomas, neurofibromas, fibroma sarcomatosum, fibroblastomas, fibroma, fibroadenomas, fibrochondromas, fibrocystic tumors, fibrous myxoma, osterfibroma, myxofibrosarcoma, fibropapillary, myxofibrosarcoma, bursal tumor, myxonchondroma, myxonchondrosarcoma, myxonchondrosarcoma, myxedema, myxoblastoma, liposarcoma, lipoma , lipoadenoma, lipoblastoma, lipochondroma, lipofibroma, lipoangioma, myxolipoma, chondrosarcoma, chondroma, chondromyoma, chordoma, chorioadenoma, chorioepithelioma, chorioblastoma, osteosarcoma, osteoblastoma, osteochondrofibroma, osteochondrosarcoma, osteochondroma, osteocystoma, cementoma, osteofibroma, fibrosarcoma, angiosarcoma, hemangioma, angiolipoma, angiochondroma, hemangioblastoma, angiokeratoma, angioglioma, hemangioendothelioma, angiofibroma, angiomyoma, angiolipoma, angiolymphoma, angiolipiomyoma, angiomyolipomas, angiomyoneuroma, angiomyxoma, angioreticuloendothelioma, lymphangiosarcoma, lymphogranuloma, lymphangioma, lymphoma, lymphomyxoma, lymphosarcoma, lymphangial fibrom, lymphocytoma, lymphoepithelioma, lymphoblastoma, endothelioma, endothelioblastoma, synovialoma, synovial sarcoma, mesothelioma, desmoplastic tumor, Ewing's tumor, leiomyoma, leiomyosarcoma, leioblastoma, leiomyofibroma, rhabdomyoma, rhabdomyosarcoma, rhabdomyomatous myxoma, acute lymphoblastic leukemia, acute myeloid leukemia, chronic disease cells, polycythemia, endometrial cancer, glioma, colorectal cancer, thyroid cancer, urothelial cancer, or multiple myeloma.

In a preferred embodiment, the tumor, the cancer are selected from non-small cell lung cancer, pancreatic cancer, breast cancer or prostate cancer. Further, the breast cancer is selected from triple-negative breast cancer.

The triple-negative breast cancer refers to breast cancer patients with negative estrogen receptor, progesterone receptor, and human epidermal growth factor receptor 2. The prognosis of triple-negative breast cancer has little relationship with tumor size and lymph node status. The recurrence of triple-negative breast cancer is relatively rapid, with a peak recurrence in 1 to 3 years and a high incidence of brain metastases.

On the other hand, as a preference, the cancer or the tumor is brain cancers, brain tumors, or metastatic cancers or tumors that metastasize to brains.

The present disclosure also provides a use of the preceding compound, or the pharmaceutical acceptable salts, the solvates, the isotopic variants, or the isomers thereof in preparing of a drug for treating the tumors and the cancers in patients with damaged DNA repair. Additionally, the damaged DNA repair is damaged by homologous recombination repair enzymes and nucleotide excision repair enzymes. That is, the preceding compound, or the pharmaceutical acceptable salts, the solvates, the isotopic variants, or the isomers thereof may be prepared as the drug for treating tumors and cancers in patients with the damaged DNA repair. This damaged DNA repair is limited to damage to the homologous recombination repair enzymes and damage to the nucleotide excision repair enzymes.

Generally, the damaged DNA repair comprises:

the homologous recombination repair enzymes,
the nucleotide excision repair enzymes,
nonhomologous end joining enzymes,
base excision repair enzymes,
mismatch repair enzymes,
fanconi anemia pathway repair enzymes, etc. are damaged. The preceding compound, or the pharmaceutical acceptable salts, the solvates, the isotopic variants, or the isomers thereof may be confirmed by experiments that have better effects on the above-mentioned two kinds of damaged cancer patients in the damaged DNA repair.

The present disclosure further provides a compound drug, which comprises the preceding compound, or the pharmaceutical acceptable salts, the solvates, the isotopic variants, or the isomers thereof, and

a. a traditional chemotherapy drug;
b. an anti-angiogenic drug;
c. a cellular checkpoint inhibitor; or
d. an immunosuppressant.

Based on the fact that the compound of the present disclosure has the same mechanism of action as the hypoxia-activated classic drug, TH-302: after hypoxia activation, the compound is metabolized to release cytotoxic DNA alkylating agents. Thus, the compound, or the pharmaceutical acceptable salts, the solvates, the isotopic variants, or the isomer thereof in the present disclosure may enhance the anticancer effect through the mechanism of combined use of four anticancer drugs.

As a result, the present disclosure further provides combination therapies as follow.

The combination therapy for treating the cancers or the tumors, which comprises administering the compound, or the pharmaceutical acceptable salts, the solvates, the isotopic variants, or the isomers thereof, and administering the traditional chemotherapy drug to the patients.

The combination therapy for treating the cancers or the tumors, which comprises administering the compound, or the pharmaceutical acceptable salts, the solvates, the isotopic variants, or the isomers thereof, and administering the anti-angiogenic drug to the patients.

The combination therapy for treating the cancers or the tumors, which comprises administering the compound, or the pharmaceutical acceptable salts, the solvates, the isotopic variants, or the isomers thereof, and administering the

cellular checkpoint inhibitor to the patients.

The combination therapy for treating the cancers or the tumors, which comprises administering the compound, or the pharmaceutical acceptable salts, the solvates, the isotopic variants, or the isomers thereof, and administering the immunosuppressant to the patients

Specifically, the four above combination therapies are described below.

The first is to be combined with the traditional chemotherapy drug. The traditional chemotherapy drug can only kill tumor cells under normoxia but are ineffective against hypoxia cells. The traditional chemotherapy drugs:

Docetaxel (Vanhoefer U, Cao S, Harstrick A, Seeber S, Rustum YM (1997) Comparative antitumor efficacy of docetaxel and paclitaxel in nude mice bearing human tumor xenografts that overexpress the multidrug resistance protein (MRP).Ann Oncol 8: 1221-1228),

Cisplatin (Bigioni M, Benzo A,Irrissuto C, Lopez G, Curatella B, Maggi CA, Manzini S, Crea A, Caroli S, Cubadda F, Binaschi M (2008) Antitumour effect of combination treatment with Sabarubicin (MEN 10755) and cis-platin (DDP) in human lung tumour xenograft. Cancer Chemother Pharmacol 62: 621-629),

Pemetrexed (Huber PE, Bischof M, Jenne J, Heiland S, Peschke P, Saffrich R, Grone HJ, Debus J, Lipson KE, Abdollahi A (2005) Trimodal cancer treatment: beneficial effects of combined antiangiogenesis, radiation, and chemotherapy. Cancer Res 65: 3643-3655),

Irinotecan (Houghton JA, Cheshire PJ, Hallman JD, 2nd, Lutz L, Luo X, Li Y, Houghton PJ (1996) Evaluation of irinotecan in combination with 5-fluorouracil or etoposide in xenograft models of colon adenocarcinoma and rhabdomyosarcoma. Clin Cancer Res 2: 107-118),

Doxorubicin (Kraus-Berthier L, Guilbaud N, Jan M, Saint-Dizier D, Rouillon MH, Burbridge MF, Pierre A, Atassi G (1997) Experimental antitumour activity of S 16020-2 in a panel of human tumours. Eur J Cancer 33: 1881-1887),

Gemcitabine (Merriman RL, Hertel LW, Schultz RM, Houghton PJ, Houghton JA, Rutherford PG, Tanzer LR, Boder GB, Grindey GB (1996) Comparison of the antitumor activity of gemcitabine and ara-C in a panel of human breast, colon, lung and pancreatic xenograft models. Invest New Drugs 14: 243-247; Teicher BA, Chen V, Shih C, Menon K, Forler PA, Phares VG, Amsrud T (2000) Treatment regimens including the multitargeted antifolate LY231514 in human tumor xenografts. Clin Cancer Res 6: 1016-1023),

Temozolomide (Middleton MR, Thatcher N, McMurry TB, McElhinney RS, Donnelly DJ, Margison GP (2002) Effect of O6-(4-bromothenyl)guanine on different temozolomide schedules in a human melanoma xenograft model. Int J Cancer 100: 615-617).

The combination of the preceding traditional chemotherapy drugs and hypoxia-activated compounds can kill all tumor cells (Liu et al · TH-302, a hypoxia-activated prodrug with broad in vivo preclinical combination therapy efficacy: optimization of dosing regimens and schedules, Cancer Chemother Pharmacol, 2012, 69:1487-1498, DOI: 10.1007/s00280-012-1852-8).

The second is to be combined with the anti-angiogenic drug to increase the hypoxia region of tumor cells, resulting in increasing the number of tumor cells sensitive to hypoxia compounds (Chang et al · Sorafenib (BAY43-9006) inhibits tumor growth and vascularization and induces tumor apoptosis and hypoxia in RCC xenograft models · Cancer Chemother Pharmacol, 2007, 59(5): 561-74.DOI: 10.1007/s00280-006-0393-4). The experimentally proven anti-angiogenic drugs comprise sorafenib (Chang et al · Sorafenib(BAY43-9006) inhibits tumor growth and vascularization and induces tumor apoptosis and hypoxia in RCC xenograft models · Cancer Chemother Pharmacol ’ 2007 ’ 59(5):561-74.DOI : 10.1007/s00280-006-0393-4), rapamycin (Su D, Stamatakis L, Singer EA, Srinivasan R.Renal cell carcinoma: molecular biology and targeted therapy. Curr Opin Oncol 2014(26) : 321-7), mTOR inhibitor (Sun et al Combination treatment with hypoxia-activated prodrug evofosfamide (TH-302) and mTOR inhibitors results in enhanced antitumor efficacy in preclinical renal cell carcinoma models, Am J Cancer Res. 2015(5):2139), and so on. The combination of these drugs and the hypoxia compounds or drug combinations of the present disclosure may lead to the increase of hypoxia region of tumor cells, so as to increase the number of tumor cells sensitive to the hypoxia compound, and thus enabling the combined drugs have a synergistic anticancer effect.

The third is to be combined with the cell checkpoint inhibitor to increase the sensitivity of tumor cells to the DNA alkylating agent (Meng et al ,· Enhancement of hypoxia-activated prodrug TH-302 anti-tumor activity by Chk1 inhibition ,· BMC Cancer ’ 2015 ’ 15:422 ,· DOI : 10.1186/s12885-015-1387-6). The experimentally proven cell check point drugs comprise LY2603618 (Wang F Z, Fei H R, Cui, Y J et al. The checkpoint 1 kinase inhibitor LY2603618 induces cell cycle arrest, DNA damage response and autophagy in cancer cells. Apoptosis, 2014(19):1389-1398), PF477736 (Balsina et al Breaching the DNA damage checkpoint via PF-00477736, a novel small-molecule inhibitor of checkpoint kinase, Mol Cancer Ther 2008;7(8):2394-404), AZD7762 (Morgan et al; Mechanism of radiosensitization by the Chk1/2 inhibitor AZD7762 involves abrogation of the G2 checkpoint and inhibition of homologous recombinational DNA repair, Cancer Researh 2010(70):4972), and so on. The combination of these drugs and the hypoxia compounds or drug combinations of the present disclosure may lead to increasing the sensitivity of tumor cells to the DNA alkylating agents, and thus enabling the combined drugs have a synergistic anticancer effect.

The fourth is to be combined with the immunosuppressant. The hypoxia compound may reduce the hypoxia

region of tumors, so as to increase the infiltration of lymphocytes in the hypoxia region and improving the effect of immunotherapy (Jayaprakash et al, Targeted hypoxia reduction restores T cell infiltration and sensitizes prostate cancer to immunotherapy, JCI, 2018 (128): 5137, DOI: 10.1172/JCI96268.). The experimentally proven immunosuppressant comprises αCTLA-4/αPD-1 (Curran et al. PD-1 and CTLA-4 combination blockade expands infiltrating T cells and reduces regulatory T and myeloid cells within B16 melanoma tumors. Proc Natl Acad Sci USA.2010, 107(9): 4275-4280). The combination of the drugs and the hypoxia compounds or drug combinations of present disclosure may increase the infiltration of lymphocytes in the hypoxia region, thereby enhancing the effect of immunotherapy.

The drugs or formulations disclosed herein may certainly comprise pharmaceutically acceptable carriers or excipients. The drugs may be any dosage form for clinical administration, such as tablets, suppositories, dispersible tablets, enteric-coated tablets, chewable tablets, orally disintegrating tablets, capsules, sugar-coated formulations, granules, dry powder, oral solutions, small-volume injections, lyophilized powder for injection, or infusion solution. According to the specific dosage forms and the modes of administration, the pharmaceutically acceptable carriers or excipients in the drug may contain one or more diluents, solubilizers, disintegrants, suspending agents, lubricants, binders, fillers, flavors, sweeteners, antioxidants, surfactants, preservatives, coatings, pigments and so on.

A third aspect of the present disclosure relates to a method for preparing the compound, or the pharmaceutical acceptable salts, the solvates, the isotopic variants, or the isomers thereof in the present disclosure.

The method may be carried out by a first scheme, comprising the following steps.

The compound of formula (II) is provided by undergoing a condensation reaction of a compound A to close a ring.

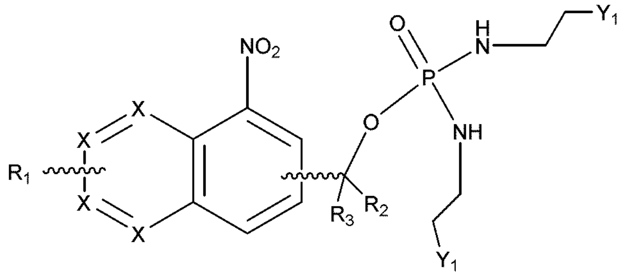

Compound A

Alternatively,

The method may be carried out by a second scheme, comprising the following steps.

The compound of formula (II) is provided by reacting a compound B with $R_1H$.

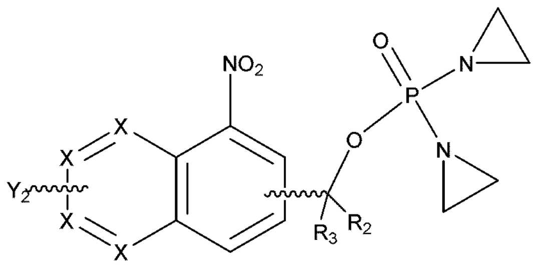

Compound B

Besides, $Y_1$ and $Y_2$ are both leaving groups, and preferably, $Y_1$ and $Y_2$ are independently Cl, Br, I, -OTs, $-ONO_2$, -OMs, $-OT_f$, or $-OSO_2Cl$. More preferably, $Y_1$ is Br in the compound A, and $Y_2$ is F in the compound B.

In addition,

-OTs is 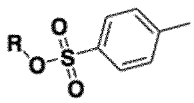 ; -OMs is 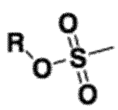 ; $-OT_f$ is 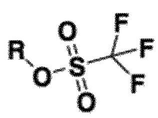 .

In a preferred embodiment, in the first scheme, DIPEA or TEA is used as an anti-acid agent, and silver oxide

($Ag_2O$) or sliver nitrate ($AgNO_3$) is used as a catalyst.

In a preferred embodiment, in the second scheme, an alkali is added in a reaction process. The alkali may be an organic base, comprising organic amines such as a monoethanolamine, a diethanolamine or a triethanolamine, etc. The alkali may also be an inorganic base such as a hydroxide of an alkali metal or an alkaline earth metal, a carbonate, a bicarbonate, a sulfite, a bisulfite salt, or a hydride, or other dehydrogenation reagents, such as an alkali metal alkylate (RM, R is alkyl group, and M is an alkali metal), an alkali alcoholate (MOR, R is a hydrocarbyl group, and M is an alkali metal).

The present disclosure also relates to a use of a compound of the following formula in preparing a drug for treating the primary brain cancers, the brain tumors, or the metastatic cancers or tumors metastasizing to brains.

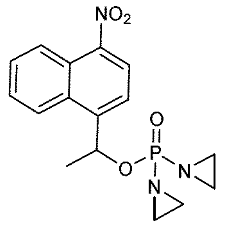

According to the above-mentioned compound synthesis routes, the synthesis and the formulation of the deuterated compounds or other isotopic variants of the present disclosure may be completed by selecting an appropriate D-substituted intermediate or starting material.

## BRIEF DESCRIPTION OF THE DRAWINGS

FIG. 1 is an inhibition rate curve diagram of different concentrations of TH-302 and compound 01 on H460 cells under normoxic and hypoxia-nitrogen conditions, wherein Log.con (μM) represents the logarithmic values of a base 10 of the concentration value in the unit of μmol/L.

FIG. 2 is a survival rate curve diagram of AA8 and UV41 cells under different concentrations of compound 01 under the normoxic condition, wherein Log.con (μM) represents the logarithmic values of a base 10 of the concentration value in the unit of μmol/L.

FIG. 3 is a scheme diagram of the average remaining percentages of TH-302 and compound 01 in samples at different times relative to an initial time after adding 53.2 μg/ml human NADPH coenzyme under a hypoxia condition (mainly nitrogen atmosphere, oxygen volume concentration < 0.1%) and air condition at 37 °C to reduce TH-302 and compound 01.

FIG. 4 is a scheme diagram of the average remaining percentages of TH-302 and compound 01 in samples at different times relative to the initial time after adding 104.6 μg/ml human NADPH coenzyme under hypoxia condition (mainly nitrogen atmosphere, oxygen volume concentration < 0.1%) and air condition at 37 °C to reduce TH-302 and compound 01.

FIG. 5 is a change curve diagram of the content percentage of compound 01 after reacting with different animal hepatomicrosomes in the presence of NADPH.

FIG. 6 is a change curve diagram of the relative content percentage of compound 01 in the serum of different animals.

FIG. 7 is a curve diagram of tumor volume change on different days under different dosage regimens in the PDX gastric cancer mouse model experiment, wherein the abscissa represents the number of days, and the ordinate represents the volume in $mm^3$.

FIG. 8 is a change curve diagram of the weight of mice on different days under different dosage regimens relative to an initial time in the PDX gastric cancer mouse model experiment, wherein the abscissa represents the number of days, and the ordinate represents the percentage of weight change.

FIG. 9 is a curve diagram of tumor volume change in different days under different dosage regimens in the CDX mouse model experiment transplanted with H460 cell line, wherein the abscissa represents the number of days, and the ordinate represents the volume in $mm^3$.

FIG. 10 is a change curve diagram of the weight of mice on different days under different dosage regimens relative to an initial time in the CDX mouse model experiment transplanted with H460 cell line, wherein the abscissa represents the number of days, the ordinate represents the percentage of weight change.

FIG. 11 is a chiral HPLC spectrogram of compound 01.

FIG. 12 is a chiral HPCL spectrogram of an optical isomer corresponding to a peak number 1 obtained after a chiral

separation of compound 01.

FIG. 13 is a chiral HPLC spectrogram of an optical isomer corresponding to a peak number 2 obtained after a chiral separation of compound 01.

FIG. 14 is a schematic diagram of an absolute stereo configuration of an optical isomer corresponding to a peak number 1 obtained after chiral separation of compound 01.

## DESCRIPTION OF THE PREFERRED EMBODIMENTS

The present disclosure will be described below with reference to specific embodiments. Person having ordinary skill in the art can understand that these embodiments are only for illustrating the present disclosure, and these embodiments do not limit the scope of the present disclosure in any way.

Unless otherwise specified, the experimental methods in the following embodiments are conventional methods, and the experiments are all carried out at normal temperature and pressure. Unless otherwise specified, the raw pharmaceutically materials, reagents, etc. used in the following embodiments are all commercially available products.

### Definition

As described herein, terms "heterocycle", "heteroaryl group" comprise 3-membered, 4-membered, 5-membere, 6-membered, or 7-membered rings. The examples are illustrated as follow.

The 3-membered ring: epoxyethane, ethyleneimine, ethylene sulfide;

The 4-membered ring: azetidine, oxetane, thietane, azete;

The 5-membered ring: pyrrolidine, pyrrolidine, 1-pyrroline, 3-pyrroline, 2-pyrroline, pyrrole, 2-pyrazoline, imidazole, pyrazole, furan, tetrahydrofuran (THF), dihydrofuran, tetrahydrothiophene (THT), thiophene, sulfolane, phosphole, oxazole, 1,2,3-triazole, 1,2,4-trizole, 1,3,4-thiadiazole;

The 6-membered ring: piperidine, tetrahydropyran (THP), thiane, pyridine, pyran, thiopyran, dihydropyridine, morpholine, piperazidine, pyridazine, pyrazine, 1,3,5-triazine, 1,3,5-trithiane;

The 7-membered ring: azepane (hexahydroazepine), oxepane, thiepane, azepine, oxepine, thiepine.

In this specification, a fused ring is defined as the combination between the above heterocycles, heteroaryl groups, or the combination with the cycloalkyl structures. The combination may be through single bond links or in a form of sharing one, two or even three atoms. Some common fused ring structures are shown as follow: naphthalene, quinolone, indole, isoindole, isoquinoline, cinnoline, quinoxaline, biphenyl, coumarin, fluorene, dibenzopyran, carbazole, anthracene, azaanthracene, phenothiazine, adamantane, azulene, phenanthrene, anthraquinone, flavone, isoflavones.

The term "leaving group" refers to a moiety that may be substituted under nucleophilic substitution conditions known to a person having ordinary skills in the art. The leaving group comprises, but without limitation, halogeno and $-OSO_2-R_{20}$. Moreover, $R_{20}$ is optionally selected from a substituted alkyl group, aryl group, cycloalkyl group, heterocycle or heteroaryl group.

The term "patient" refers to mammals that need cancer treatments. Usually, the patient refers to a human. In general, the patient refers to the human diagnosed with cancer. In certain embodiments, the "patient" may refer to a non-human mammal, for example non-human primates, dogs, cats, rabbits, pigs, mice or rats, used to select, characterize and evaluate drugs and therapies.

The term "prodrug" refers to a compound that is metabolized or transformed through other ways into a compound (or a medicine) with biological activity or higher activity after administering or administration of. The prodrug is performed chemical modifications that make the prodrug less active or inactive related to a drug. Nevertheless, the chemical modifications allow the corresponding medicine to be produced by metabolism or other biological processes after administrating of the prodrug. The prodrug may have altered metabolic stability or delivery characteristics, fewer side effects, lower toxicity, or improved flavor relative to the drug (for example, referring to reference Nogrady, 1985, Medicinal Chemistry A Biochemical Approach, Oxford University Press, New York, pages 388 to 392, which are incorporated in this specification by reference). Additionally, the prodrug can be synthesized by using reactants other than the corresponding medicine.

The term "solid tumor" refers to a solid tumor comprising, but without limitation, primary or metastatic tumors in bone, brain, liver, lung, lymph node, pancreas, prostate, skin, and soft tissue (sarcoma).

The term "therapeutically effective amount" of a drug refers to an amount of the drug that will have expected therapeutic effects when administered to a patient with cancer. It is worth to mention that the so-called expected therapeutic effects refer to alleviation, improvement, remission, or elimination of clinical manifestations of one or more cancers in patients. The expected therapeutic effects do not necessary have to occur by administration of one dose, and may only occur after administrating of a series of doses. Thus, the therapeutically effective amount can be administrated one or more times.

The term "treatment" of a condition of illness or the patient refers to taking approaches to achieve beneficial or

expected results, including clinical results. For purposes of the present disclosure, the beneficial or expected results are indicated when any of the following conditions occur, but the present disclosure is not limited thereto. The conditions include alleviation of the disease degree; postponement or improvement of diseases progression; improvement, ease, or stabilization of disease state; or other beneficial results. In some cases, cancer treatment can result in a partial response or stabilizing the disease.

The term "tumor cells" refers to tumor cells of any suitable species like mammals, for instance rodent, canine, cats, horses, or human.

## Embodiments of preparation

English abbreviation description

The term "THF" refers to tetrahydrofuran; the term "DCM" refers to dichloromethane; the term "EA" refers to ethyl acetate; the term "TEA" refers to triethylamine; the term "HPLC" refers to high performance liquid chromatography; the term "MTBE" refers to methyl tert-butyl ether; the term "DMAP" refers to 4-dimethylaminopyridine; the term "DBAD" refers to di-tert-butyl azodicarboxylate; the term "TFA" refers to trifluoroacetate; the term "MS" refers to mass spectrometry; the term "EtOH" refers to ethanol; the term "t-BuOH" refers to tert-butanol; the term "DMF" refers to N,N-dimethylformamide; the term "PE" refers to petroleum ether; the term " DMF-DMA " refers to N,N-dimethylformamide dimethyl acetal; the term "TBAF" refers to tetra-n-butylammonium fluoride; the term "DIPEA" or "DIEA" refers to N,N-diisopropylamine; the term "DIAD " refers to diisopropyl azodiformate; "t-BuOK " refers to potassium tert-butoxide.

The "Calculated refers to theoretical calculated values of mass spectrometry; "found" refers to measured values of mass spectrometry; "eq" refers to an equivalence being a molar ratio.

Other unspecified abbreviations or terms are interpreted or operated in accordance with the definitions or descriptions in the manual of organic chemistry or organic synthesis.

Relevant reagents and medicines that are not specified are purchased commercially.

The $^1$H-NMR nuclear magnetic assay was measured with a 400MHz instrument if not specified, and the MS mass spectrometry assay was measured with a LC-MS liquid chromatography-mass spectrometry.

### Preparation of Example 1: Preparation of compound 01

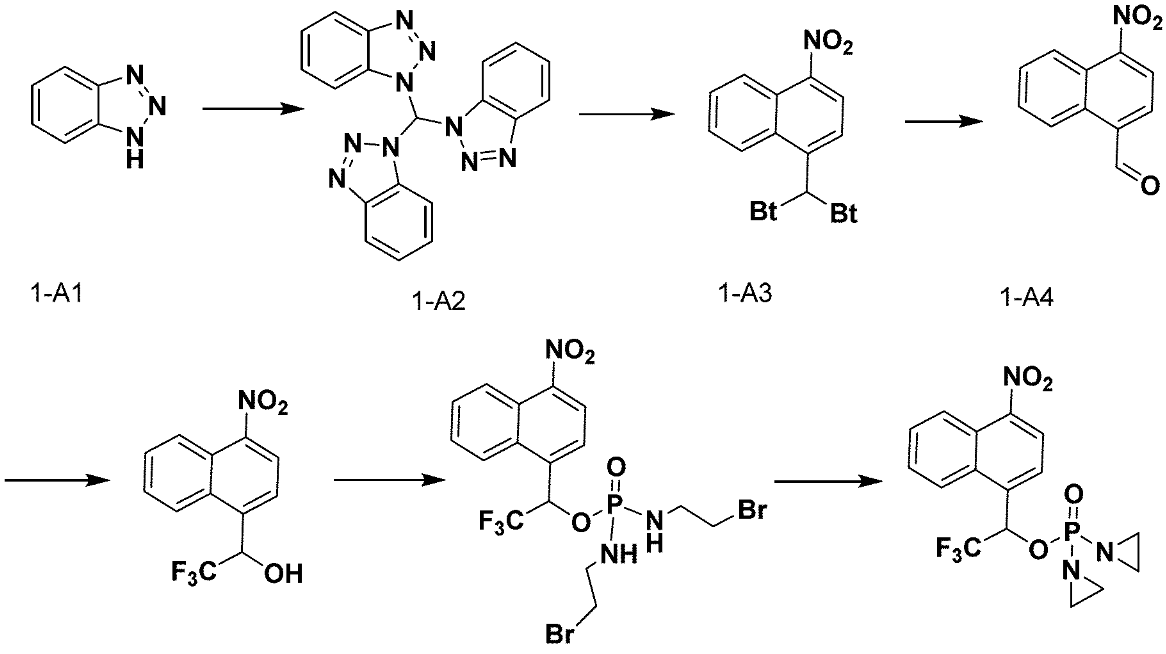

1-A1 1-A2 1-A3 1-A4

1-A5 1-A6 1

Bt= 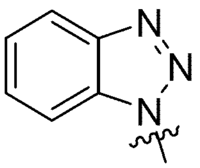

Under nitrogen protection, compound 1-A1 (20.0g, 167.9mmol), $CHCl_3$ (116.1g, 335.9mmol), tetrabutyl ammonium bromide (1.6g, 1.68mmol), and 40% of NaOH aqueous solution (50ml) were added into a 500ml-four-neck bottle. Next, the mixture was heated to 62 °C until the reaction was completed. Then, a post-treatment was performed. After the reaction system was cooled to room temperature, suction filtration was performed with diatomite. The original liquid was extracted with $CHCl_3$, dried and concentrated to obtain compound 1-A2 (7.0 g, yield 34.0%), an off-white solid. $^1$H-NMR (400MHz, DMSO): δ11.29 (s, 1H), 8.22 (d, *J* = 8.4Hz, 3H), 7.63-7.61(m, 3H), 7.55-7.51 (m, 6H).

Compound 1-A2 (6.0g, 16.3mmol), 1-nitronaphthalene (2.8g, 16.3mmol), and KOH (5.8g, 102.9mmol) was added to DMSO (80ml) at room temperature overnight, and the reaction was completed. Then, a post-treatment was performed. The reaction system was poured into hydrochloric acid so that a large amount of solid was precipitated. Chloroform was added to dissolve and extract an organic phase. Then, the mixture was dried and concentrated to obtain compound 1-A3 (7.3g). Compound 1-A3 was directly used in the next step without purification.

Under nitrogen protection, compound 1-A3 (crude product 7.0 g, 16.33 mmol) and $ZnBr_2$ (7.4 g, 32.66 mmol) were added to 100 ml of dioxane. After heating up, the mixture was stirred for 30 minutes. Next, 15 ml of concentrated hydrochloric acid was added dropwise to the reaction system, and then heating and stirring were continued until the reaction was completed. Moreover, a post-treatment was performed. After the reaction system was cooled down to room temperature, the reaction system was concentrated, dissolved in water, extracted with DCM, washed with 5% of NaOH solution, dried and concentrated, and separated with chromatography to obtain compound 1-A4 (1.3 g), a yellow solid. $^1$H-NMR (400 MHz, $CDCl_3$): *δ*10.5 3 (s, 1H), 9.30 (dd, $J_1$=7.2 Hz, 2.0 Hz, 1H), 8.37 (dd, $J_1$= 8.0 Hz, 1.8 Hz, 1H), 8.17 (d, *J* =7.6 Hz, 1H), 8.09 (d, *J* =7.6 Hz, 1H), 7.86-7.80 (m, 2H).

Under nitrogen protection, compound 1-A4 (200 mg, 0.99 mmol) and $TMSCF_3$ (283 mg, 1.98 mmol) were added to 10 ml of THF. After cooled to 0 °C, TBAF (0.1 ml) was added dropwise to the reaction system, and the reaction system was continued until the raw materials were completely converted. Then, 3 ml of hydrochloric acid (3 mol/L) was added dropwise to the reaction system, extracted with DCM. The organic phase was washed with water, washed with brine, dried and concentrated, and separated with column chromatography to obtain a product, compound 1-A5 (190 mg, 70.5%), a yellow solid. $^1$H-NMR (400 MHz, $CDCl_3$): *δ*8.49 (d, *J*= 8.0 Hz, 1H), 8.15 (d, *J*= 8.0 Hz, 1H), 7.98 (d, *J* =7.2 Hz, 1H), 7.40-7.32 (m, 2H), 5.98 (m, 1H), 2.98 (s, 1H).

Under nitrogen protection, $POCl_3$ (210mg, 1.36mmol) was added to 10 ml of DCM. After cooled to -30 °C, compound 1-A5 (185mg, 0.68mmol) was dissolved in DCM, which was added dropwise to the reaction system. Next,

TEA (173mg, 1.71mmol) was dissolved in 5ml of DCM, which was added dropwise to the reaction system. The reaction system was kept carrying out at -30 °C until a conversion of the raw materials was completed. Bromoethylamine hydrobromide (1.1g, 5.46mmol) and TEA (552mg, 5.46mmol) were added to the reaction system. 30 minutes later, the reaction was completed. At 0 °C, after 20 ml of saturated ammonium chloride aqueous solution was added, the mixture was extracted with DCM. The organic phase was washed with water, washed with brine, dried and concentrated, and separated with column chromatography to obtain a product, compound 1-A6 (170mg · 44.3%), a yellow solid. $^1$H-NMR (400 MHz, $CDCl_3$): δ8.49 *(d, J* = 1.2 Hz, 1H), 8.26 (d, *J* = 7.6 Hz, 1H), 8.17 (d, *J* = 8.0 Hz, 1H), 7.90 (d, *J*= 8.0 Hz, 1H), 7.80-7.75 (m, 2H), 6.63-6.61 (m, 1H), 3.56-2.94 (m, 8H). MS : Calculated 562.9, found 563.9 ([M+H]$^+$).

Under nitrogen protection, compound 1-A6 (160mg, 0.28mmol) was dissolved in THF (15ml). Next, silver oxide (329mg, 1.42mmol) and DIEA (167mg, 1.42mmol) were added to the reaction system. After the reaction system was heated to 65 °C, the reaction system was kept stirring until the reaction was completed. The reaction system was suction filtered with diatomite. Then, the solid was washed with water. The original liquid was concentrated and prepared with high performance liquid chromatography (HPLC) to obtain a pure product, compound 01, a light yellow solid. $^1$H-NMR (400 MHz, $CDCl_3$): δ8.57-8.44 (m, 1H), 8.31-8.10 (m, 2H), 7.96 (d, *J*= 7.9 Hz, 1H), 7.84-7.67 (m, 2H), 6.65 (s, 1H), 2.33-2.21 (m, 2H), 2.20-2.09 (m, 2H), 2.06-1.96 (m, 4H). MS: Calculated 401.1, found 402.0 ([M+H]$^+$).

**Preparation of Example 2: Preparation of compound 02**

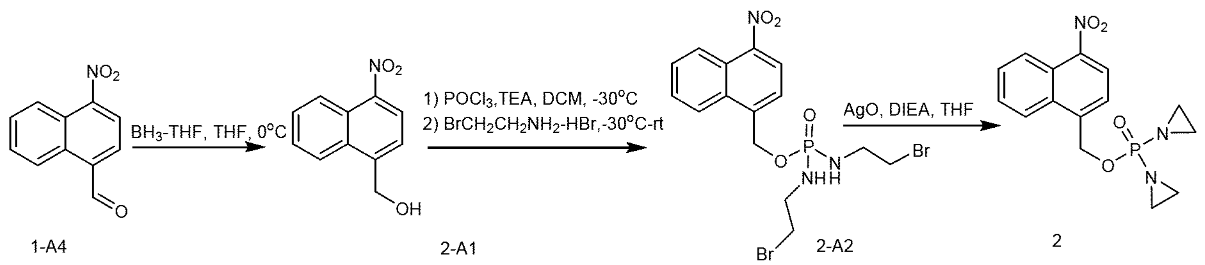


1-A4 2-A1 2-A2 2

Under nitrogen protection, compound 1-A4 (500 mg, 2.5 mmol) was added to THF (10 ml). After cooled to 0 °C, $BH_3$-THF (12.4 mL, 12.4 mmol, 1M in THF) was added dropwise to the system. After the addtion, the system was stirred at 0 °C for 30 minutes to complete the reaction. Methanol (10 ml) was added dropwise and stirred for 30 minutes, and the solvent was evaporated dry. A crude product was dissolved in DCM (20 ml), which was washed with 1M of HCl (20 ml×3), washed with water (10 ml), washed with brine (10 ml), dried with anhydrous sodium sulfate. Then, the solvent was evaporated dry to obtain compound 2-A1 (470 mg · 93.1 %), a yellow solid. $^1$H-NMR (400 MHz, $CDCl_3$): δ8.57 (d, *J* = 8.4 Hz, 1H), 8.19 (d, *J* =8.0 Hz, 1H), 8.13 (d, *J* =8.0 Hz, 1H), 7.76-7.66 (m, 3H), 5.26 (s, 2H).

Under nitrogen protection, $POCl_3$ (151 mg, 1.0 mmol) was added to DCM (5 ml), and the mixture was cooled to -30 °C. Next, compound 2-A1 (100 mg, 0.5 mmol) was dissolved in DCM (2 ml), which was added dropwise to the system. TEA (125 mg, 1.2 mmol) was dissolved in DCM (2 ml), which was added dropwise to the system to react for 1.5 hours. At the same temperature, after 2-bromoethylamine hydrobromide (822 mg, 3.9 mmol) and TEA (398 mg, 3.9 mmol) were added to the system sequentially. 30 minutes later, the reaction was completed. After heating up to 0 °C, saturated ammonium chloride solution (10 ml) was added dropwise. Water (10 ml) was added, which was extracted with DCM (10 ml×3), washed with water (5 ml), washed with saturated brine (5 ml), dried with anhydrous sodium sulfate, evaporated the solvent, and purified with column chromatography to obtain compound 2-A2 (110 mg, 45.1 %), a light yellow solid. $^1$H-NMR (400 MHz, $CDCl_3$): δ8.57 (d, *J* =8.4 Hz, 1H), 8.16 (d, *J* =7.6 Hz, 2H), 7.79-7.68 (m, 3H), 5.56 (d, *J* =7.2 Hz, 1H), 3.44-3.12 (m, 8H). MS: Calculated 492.9, 494.9, 496.9, found 493.9, 495.9, 497.9 ([M+H]$^+$).

Under nitrogen protection, compound 2-A2 (100 mg, 0.2 mmol) was added to THF (10 ml). Then, $Ag_2O$ (234 mg, 1.0 mmol) and DIEA (130 mg, 1.0 mmol) were added to the system. After heated up to reflux for 7 hours, the reaction was completed. After cooled to room temperature, the mixture was suction filtered with diatomite, washed with DCM, evaporated the solvent, and prepared by HPLC to obtain compound 02 (45.0 mg, 67.2%), a yellow oily substance. $^1$H-NMR (400 MHz, $CD_3OD$): δ 8.44-8.42 (m, 1H), 4.30-8.28 (m, 1H), 8.19 (d, *J* = 8.0 Hz, 1H), 7.81-7.76 (m, 3H), 5.72 (d, *J* = 7.6 Hz, 1H), 2.23-2.16 (m, 8H). MS: Calculated 333.1, found 334.0 ([M+H]$^+$).

**Preparation of Example 3: Preparation of compound 03**

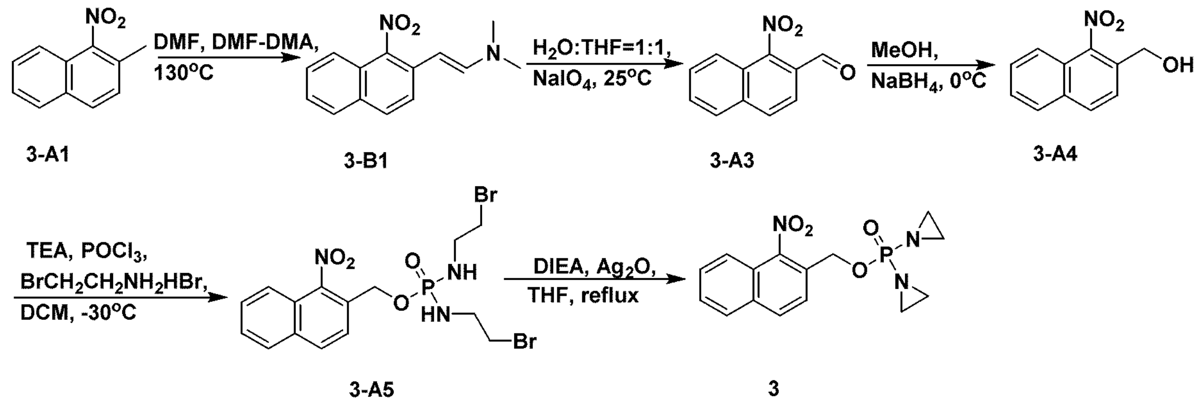


3-A1 3-B1 3-A3 3-A4 3-A5 3

Under nitrogen protection, compound 3-A1 (1.0 g, 5.3 mmol, purchased) was dissolved in DMF (8 ml). Next, after DMF-DMA (1.9 g, 16.0 mmol) was added, the mixture was heated up to 130 °C and stirred for 16 hours. Afterwards, the reaction was completed, and the mixture was cooled to room temperature. DMF was steamed off 2/3, and water (20 ml) was added. A solid was precipitated and filtered with suction. The solid was dried to obtain a crude product, compound 3-B1 (1.3 g, 100 %), a black solid. $^1$H-NMR (400 MHz, DMSO-$d_6$): $\delta$ 7.89-7.90 (m, 3H), 7.56-7.51 (m, 2H), 7.43-7.39 (m, 2H), 4.89 (d, *J* = 13.2 Hz, 1H), 2.89 (s, 6H).

Under nitrogen protection, compound 3-B1 (1.0 g, 4.1 mmol) was dissolved in THF-aqueous solution (1:1, 40 ml). Sodium periodate (2.6 g, 12.4 mmol) was added in batches, which was stirred at 25 °C for 2 hours, and the reaction was completed. After suction filtration, the solid was soaked in DCM and suction filtered again. The original liquid was dried with anhydrous sodium sulfate, and evaporated dry to obtain compound 3-A3 (600 mg, 72.2%), a yellow solid. $^1$H-NMR (400 MHz, $CDCl_3$): $\delta$10.17 (s, 1H), 8.11 (d, *J* = 8.4 Hz, 1H), 8.00-7.91 (m, 3H), 7.77-7.72 (m, 2H).

Compound 3-A3 (450 mg, 2.2 mmol) was dissolved in MeOH (12 mL). After cooled to 0 °C, sodium borohydride (127 mg, 3.4 mmol) was added in batches. As soon as the addition was completed, the reaction ended. Water (10 ml) was added to quench, and the mixture was extracted with DCM (20 ml×2). The organic phase was washed with 5 % of citric acid aqueous solution (10 ml×2), washed with water (10 ml×2), and washed with brine (10 ml). The organic phase was dried with anhydrous sodium sulfate and evaporated dry to obtain compound 3-A4 (450 mg, 99.0%), a yellow solid. $^1$H-NMR (400 MHz, $CDCl_3$): $\delta$ 8.03 (d, *J* = 8.8 Hz, 1H), 7.92 (d, *J*= 8.0 Hz, 1H), 7.85 (d, *J* = 8.4 Hz, 1H), 7.68-7.59 (m, 3H), 4.85 (s, 2H).

Under nitrogen protection, $POCl_3$ (679 mg, 4.43 mmol) was dissolved in DCM (12 mL). After cooled to -30 °C, compound 3-A4 (450 mg, 2.21 mmol) and TEA (560 mg, 5.54 mmol) in DCM (2 ml) were added dropwise to the system. After the temperature was kept for 2 hours, 2-bromoethylamine hydrobromide solid (2.7 g, 13.3 mmol) was added. Finally, TEA (1.8 g, 17.7 mmol) was added. 30 minutes later, and the reaction was completed. Saturated ammonium chloride aqueous solution (2 ml) was added to quench. The mixture was extracted with DCM (10 ml×3), and washed with water (3 ml×2). The organic phase was dried with anhydrous sodium sulfate, evaporated dry, and purified with column chromatography to obtain compound 3-A5 (1.0 g, 91.2%), a yellow solid. $^1$H-NMR (400 MHz, $CDCl_3$): $\delta$8.03 (d, *J* = 8.4 Hz, 1H), 7.94 (d, *J* = 7.6 Hz, 1H), 7.82 (d, *J*= 8.4 Hz, 1H), 7.70-7.60 (m, 3H), 5.25 (d, *J* = 7.6 Hz, 2H), 3.46-3.43 (m, 4H), 3.36-3.32 (m, 4H), 3.11 (brs, 2H). MS: Calculated 492.9, 494.9, 496.9, found 494.0, 496.0, 498.0 ($[M+H]^+$).

Under nitrogen protection, compound 3-A5 (500 mg, 1.0 mmol) was dissolved in THF (20 ml). DIEA (653 mg, 5.1 mmol) and silver oxide (625 mg, 2.8 mmol) were added, and the mixture was heated up to reflux. One hour later, the reaction was completed. After suction filtered with diatomite, the original liquid was evaporated dry, and purified with HPLC to obtain a product, compound 03 (75 mg, 22.3%), an off-white solid. $^1$H-NMR (400 MHz, $CD_3OD$): $\delta$8.17 (d, *J* = 8.4 Hz, 1H), 8.06 *(d, J* = 7.6 Hz, 1H), 7.80 (d, *J*= 8.2 Hz, 1H), 7.74-7.67 (m, 3H), 5.38 (d, *J* = 7.6 Hz, 2H), 3.23-2.16 (m, 8H). MS: Calculated 333.1, found 334.1 ($[M+H]^+$).

**Preparation of Example 4: preparation of compound 04**

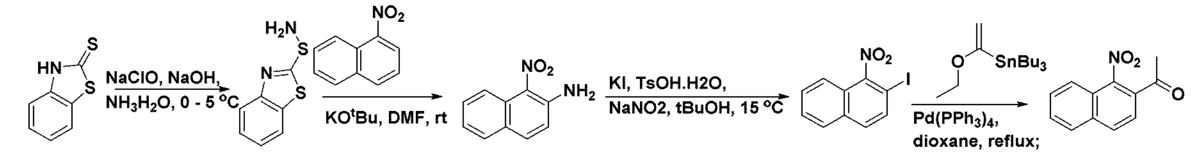


4-A1 4-A2 4-A3 4-A4 4A5

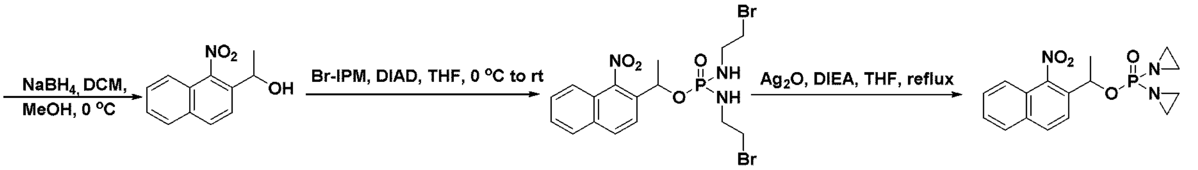


4-A6 4-A7 4

With 2 dropping-funnels, compound 4-A1 (30.0 g, 179.4 mmol, purchased) in NaOH solution (1.58 M, 120 ml) and NaClO aqueous solution (10 % active chlorine, 144 ml) were slowly added dropwise into stirred concentrated ammonia (450 ml) at 0 °C. Besides, the dropping time was 2 hours, and the temperature was kept between -5 °C to 0 °C. After dropping finished, the reaction was continued for 2 hours. After the reaction was completed, the solid was suction filtered. Then, the filter cake was washed with cold water (2 L). The filter cake was dissolved in hot DCM (200 ml), dried with anhydrous sodium sulfate, and concentrated to obtain compound 4-A2 (26.0 g, 79.5 %), an off-white solid. $^1$H-NMR (400 MHz, DMSO-d6): $\delta$7.99 (d, $J$ = 8.1 Hz, 1H) 7.72 (d, $J$ = 8.1 Hz, 1H), 7.40 (t, $J$ = 7.5 Hz, 1H), 7.31-7.28 (m, 1H), 4.94 (brs, 2H). MS: Calculated 182.0, found 183.0 ([M+H]$^+$).

At room temperature, a mixed DMF solution (200 ml) of compound 4-A2 (20.0 g, 109.7 mmol) and 1-nitronaphthalene (21 g, 121.2 mmol) was added dropwise to DMF (100 ml) dissolved with t-BuOK (31 g, 276.3 mmol) in 2 hours. After the addition, the reaction was carried out for 0.5 hours. The above system was slowly added to cold water (500 ml), which was stirred for 0.5 hours. The mixture was extracted with EA (200 ml×3). The organic phase was washed with water (50 ml×2), dried with anhydrous sodium sulfate, and evaporated dry. Compound 4-A3 (11.1 g, LC content about 80 %, yield 53.8 %), a yellow solid, was obtained with column chromatography. $^1$H-NMR (400 MHz, $CDCl_3$): $\delta$7.90-7.85 (m, 2H), 7.70 -7.75 (m, 4H). MS: Calculated 188.1, found 189.2 ([M+H]$^+$).

Compound 4-A3 (3.1 g, 16.5 mmol) and para-toluenesulfonic acid monohydrate (16.9 g, 98.8 mmol) were dissolved in tert-butanol (150 ml). At 15 °C, an aqueous solution (75 ml) of $NaNO_2$ (4.6 g, 66.7 mmol) and KI (13.7 g, 82.5 mmol) was added dropwise. After the addition, the temperature was raised to 25 °C and the mixture was reacted for 2 hours. After the reaction was completed, saturated $NaHCO_3$ solution was added to adjust the pH value to 9-10. Next, the mixture was extracted with EA (50 ml×3), washed with saturated $Na_2S_2O_3$ aqueous solution (20 ml×3), washed with water (10 ml×3), dried with anhydrous sodium sulfate, and evaporated dry. Then, compound 4-A4 (2.0 g, 40.8%), a light yellow solid, was obtained with column chromatography. $^1$H-NMR (400 MHz, $CDCl_3$): $\delta$7.91-7.84 (m, 2H), 7.70 -7.61 (m, 4H).

Under nitrogen protection, compound 4-A4 and tributyl-(1-ethoxyvinyl)tin (8.0 g, 22.0 mmol) were dissolved in 1,4-dioxane (40 ml). Nitrogen was sucked and refilled three times, and tetrakis(triphenylphosphine)palladium (454mg, 0.4 mmol) was added. Next, nitrogen was replaced three times, and the temperature was heated to 110 °C to react overnight. After cooled to room temperature, 3M of HCl (30 ml) was added. The mixture was stirred for 2 hours, extracted with DCM (40 ml×3), dried, and evaporated dry. A crude product was subjected to column chromatography to obtain compound 4-A5 (2.5 g, 73.5 %), a yellow solid. $^1$H-NMR (400 MHz, $CDCl_3$): $\delta$8.07 (d, $J$ = 8.6 Hz, 1H), 7.98-7.96 (m, 1 H), 7.90-7.87 (m, 1H), 7.80 - 7.77 (m, 1H), 7.72 (m, 2H), 2.69 (s, 3H). MS : Calculated 215.1, found 216.2 ([M+H]$^+$).

Compound 4-A5 (750 mg, 3.5 mmol) was dissolved in MeOH (20 mL) and DCM (10 mL), which cooled to 0 °C. $NaBH_4$ (158 mg, 4.2 mmol) was added in batches, and the temperature was kept for 1 hour to react. After the reaction was completed, $H_2O$ (5 mL) was added. The mixture was extracted with DCM (10 ml×3), and washed with 25 % of citric acid (10 ml×2). The organic phase was washed with water (5 ml×2), dried with anhydrous sodium sulfate, spun off the solvent, and separated with column chromatography to obtain compound 4-A6 (300 mg · 39.5 %), a light yellow solid.

Under nitrogen protection, compound 4-A6 (130 mg, 0.6 mmol), Br-IPM (557 mg · 1.8 mmol), and triphenylphosphine (479 mg · 1.8 mmol) were added to THF (10 ml). After cooled to 0°C, DIAD (364 mg · 1.8 mmol) was added dropwise, and the temperature was naturally raised to room temperature to react for 1 hour. After the reaction was completed, the original liquid was concentrated. A crude product, compound 4-A7 (150 mg · 49.2 %), a light yellow oily substance, was obtained with column chromatography.

Under nitrogen protection, compound 4-A7 (150 mg, 0.3 mmol), DIEA (190 mg, 1.5 mmol), and $Ag_2O$ (348 mg · 1.5 mmol) were added to THF (10 ml). Then, the mixture was stirred to reflux overnight. After the reaction was completed, the mixture was suction filtered with diatomite. Next, the filtrate was concentrated, and compound 04 (40 mg, 39.2 %), a yellow oily substance, was obtained with HPLC. $^1$H-NMR (400 MHz, $CD_3OD$) $\delta$8.19 (d, $J$ = 8.8 Hz, 1H), 8.05 (d, $J$ =

7.2 Hz, 1H), 7.80-7.68 (m, 4H), 5.79-5.77 (m, 1H), 2.19-2.01 (m, 8H), 1.74 (d, *J*= 6.4 Hz, 3H). MS: Calculated 347.1, found 348.1 ([M+H]$^+$).

**Preparation of Example 5: preparation of compound 05**

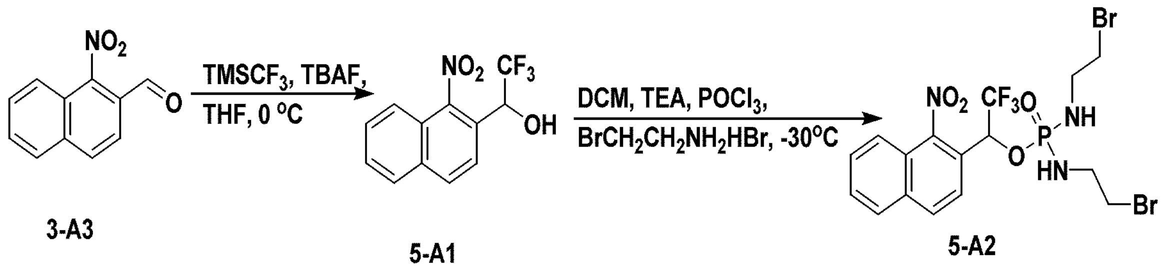


3-A3

5-A1

5-A2

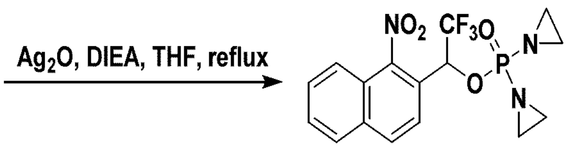


5

Under nitrogen protection, compound 3-A3 (1.8 g, 9.0 mmol) was dissolved in THF (18 ml). Next, TMSCF$_3$ (1.9 g, 13.4 mmol) was added, which was cooled to 0 °C. A THF solution (IN, 0.179 mL, 0.18 mmol) of TBAF was added. 0.5 hours later, the reaction was completed. 3M of hydrochloric acid (9 ml) was added. The mixture was stirred for 15 minutes, extracted with DCM (20 ml×3), and washed with brine (10 ml×2). The organic phase was dried with anhydrous sodium sulfate, evaporated dry. Compound 5-A1 (2.2 g, 90.7%), a black-like solid, was obtained with column chromatography. $^1$H-NMR (400 MHz, CDCl$_3$): $\delta$8.07 (d, *J* = 8.7 Hz, 1H), 7.96 - 7.94 (m, 1H), 7.86 (d, *J*= 8.7 Hz, 1H), 7.77 (m, 1H), 7.69-7.66 (m, 2H), 5.46 (m, 1H), 3.36 (brs, 1H).

Under nitrogen protection, POCl$_3$ (565 mg, 3.7 mmol) was dissolved in DCM (12 ml). After cooled to -30 °C, a solution of compound 5-A1 (500 mg, 1.8 mmol) and TEA (466 mg, 4.6 mmol) in DCM (2 ml) was added dropwise to the reaction system. After the temperature was kept for 2 hours, 2-bromoethylamine hydrobromide (2.3 g, 11.1 mmol) was added. Finally, after TEA (1.5 g, 14.7 mmol) was added. 0.5 hours later, the reaction was completed. Saturated ammonium chloride aqueous solution (3 ml) was added to quench. The mixture was extracted with DCM (10 ml×3), and washed with water (3 ml×2). The organic phase was dried with anhydrous sodium sulfate, evaporated dry. Compound 5-A2 (850 mg, 81.9%), a yellow oily substance, was obtained with column chromatography. $^1$H-NMR (400 MHz, CDCl$_3$): $\delta$8.11 (d, *J* = 8.4 Hz, 1H), 7.98 (m, 1H), 7.78-7.69(m, 4H), 6.04-5.98 (m, 1H), 3.41-3.18 (m, 8H). MS: Calculated 560.9, 562.9, 564.9, found 561.9, 563.9, 565.9 ([M+H]$^+$).

Under nitrogen protection, compound 5-A2 (300 mg, 0.5 mmol) was dissolved in THF (20 ml). DIEA (344 mg, 2.7 mmol) and silver oxide (344 mg, 2.7 mmol) were added. The mixture was heated to reflux for one hour to complete the reaction. The mixture was suction filtered with diatomite. The original liquid was evaporated dry, and prepared with HPLC to obtain a product, compound 05 (70 mg, 32.7%), a yellow oily substance. $^1$H-NMR (400 MHz, CD$_3$OD): $\delta$8.29 (d, *J* = 8.8 Hz, 1H), 8.13-8.10 (m, 1H), 7.88 (d, *J* = 8.8 Hz, 1H), 7.80-7.76 (m, 3H), 6.16-6.09 (m, 1H), 2.25-2.03 (m, 8H). MS: Calculated 401.1, found 402.1 ([M+H]$^+$).

**Preparation of Example 6: preparations of compound 06, 24 and 25**

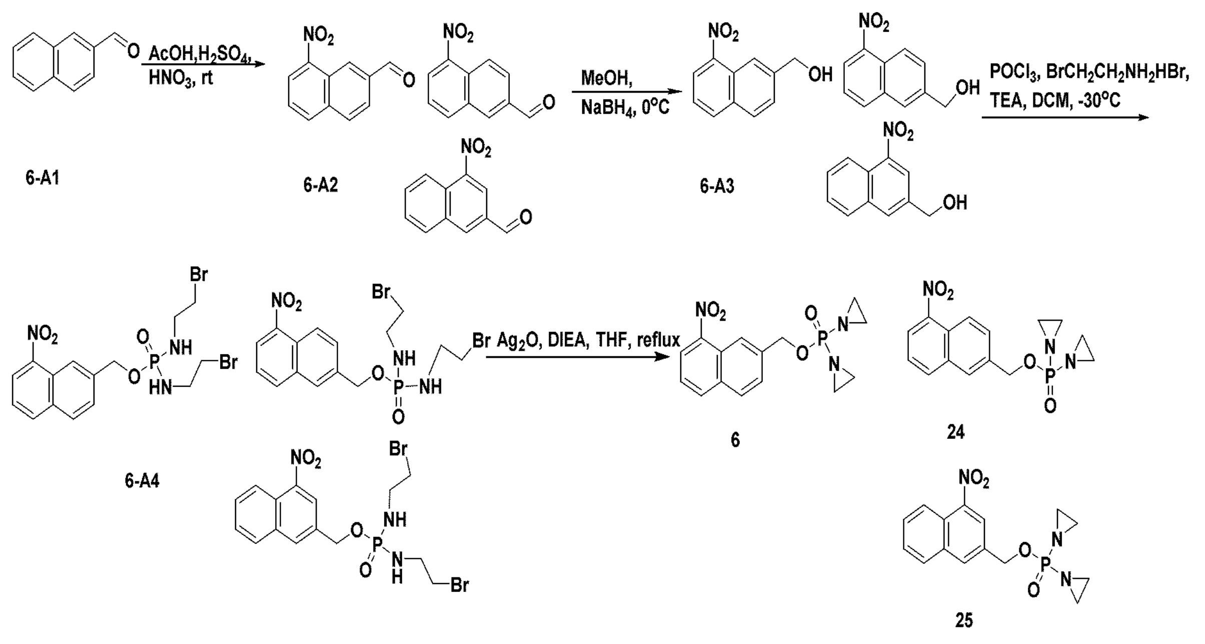


Under nitrogen protection, concentrated sulfuric acid (2.3 mL, 42.2mmol) was added to acetic acid (90 ml), and compound 6-A1 (4.5 g, 28.8 mmol) was added thereto. Then, fuming nitric acid (48 mL, 1.15 mol) was added dropwise to the reaction system overnight, and the reaction was completed. Next, a post-treatment was performed. The reaction system was added to ice water (350 ml). The mixture was suction filtered, washed with water (80 ml), and dried to obtain compound 6-A2 and isomers of compound 6-A2 (4.0 g, 69.0%, three isomers), yellow solids. The compound 6-A2 and the isomers of compound 6-A2 were directly used in the next reaction.

Under nitrogen protection, compound 6-A2 was dissolved in methanol (20 ml). After cooled to 0 °C, sodium borohydride (423 mg, 11.2 mmol) was added in batches. The reaction was completed after the addition. 10 ml of water was added to quench. The mixture was extracted with DCM (15 ml×2), washed with 5 % of citric acid aqueous solution (8 ml×2), washed with water (8 ml×2), washed with brine (8 ml), dried, and evaporated dry to obtain compound 6-A3 and an isomer of compound 6-A3 (1.4 g, 92.4%), yellow solids. MS: Calculated 203.1, found 204.1 ($[M+H]^+$).

Under nitrogen protection, compound 6-A3, the isomer of compound 6-A3 (400 mg, 2.0 mmol), and $POCl_3$ (604 mg, 3.9 mmol) were added to DCM (8 ml). After cooled to -30 °C, TEA (498 mg, 4.9 mmol) was added. One hour later, 2-bromoethylamine hydrobromide (3.3 g, 15.7 mmol) was added. Next, TEA (1.6 g, 15.7mmol) was added dropwise, and the reaction ended 0.5 hours later. Water (5 ml) was added. The mixture was extracted with DCM (15 ml×2), washed with water (10 ml), dried, and evaporated dry. Compound 6-A4 and isomers (850 mg, 87.2%, mainly three isomers), yellow oily substances, were obtained with column chromatography.

Under nitrogen protection, compound 6-A4 and the isomers of compound 6-A4 (850 mg, 1.7 mmol) were dissolved in THF (20 ml). Silver oxide (1.2 g, 5.2 mmol) and DIEA (1.1 g, 8.6 mmol) were added. The reaction was completed by heating to reflux overnight. The mixture was suction filtered with diatomite, evaporated dry, and prepared with HPCL (acetonitrile/water reversed-phase chromatography) to obtain three products compounds 06, 24 and 25.

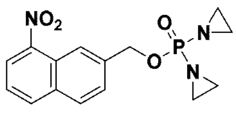

6

was a yellow solid, 25 mg, and yield 4.4 %. $^1$H-NMR (400 MHz, $CD_3OD$): $\delta$8.52 (s, 1H), 8.28 (dd, $J$ = 8.0, 4.0 Hz, 2H), 8.12 (d, $J$ = 8.4 Hz, 1H), 7.72-7.64 (m, 2H), 5.40 (d, $J$ = 8.0 Hz, 2H), 2.28 - 2.19 (m, 8H). MS: Calculated 333.1, found 334.1 ($[M+H]^+$).

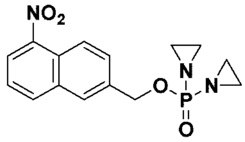

24

was a white solid, 26 mg, and yield 4.5 %. $^1$H-NMR (400 MHz, $CD_3OD$): $\delta$8.50 (d, $J$ = 8.8 Hz, 1H), 8.28 (dd, $J$ = 8.0, 4.0 Hz, 2H), 8.11(s, 1H), 7.80(dd, $J$ = 8.8, 1.6 Hz, 1H), 7.67(t, $J$ = 8.0 Hz, 1H), 5.38(d, $J$ = 8.4 Hz, 2H), 2.25-2.18 (m, 8H). MS: Calculated 333.1, found 334.1 ([M+H]$^+$).

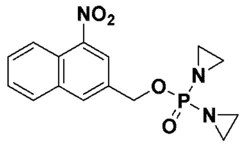

25

was a yellow oily substance, 8.7 mg, and yield 1.5 %. $^1$H-NMR (400 MHz, $CD_3OD$): $\delta$8.45 (d, $J$ = 8.4 Hz, 1H), 8.33-8.28 (m, 2H), 8.11 (d, $J$ = 8.0 Hz, 1H), 7.80-7.69 (m, 2H), 5.41(d, $J$ = 8.8 Hz, 2H), 2.27-2.19 (m, 8H). MS: Calculated 333.1, found 334.1 ([M+H]$^+$).

**Preparation of Example 7: preparation of compound 07**

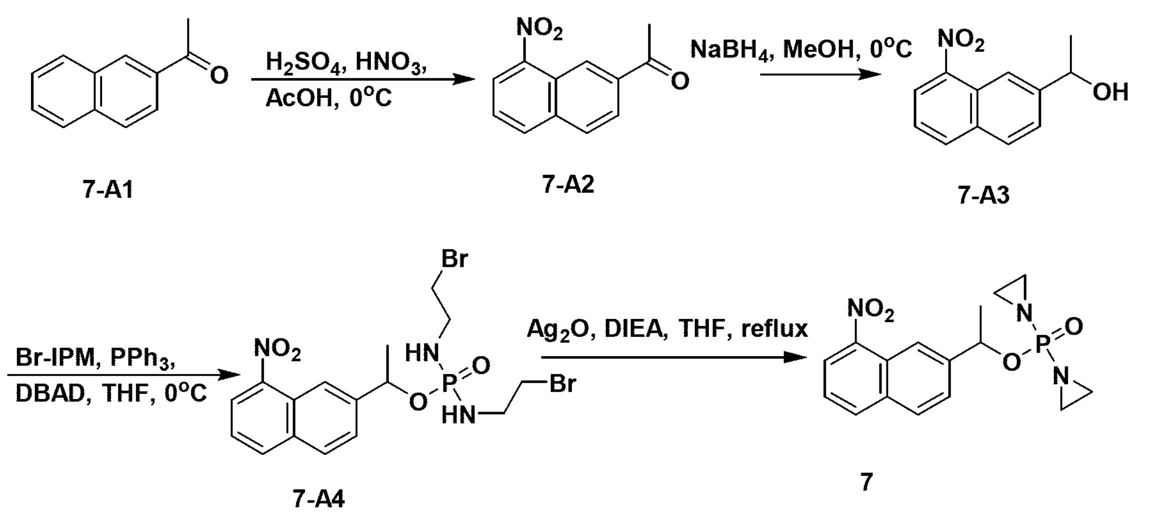


7-A1 7-A2 7-A3 7-A4 7

Under nitrogen protection, concentrated sulfuric acid (2.4 mL, 44.1 mmol) was added dropwise to acetic acid (50 ml), then and compound 7-A1 (5.0 g, 29.4 mmol, Energy) was added thereto. After cooled to 0 °C, fuming nitric acid (49 ml) was added dropwise. The mixture was rapidly stirred at room temperature. The end of the reaction was monitored by liquid chromatography. The system was carefully poured into ice water (400 ml), which was suction filtered. The obtained solid was dissolved in DCM. Compound 7-A2 (170 mg, 2.7%), a yellow solid, was obtained by column chromatography. $^1$H-NMR (400 MHz, $CDCl_3$): $\delta$9.22 (s, 1H), 8.33 (d, $J$ = 7.6 Hz, 1H), 8.21-8.17 (m, 2H), 8.04 (d, $J$ = 8.8 Hz, 1H), 7.72-7.68 (m, 1H), 2.78 (s, 3H). MS: Calculated 215.1, found 216.1 ([M+H]$^+$).

Under nitrogen protection, compound (120 mg, 0.6 mmol) was dissolved in methanol (3 ml). After cooled to 0 °C, sodium borohydride (23 mg, 0.6 mmol) was added in batches. After the addition, the end of the reaction was monitored by thin-layer chromatography. Water (2 ml) was added to quench, which was extracted with DCM (10 ml×2), washed with 5 % of citric acid aqueous solution (5 ml×2), washed with water (5 ml×2), and washed with brine (5 ml). The organic phase was dried with anhydrous sodium sulfate, and evaporated dry to obtained compound 7-A3 (100 mg, 82.6%), a yellow solid. $^1$H-NMR (400 MHz, $CDCl_3$) $\delta$8.52 (s, 1H), 8.23 (d, $J$ = 0.8 Hz, 1H), 8.11 (d, $J$ = 8.4 Hz, 1H), 7.97 (d, $J$ = 8.4

Hz, 1H), 7.72 (d, *J* = 1.2 Hz, 1H), 7.53-7.51 (t, *J* = 7.9 Hz, 1H), 5.16-5.12 (m, 1H), 1.60 (d, *J* = 6.4 Hz, 3H).

Under nitrogen protection, compound 7-A3 (150 mg, 0.7 mmol) and Br-IPM (533 mg, 1.7 mmol) were dissolved in ultra-dry THF, and triphenylphosphine (452 mg, 1.7 mmol) was added. After cooled to 0 °C, di-tert-butyl azodicarboxylate (397 mg, 1.7 mmol) was added. 0.5 hours later, the end of the reaction was monitored by liquid chromatography. The solvent was evaporated dry, and a crude product, compound 7-A4 (150 mg, 42.7%), a yellow oily substance, was obtained by column chromatography.

Under nitrogen protection, compound 7-A4 (150 mg, 0.3 mmol), silver oxide (205 mg, 0.9 mmol), and DIEA (190 mg, 1.5 mmol) were added to THF (3 ml). One hour later, the end of the reaction was monitored by liquid chromatography. After suction filtered, the original liquid was evaporated dry. Compound 07 (32 mg, 31.3%), a yellow oily substance, was obtained with HPLC. $^1$H-NMR (400 MHz, $CD_3OD$) $\delta$8.50 (s, 1H), 8.29-8.26 (m, 2H), 8.12 (d, *J* = 8.4 Hz, 1H), 7.73 (dd, *J* = 8.4, 1.6 Hz, 1H), 7.66 (t, *J* = 8.0 Hz, 1H), 5.86-5.80 (m, 1H), 2.24-2.07 (m, 8H), 1.72 (d, *J* = 6.4 Hz, 3H). MS: Calculated 347.1, found 694.9 ([2M+H]$^+$).

**Preparation of Example 8: preparation of compound 08**

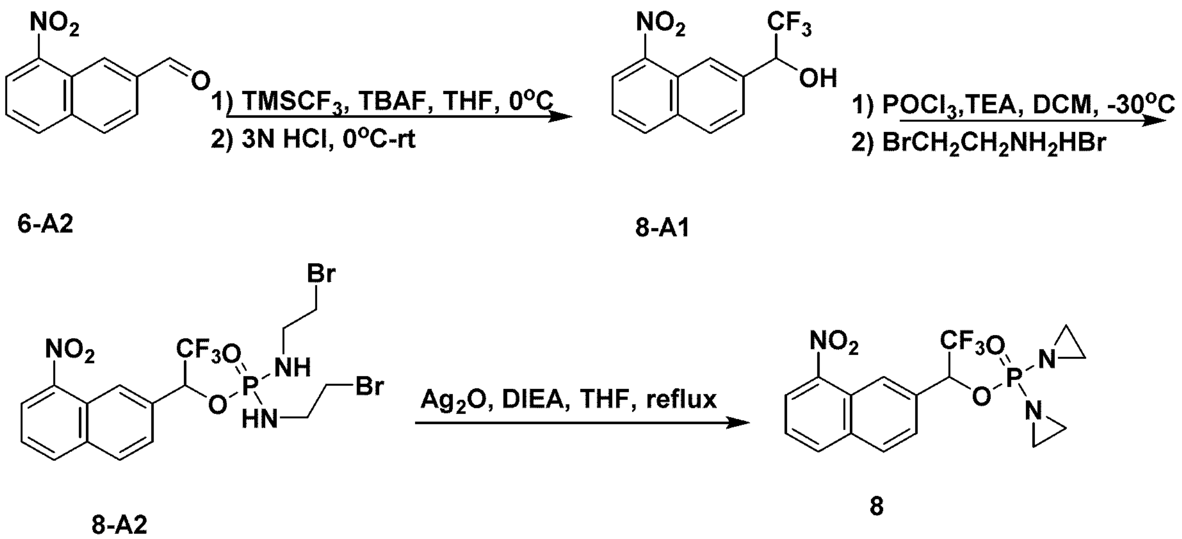


6-A2 8-A1 8-A2 8

Under nitrogen protection, compound 6-A2 (1.5 g, 7.5 mmol) and $TMSCF_3$ (2.1 g, 14.9 mmol) were added to THF (25 ml). After cooled to 0 °C, TBAF (0.15 mL, 0.15 mmol, 1M in THF) was added dropwise to the system. After being kept at 0 °C for 30 minutes, the raw materials was vanished. 3 N of HCl (20 mL) was added to the reaction system. The mixture was extracted with DCM (30 ml×3) after stirred at room temperature for one hour. Then, the organic phase was washed with brine (5 ml×2), dried and concentrated, and purified with column chromatography to obtained compound 8-A1 (1.5 g, 74.2%), a yellow oily substance. $^1$H-NMR (400 MHz, ($CDCl_3$): $\delta$8.70 (s, 1H), 8.31-8.29 (m, 1H), 8.16 (d, *J* = 8.4 Hz, 1H), 8.03 (d, *J* = 8.8 Hz, 1H), 7.79 (d, *J* = 8.4 Hz, 1H), 7.62 (t, *J* = 8.0 Hz, 1H), 5.32-5.27 (m, 1H).

Under nitrogen protection, $POCl_3$ (678 mg, 4.4 mmol) and compound 8-A1 (600 mg, 2.2 mmol) were added to DCM (15 ml). After cooling to -30 °C, TEA (560 mg, 5.5 mmol) was dissolved in DCM (5 ml) and added dropwise to the reaction system. After being kept at -30 °C for 2 hours, 2-bromoethylamine hydrobromide (3.7 g, 17.7 mmol) and TEA (1.8 mg, 17.7 mmol) were added sequentially. 30 minutes later, the reaction was completed. Saturated ammonium chloride aqueous solution (10 ml) was added dropwise, which was extracted with DCM (20 ml×3). The organic phase was washed with water, washed with brine, dried with anhydrous sodium sulfate, evaporated the solvent, and separated with column chromatography to obtain compound 8-A2 (830 mg, LC content about 70 %, yield 66.6 %), a yellow oily substance. Compound 8-A2 was directly put into the next reaction without NMR and MS assay.

Under nitrogen protection, compound 8-A2 (330 mg, 0.6 mmol) was dissolved in THF (10 ml). Then, silver oxide (679 mg, 2.9 mmol) and DIEA (379 mg, 2.9 mmol) were added, which was heated to reflux and stirred overnight. After the reaction was completed, the mixture was suction filtered with diatomite, washed with DCM. The original liquid was concentrated, and prepared with HPLC to obtain a pure product, compound 08 (29 mg, 12.3%), an off-white solid. $^1$H-NMR (400 MHz, $CDCl_3$): $\delta$8.75 (s, 1H), 8.33 (d, *J* = 7.6 Hz, 1H), 8.18 (d, *J* = 8.0 Hz, 1H), 8.04 (d, *J* = 8.4 Hz, 1H), 7.76 (d, *J* = 8.4 Hz, 1H), 7.65 (t, *J* = 7.6 Hz, 1H), 5.95-5.93 (m, 1H), 2.31-2.02 (m, 8H). MS: Calculated 401.1, found 402.0 ([M+H]$^+$).

## Preparation of Example 9: preparation of compound 09

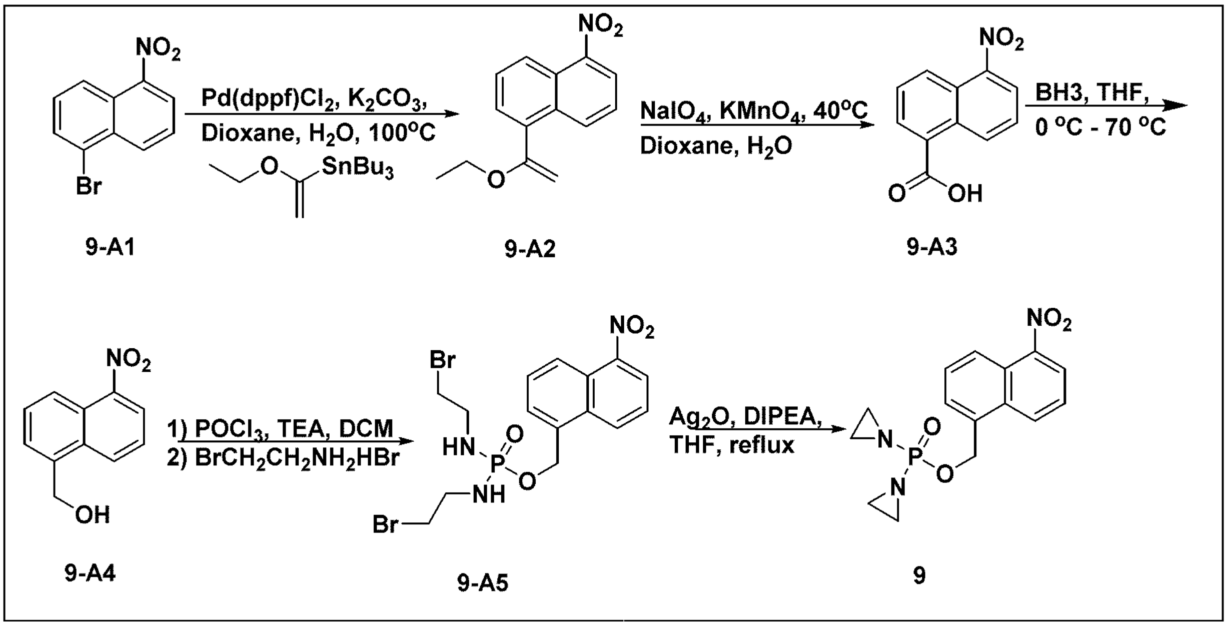

9-A1 9-A2 9-A3 9-A4 9-A5 9

Under nitrogen protection, compound 9-A1 (2.0 g, 7.9 mmol) was dissolved in dioxane (20 ml) and water (20 ml). Then, $PdCl_2$ (dppf) (0.28 g, 0.39 mmol) and $K_2CO_3$ (580 mg, 15.9 mmol) were added. 1-ethoxyvinyltri-n-butyltin (2.20 g, 7.93 mmol) was added, and the mixture was heated to 100 °C and stirred for 5 hours. After the reaction was completed, the mixture was cooled to room temperature. Saturated ammonium chloride aqueous solution (5 ml) was added, and the mixture was extracted with EA (20 ml×3). The organic phase was combined, which was dried with anhydrous sodium sulfate, evaporated the solvent, and separated with column chromatography to obtain compound 9-A2 (1.2 g, 62.1 %), a light yellow oily substance. $^1$H-NMR (400 MHz, $CDCl_3$): $\delta$8.50-8.44 (m, 2H), 8.17 (d, $J$= 7.6 Hz, 1H), 7.67-7.66 (m, 2H), 7.55 (t, $J$ = 8.0 Hz, 1H), 4.56 (d, $J$ = 1.6 Hz, 1H), 4.40 (d, $J$ = 1.6 Hz, 1H), 4.04 (q, $J$ = 7.6 Hz, 2H), 0.92 (t, $J$ = 7.6 Hz, 3H).

Compound 9-A2 (1.2 g, 4.9 mmol) was dissolved in dioxane (20 ml) and water (4 ml). Then, $NaIO_4$ (4.2 g, 19.7 mmol) was added in batches, and the temperature was raised to 35 °C. $KMnO_4$ (390 mg, 2.5 mmol) was added in batches, and the temperature was raised to 40 °C and stirred for 4 hours. After the reaction was completed, the mixture was cooled to room temperature. The system was suction filtered, and the solid was washed with EA. The aqueous phase was extracted with EA (20 ml×3). When the aqueous phase was adjusted to pH=2, there were solid precipitated, which was suction filtered to obtain compound 9-A3 (350 mg, 32.7 %), an off-white solid. $^1$H-NMR (400 MHz, $CD_3OD$): $\delta$9.31 (d, $J$ = 8.8 Hz, 1H), 8.56(d, $J$ = 8.8 Hz, 1H), 8.36(d, $J$ = 7.6 Hz, 1H), 8.22(d, $J$ = 7.6 Hz, 1H), 7.83-7.74(m, 2H). MS: Calculated 217.0, found 216.0([M-H]$^-$).

Under nitrogen protection, compound 9-A3 (350 mg, 1.6 mmol) was dissolved in THF (10 ml), which was cooled to 0 °C. Then, $BH_3$THF solution (4.0 mL, 4.0 mmol, 1 mol/L) was added, and the temperature was raised to 50 °C and stirred for 2 hours. After the reaction was completed, the mixture was cooled to 0 °C. Methanol (5 ml) was added, and the mixture was heated to 70 °C to react for 1 hour. $^1$H-NMR (400 MHz, $CDCl_3$): δ8.48-8.45 (m, 2H), 8.19 (d, $J$ = 7.6 Hz, 1H), 7.69-7.60 (m, 3H), 5.20 (s, 2H), 3.70-3.69 (m, 1H). MS: Calculated 203.1, found 204.1 ([M+H]$^+$).

Under nitrogen protection, compound 9-A4 (150 mg, 0.7 mmol) was dissolved in DCM (10 ml), which was cooled to -40 °C. Then, $POCl_3$ (0.23 g, 1.5 mmol) and TEA (0.19 g, 1.85 mmol) were added successively, and the mixture was stirred at -40 °C for 2 hours. Bromoethylamine hydrobromide (950 mg, 5.92 mmol) and TEA (1.49 g, 14.8 mmol) were added, and the temperature was kept at -40 °C for 3 hours. After the reaction was completed, saturated ammonium chloride aqueous solution (5 ml) was added, which was extracted with DCM (10 ml), evaporated the solvent, and separated with column chromatography to obtain compound 9-A5 (210 mg, 57.3 %), a light yellow oily substance. Compound 9-A5 was directly used in the next reaction after NMR and MS assay.

Under nitrogen protection, compound 9-A5 (280 mg, 0.6 mmol) was dissolved in THF (10 ml). Then, silver oxide (1.0 g, 4.5 mmol) and DIPEA (580 mg, 4.5 mmol) were added, and the mixture was heated to 65 °C and stirred for 3 hours. After the reaction was completed, the mixture was cooled to room temperature. The mixture was suction filtered with diatomite, and the solid was washed with DCM. The original liquid was concentrated, and prepared with

HPLC to obtain compound 09 (30 mg, 16.0 %), a white solid. $^1$H-NMR (400 MHz, $CD_3OD$):δ8.54 (d, *J* = 8.8 Hz, 1H), 8.41 (d, *J* = 8.4 Hz, 1H), 8.23 (d, *J* = 7.6 Hz, 1H), 7.79-7.73 (m, 3H), 5.68 (d, *J* = 8.0 Hz, 2H), 2.19-2.14 (m, 8H). MS: Calculated 333.1, found 334.1($[M+H]^+$).

**Preparation of Example 10: preparation of compound 10**

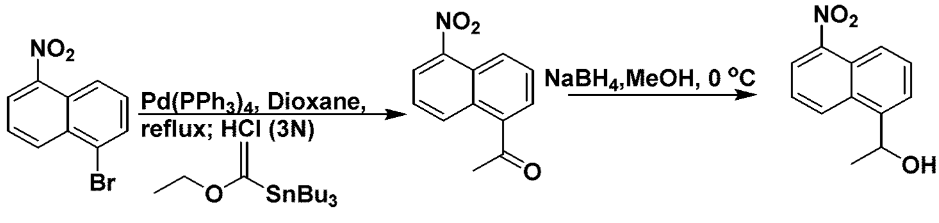


10-A1 10-A2 10-A3

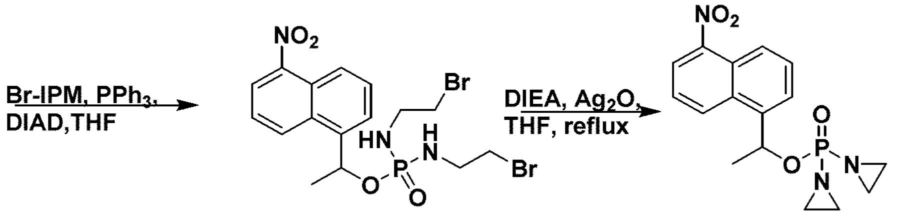


10-A4 10

Under nitrogen protection, compound 10-A1 (3.0 g, 11.9 mmol) and 1-ethoxyvinyltri-n-butyltin (6.0 g, 16.7 mmol) were dissolved in 1, 4-dioxane (75 ml). Tetrakis(triphenylphosphine)palladium (347 mg, 0.3 mmol, Greenchem) was added. $N_2$ was sucked and refilled three times, and the reaction was performed at 105 °C overnight. After the reaction was completed, 3 N of hydrochloric acid (5 mL) was added, and the mixture was stirred for 0.5 hours. Then, the mixture was extracted with EtOAc (30 ml×3), dried, and evaporated dry. A crude product was prepared with column chromatography to obtain compound 10-A2 (1.7 g, 65.4 %), a yellow solid. $^1$H-NMR (400 MHz, $CDCl_3$) δ9.01 (d, *J* = 8.7 Hz, 1H), 8.65 (d, *J* = 8.8 Hz, 1H), 8.21 (d, *J* = *7.0* Hz, 1H), 8.06 (d, *J* = 6.8 Hz, 1H), 7.77-7.73 (m, 1H), 7.69 - 7.65 (m, 1H), 2.79 (s, 3H). MS: Calculated 215.1, found 216.2 ($[M+H]^+$).

Under nitrogen protection, compound 10-A2 (1.2 g, 5.6 mmol) was dissolved in MeOH (12 ml). After cooled to 0 °C, $NaBH_4$ (253 mg, 6.7 mmol) was added in batches. Next, the temperature was kept at 0 °C for 0.5 hours to react. After the reaction was completed, $H_2O$ (10 ml) was added, which was extracted with DCM (10 ml ×3), washed with 10% of citric acid aqueous solution (25 ml), and extracted with DCM (10 ml). The organic phase was washed with water (10 ml ×3), washed with saturated brine (10 ml), and the solvent was evaporated. A crude product was prepared with column chromatography to obtain compound 10-A3 (1.0 g, 82.0%), a light yellow. $^1$H-NMR (400 MHz, $CDCl_3$) δ8.46 (d, *J* = 8.4 Hz, 1H), 8.38 (d, *J* = 8.8 Hz, 1H), 8.13 (d, *J* = 7.6 Hz, 1H), 7.82 (d, *J* = 7.2 Hz, 1H), 7.72-7.67 (m, 1H), 7.58 (t, *J* = 8.2 Hz, 1H), 5.69-5.64 (q, *J* = 6.4 Hz, 1H), 1.69 (d, *J*= 6.4 Hz, 3H).

Under nitrogen protection, compound 10-A3 (200 mg, 0.9 mmol), Br-IPM (428 mg · 1.4 mmol), and triphenylphosphine (561 mg, 1.8 mmol) were added to anhydrous THF (10 ml). After cooled to 0 °C, DIAD (372 mg, 1.8 mmol) was added dropwise and the temperature was kept for 1 hour to react. After the reaction was completed, the original liquid was concentrated. A crude product was prepared with column chromatography to obtain compound 10-A4 (234 mg, 51.1 %), a light yellow liquid. Compound 10-A4 was directly used in the next step after NMR and MS assay.

Under nitrogen protection, compound 10-A4 (250 mg, 0.5 mmol), DIEA (318 mg, 2.4 mmol), and $Ag_2O$ (569 mg, 2.5 mmol) were added to THF (30 ml) sequentially. The mixture was stirred under refluxed overnight. After the reaction was completed, the mixture was suction filtered with diatomite. The filtrate was concentrated, which was prepared with HPLC to obtain compound 10 (18 mg, 7.2 %), a yellow oily substance. $^1$H-NMR (400 MHz, $CD_3OD$): δ8.57 (d, *J* = 8.8 Hz, 1H), 8.32 (d, *J* = 8.8 Hz, 1H), 8.18 (d, *J* = 7.2 Hz, 1H), 7.86 (d, *J* = 7.2 Hz, 1H), 7.79-7.70 (m, 2H), 6.49-6.29 (m, 1H), 2.23-2.11 (m, 4H), 2.06-1.99 (m, 4H), 1.81 (d, *J*= 6.4 Hz, 3H). MS: Calculated 347.1, found 370.0 ($[M+Na]^+$).

**Preparation of Example 11: preparation of compound 11**

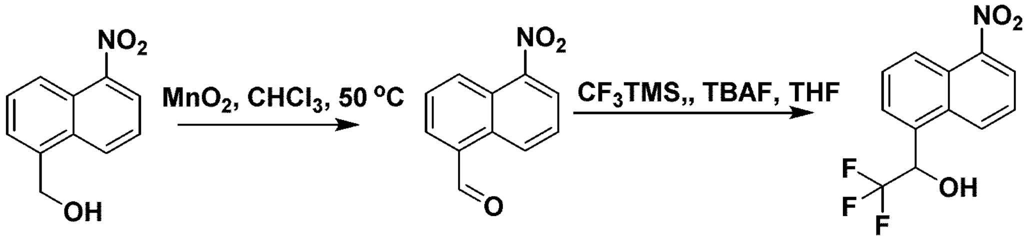


11-A1 11-A2 11-A3

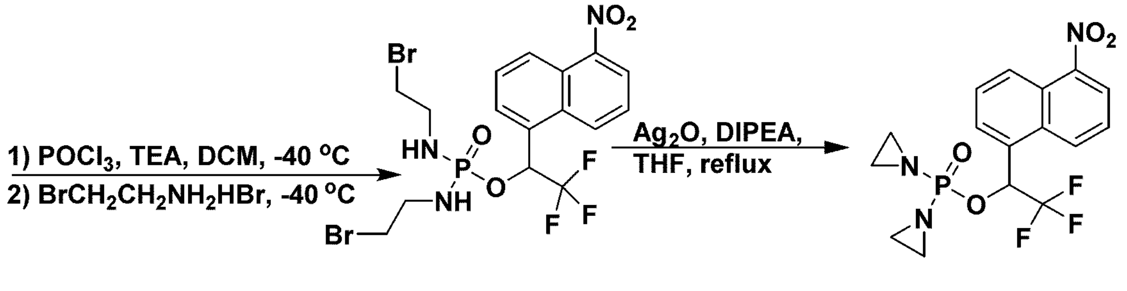


11-A4 11

Under nitrogen protection, compound 11-A1 (420 mg, 2.07 mmol) was dissolved in chloroform (8 ml). Then, $MnO_2$ (2.7 g, 31.03 mmol) was added, and the temperature was raised to 50 °C for 4 hours to react. After cooled to 0 °C, the mixture was suction filtered. The original liquid was evaporated dry directly to obtain compound 11-A2 (280 mg, 67.3 %). The crude product was used in the next reaction directly.

Under nitrogen protection, compound 11-A2 (280 mg, 1.39 mmol) was dissolved in THF (10 ml). $CF_3TMS$ (296 mg, 2.09 mmol) was added, and the mixture was cooled to 0 °C. Next, TBAF (0.27 mL, 1.0 M in THF 0.27 mmol) was added dropwise, and the mixture was stirred at 0 °C for 2 hours. 3 N of HCl (5 ml) was added, and the mixture was stirred at room temperature for 0.5 hours. Then, the mixture was extracted with EA (10 ml×3). Compound 11-A3 (180 mg, 47.7 %, with TBAF residue), a light yellow oily substance, was obtained with column chromatography. Compound 11-A3 was directly used in the reaction after NMR and MS assay.

Under nitrogen protection, compound 11-A3 (120 mg, 0.44 mmol) was dissolved in DCM (5 ml), and the mixture was cooled to -40 °C. Then, $POCl_3$ (135 mg, 0.88 mmol) and TEA (111 mg, 1.1 mmol) were added, and the mixture was stirred at -40 °C for 2 hours. Bromoethylamine hydrobromide (721 mg, 3.52 mmol) and TEA (404 mg, 4.0 mmol) were added. After the reaction was completed, saturated ammonium chloride aqueous solution (3 ml) was added. The mixture was extracted with DCM (10 ml ×3). Compound 11-A4 (105 mg, 42.3%), a light yellow oily substance, was obtained with column chromatography. $\delta$8.58 (d, $J$ = 8.4 Hz, 1H), 8.47 (d, $J$ = 8.8 Hz, 1H), 8.21 (d, $J$ = 7.6 Hz, 1H), 7.96 (d, $J$ = 6.8 Hz, 1H), 7.81-7.77 (m, 1H), 7.72-7.67 (m, 1H), 6.58-6.51 (m, 1H), 3.56-3.44 (m, 4H), 3.16-2.87 (6 H).

Under nitrogen protection, compound 11-A4 (105 mg, 0.19 mmol) was dissolved in THF (8 ml). Then, silver oxide (352 mg, 1.48 mmol) and DIPEA (190 mg, 1.48 mmol) were added, and the mixture was heated to reflux to react for 3 hours. After the reaction was completed, the mixture was cooled to room temperature. The mixture was suction filtered with diatomite, and washed with DCM. The original liquid was evaporated dry. A pure product, compound 11 (32.4 mg, 43.3 %), an off-white solid, was obtained with HPLC. $^1$H-NMR (400 MHz, $CD_3OD$) $\delta$8.62 (d, $J$ = 8.8 Hz, 1H), 8.50 (d, $J$ = 8.8 Hz, 1H), 8.23 (d, $J$ = 6.8 Hz, 1H), 8.06 (d, $J$ = 7.2 Hz, 1H), 7.90-7.88 (m, 1H), 7.80-7.76 (m, 1H), 6.82-6.80 (m, 1H), 2.27-2.01 (m, 8 H). MS: Calculated 401.1, found 402.1 ($[M+H]^+$).

**Preparation of Example 12: preparation of compound 12**

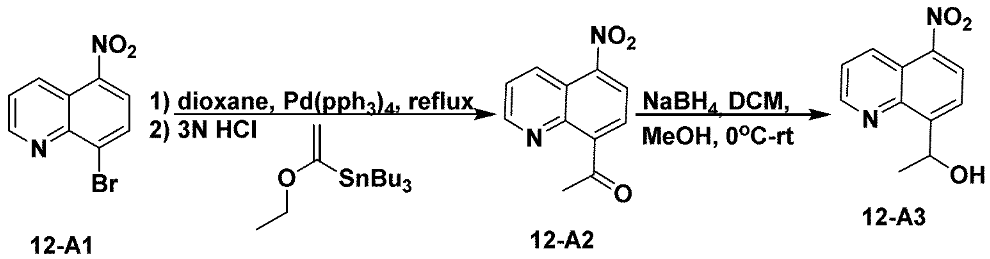


12-A1 12-A2 12-A3

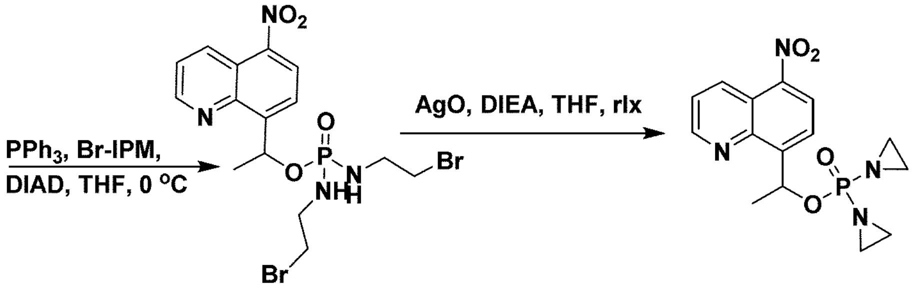


12-A4 12

Under nitrogen protection, compound 12-A1 (1.5 g, 5.93 mmol) and 1-ethoxyvinyltri-n-butyltin (3.0 g, 8.30 mmol) were added to dioxane (30 ml). After nitrogen was sucked and refilled three times, $Pd(PPh_3)_4$ (170 mg, 0.15 mmol) was added. Nitrogen was sucked and refilled three times again, and the mixture was heated to reflux overnight. After the reaction was completed, the mixture was cooled to room temperature. Next, 3N of HCl (30 ml) was added. After stirred for 4 hours, the mixture was extracted with DCM (30 mL×3), washed with water (10 mL), washed with brine (10 mL), dried with anhydrous sodium sulfate, evaporated the solvent, and separated with column chromatography to obtain compound 12-A2 (610 mg , 47.6 %), a light yellow solid. $^1$H-NMR (400 MHz, $CDCl_3$): $\delta$9.07- 9.06 (m, 1H), 8.98 (dd, *J* = 8.8, 1.6 Hz, 1H), 8.36 (d, *J* = 8.0 Hz, 1H), 7.88 (d, *J* = 7.6 Hz, 1H), 7.70 (dd, *J* = 8.8, 4.0 Hz, 1H), 2.89 (s, 3H). MS: Calculated 216.1, found 217.2 ($[M+H]^+$).

Compound 12-A2 (600 mg, 2.78 mmol) was dissolved in MeOH (15 mL) and DCM (5 mL). After cooled to 0 °C, $NaBH_4$ (126 mg, 3.33 mmol) was added in batches, and the reaction was completed 30 minutes later. $H_2O$ (15 mL) was added dropwise to quench, which was extracted with DCM (20 mL×3). The organic phase was sequentially washed with water (5 mL), washed with brine (5 mL), dried with anhydrous sodium sulfate, and concentrated to obtain a crude product, compound 12-A2 (342 mg, yield 56.5 %), a light yellow oily substance.

Under nitrogen protection, compound 12-A3 (340 mg, 1.56 mmol), Br-IPM (1.2 g, 3.90 mmol), and $PPh_3$ (1.0 g, 3.82 mmol) were added to anhydrous THF (20 mL). After cooled to 0 °C, DIAD (896 mg, 3.90 mmol) was slowly added dropwise. The reaction was completed after the temperature was kept at 0 °C for 30 minutes. Water (10 mL) was added dropwise, which was extracted with DCM (30 mL×3). The organic phase was washed with water (5 mL), washed with saturated brine (5 mL), dried with anhydrous sodium sulfate, evaporated the solvent, and separated with column chromatography to obtain compound 12-A4 (350 mg, 44.0 %), a yellow oily substance. $^1$H-NMR (400 MHz, $CDCl_3$): $\delta$9.02-8.98 (m, 2H), 8.39 (d, *J* = 8.0 Hz, 1H), 7.97 (d, *J* = 8.0 Hz, 1H), 7.65 (dd, *J* = 8.8, 4.4 Hz, 1H), 6.76-6.73 (m, 1H), 3.47-3.32 (m, 8H), 1.76 (d, *J* = 6.4 Hz, 3H). MS: Calculated 507.9, 509.9, 511.9, found 509, 511.0, 513.0 ($[M+H]^+$).

Under nitrogen protection, compound 12-A4 (150 mg, 0.29 mmol) was dissolved in THF (10 mL). Then, silver oxide (341 mg, 1.47 mmol) and DIEA (190 mg, 1.47 mmol) were added, and the mixture was heated to reflux and reacted overnight. The mixture was suction filtered with diatomite, which was washed with DCM. The original liquid was concentrated, and prepared with HPLC to obtain a pure product, compound 12 (30 mg, 29.4%), a colorless oily substance. $^1$H-NMR (400 MHz, $CDCl_3$): $\delta$9.03 - 9.01 (m, 2H), 8.41 (d, *J* = 8.0 Hz, 1H), 8.06 (d, *J* = 8.0 Hz, 1H), 7.65 (dd, *J* = 8.4, 4.4 Hz, 1H), 7.00-6.95 (m, 1H), 2.23-2.07 (m, 8H), 1.77 (d, *J* = 6.4 Hz, 3H). MS: Calculated 348.1, found 349.1 ($[M+H]^+$).

**Preparation of Example 13: preparation of compound 13**

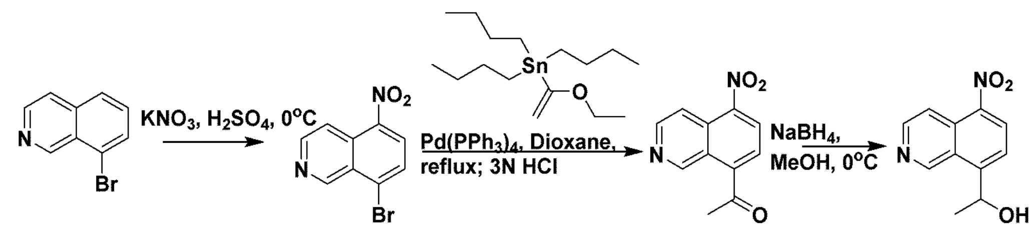


13-A1 13-A2 13-A3 13-A4

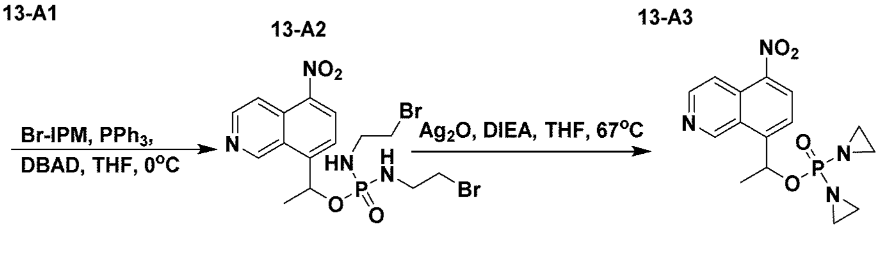


13-A5 13

Compound 13-A1 (10 g, 48.0 mmol) was dissolved in concentrated sulfuric acid (50 mL). After cooled to 0 °C, potassium nitrate (5.8 g, 57.7 mmol) was added in batches. The reaction was completed after heated to at 25 °C to react for 1 hour. The mixture was added to ice water (300 mL), which was extracted with DCM (60 mL×6), washed with water (30 mL×2), dried with anhydrous sodium sulfate, and evaporated dry to obtain compound 13-A2 (8.0 g, 63.3%), a yellow solid. $^1$H-NMR (400 MHz, $CDCl_3$): δ9.79 (s, 1H), 8.85 (d, *J* = 6.4 Hz, 1H), 8.50 (d, *J* = 6.4 Hz, 1H), 8.39 (d, *J* = 8.4 Hz, 1H), 8.00 (d, *J* = 8.4 Hz, 1H). MS: Calculated 252.0, 254.0, found 253.0, 255.0 ($[M+H]^+$).

Under nitrogen protection, compound 13-A2 (1.5 g, 5.9 mmol) and 1-ethoxyvinyltri-n-butyltin (3.0 g, 8.3 mmol) were dissolved in dioxane (30 mL). After nitrogen was sucked and refilled three times, tetrakis(triphenylphosphine)palladium (173 mg, 0.15 mmol) was added. Then, nitrogen was sucked and refilled three times. After the mixture was heated to reflux for reacting overnight, the disappearance of raw materials was monitored by liquid chromatography. After cooling to room temperature, saturated ammmonium chloride aqueous solution (20 mL) was added. The mixture was extracted with ethyl ester (15 mL×3), and evaporated the solvent. Then, 3 N of hydrochloric acid (20 ml) was added. After the mixture was stirred at room temperature for 2 hours, the end of the reaction was monitored by liquid chromatography. The mixture was extracted with DCM (15 mL×3). The organic phase was washed with brine (10 mL), dried with anhydrous sodium sulfate, and evaporated the solvent. A crude product was separated with column chromatography to obtain compound 13-A3 (600 mg, 46.8%), a yellow solid. $^1$H-NMR (400 MHz, $CDCl_3$): δ9.90 (s, 1H), 8.83 (d, *J* = 6.0 Hz, 1H), 8.48 (d, *J* = 8.0 Hz, 1H), 8.40 (d, *J* = 6.0 Hz, 1H), 7.98 (d, *J* = 8.0 Hz, 1H), 2.83 (s, 3H). MS: Calculated 216.1, found 217.1 ($[M+H]^+$).

Compound 13-A3 (600 mg, 2.8 mmol) was dissolved in methanol (12 mL). After cooled to 0 °C, sodium borohydride (105 mg, 2.8 mmol) was added in batches. After the addition, the end of the reaction was monitored by thin-layer chromatography. Water (8 mL) was added to quench, which was extracted with DCM (15 mL×2), washed with 5 % of citric acid aqueous solution (8 mL×2), washed with water (8 mL×2), washed with brine (8 mL), dried with anhydrous sodium sulfate, and evaporated the solvent to obtain compound 13-A4 (320 mg, 52.8%), a yellow solid. $^1$H-NMR (400 MHz, $CDCl_3$): δ9.68 (s, 1H), 8.77 (d, *J* = 5.9 Hz, 1H), 8.54-8.51 (m, 2H), 7.97 (d, *J* = 8.1 Hz, 1H), 5.89 (m, 1H), 1.73 (d, *J* = 6.4 Hz, 3H). MS: Calculated 218.1, found 219.1 ($[M+H]^+$).

Under nitrogen protection, compound 13-A4 (300 mg, 1.4 mmol) and Br-IPM (1.1 g, 3.4 mmol) were dissolved in anhydrous THF. Triphenylphosphine (900 mg, 3.4 mmol) was added, and the mixture was cooled to 0 °C. Di-tert-butyl azodicarboxylate (790 mg, 3.4 mmol) was added, and 30 minutes later, the end of the reaction was monitored by liquid chromatography. The mixture was evaporated to remove the solvent and separated with column chromatography to obtain compound 13-A5 (300 mg, 42.8%, content 80%), a yellow oily substance. Compound 13-A5 was directly used in the next reaction after NMR and MS assay.

Under nitrogen protection, compound 13-A5 (200 mg, 0.39 mmol) was dissolved in THF (5 mL). DIEA (254 mg, 2.0 mmol) and silver oxide (273 mg, 1.2 mmol) were added. The mixture was heated to reflux and reacted overnight to end. After cooling to room temperature, the mixture was suction filtered with diatomite. The original liquid was evaporated dry, and neutrally prepared with high performance liquid chromatography to obtain compound 13 (27 mg, 19.8%), a brown liquid. $^1$H-NMR (400 MHz, $CDCl_3$): δ9.70 (s, 1H), 8.80 (d, *J* = 6.4 Hz, 1H), 8.53 - 8.50 (m, 2H), 7.94 (d, *J* = 8.0 Hz, 1H), 6.57-6.50 (m, 1H), 2.25-2.04 (m, 8H), 1.86 (d, *J*= 6.4 Hz, 3H). MS: Calculated 348.1, found 349.1 ($[M+H]^+$).

**Preparation of Example 14: preparation of compound 14**

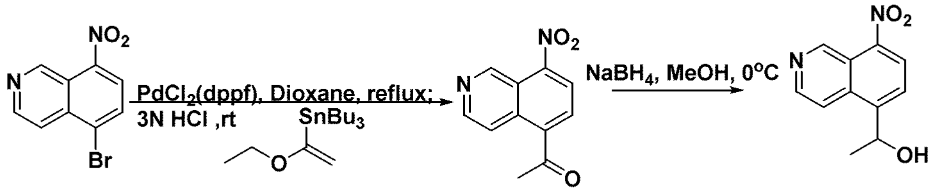


14-A1 14-A2 14-A3

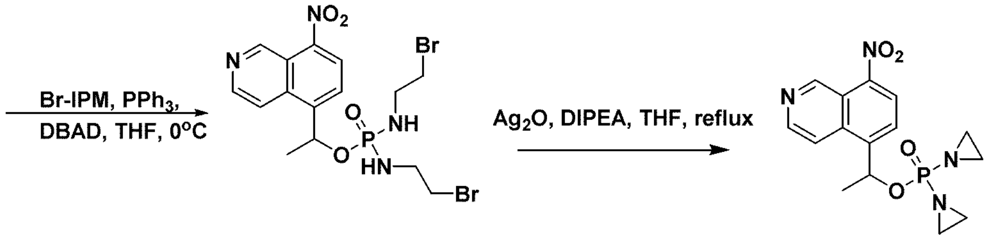


14-A4 14

Under nitrogen protection, compound 14-A1 (2.0 g, 7.90 mmol) was dissolved in dioxane (20 ml). Then, Pd(dppf)$Cl_2$ (0.28g, 0.39 mmol) was added, and 1-ethoxyvinyltri-n-butyltin (2.8 g, 7.93 mmol) was added. The mixture was heated to 100°C and stirred for 5 hours. After the reaction was completed, the mixture was cooled to room temperature. 3N of HCl solution (15 mL) was added, and the mixture was stirred for 30 minutes. The mixture was extracted with EA (20mL×3) three times. The organic phase was combined, and separated with column chromatography to obtain compound 14-A2 (1.1g, 64.7%, content about 80%), a light yellow solid. MS: Calculated 216.1, found 217.1 ($[M+H]^+$).

Compound 14-A2 (1.1 g, 5.1 mmol) was dissolved in MeOH (10 mL). At 0 °C, $NaBH_4$ (230 mg, 6.1 mmol) was added in batches. The mixture was stirred at 0 °C for 0.5 hours. After the reaction was completed, ice water (5 mL) was added to quench, and extracted with EtOAc (10mL×3). The mixture was separated with column chromatography to obtain compound 14-A3 (540 mg, 47.0%), a light yellow oily substance. $^1$H-NMR (400 MHz, $CD_3OD$): $\delta$10.01 (s, 1H), 8.74 (d, $J$ = 6.0 Hz, 1H), 8.32 (d, $J$ = 8.0 Hz, 1H), 8.05 (d, $J$ = 8.4 Hz, 1H), 7.95 (d, $J$ = 6.0 Hz, 1H), 5.69-5.67 (m, 1H), 1.68 (d, $J$ = 6.4 Hz, 3H). MS: Calculated 218.1, found 219.1 ($[M+H]^+$).

Under nitrogen protection, compound 14-A3 (190 mg, 0.9 mmol) and Br-IPM (664 mg, 2.1 mmol) were dissolved in anhydrous THF. Triphenylphosphine (550 mg, 2.1 mmol) was added, which was cooled to 0 °C. Di-tert-butyl azodicarboxylate (484 mg, 2.1 mmol) was added to react for 3 hours, and the end of the reaction was monitored by liquid chromatography. The solvent was evaporated dry, and compound 14-A4 (180 mg, 40.5%), a yellow oily substance, was obtained with column chromatography.

Under nitrogen protection, compound 14-A4 (180 mg, 0.4 mmol) was dissolved in THF (10mL). Then, silver oxide (407 mg, 1.8 mmol) and DIPEA (226 mg, 1.8 mmol) were added, and the mixture was heated to reflux to react for 3 hours. After the reaction was completed, the mixture was cooled to room temperature, and suction filtered with diatomite. The solid was washed with DCM. The original liquid was concentrated, and prepared with HPCL to obtain a pure product, compound 14 (33 mg, 27.0 %), an off-white solid.$^1$H-NMR (400 MHz, $CD_3OD$): $\delta$9.90 (s, 1H), 8.74 (d, $J$= 6.0 Hz, 1H), 8.48 (d, $J$= 8.0 Hz, 1H), 8.28 (d, $J$ = 6.0 Hz, 1H), 8.15 (d, $J$ = 8.0 Hz, 1H), 6.45-6.42 (m, 1H), 2.28-2.11 (m, 8H), 1.83 (d, $J$ = 6.4 Hz, 3H). MS: Calculated 348.1, found 349.1($[M+H]^+$).

**Preparation of Example 15: preparation of compound 15**

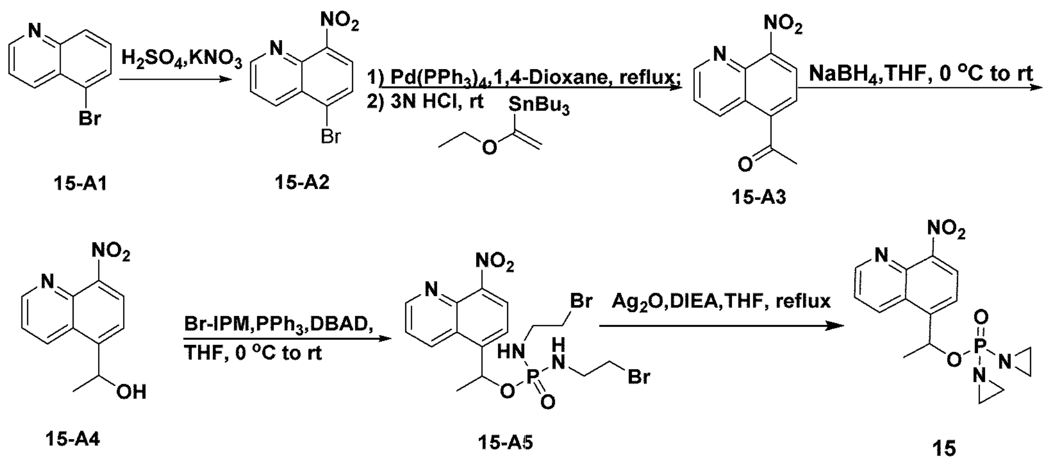


15-A1 15-A2 15-A3

15-A4 15-A5 15

Compound 15-A1 (8.0 g, 38.4 mmol) was added to concentrated sulfuric acid (30 mL). After cooled to 0 °C, potassium nitrate (4.7 g, 46.1mmol) was added. The mixture was stirred at room temperature for 1.5 hour, and the reaction was completed. The reaction system was slowly poured into ice water, and the mixture was stirred for 30 minutes. The mixture was extracted with DCM (50 mL×3), washed with water (15mL×3), washed with saturated brine (10 mL), dried with anhydrous sodium sulfate, and evaporated the solvent to obtain compound 15-A2 (7.2 g, 74.2%), a light yellow solid. $^1$H-NMR (400 MHz, DMSO-*d6*): δ9.11-9.10 (m, 1H), 8.67-8.65 (m, 1H), 8.24 (d, *J* = 8.0 Hz, 1H), 8.16 (d, *J* = 8.0 Hz, 1H), 7.90-7.87 (m, 1H). MS: Calculated: 252.0, 254.0, found 253.0, 255.0 ([M+H]$^+$).

Under nitrogen protection, compound 15-A2 (1.5g, 5.93 mmol) dissolved in 1,4-dioxane (30mL). 1-ethoxyvinyltri-n-butyltin (3.0g, 8.30mmol) was added, and tetrakis(triphenylphosphine)palladium (206mg, 0.178mmol) was added. The mixture was reacted at 100 °C overnight. After the reaction was completed, the mixture was cooled to room temperature naturally. 3 N of hydrochloric acid (4 ml) was added, and the mixture was stirred for 1 hour. The mixture was extracted with EA (15mL ×3), washed with water (5 mL), washed with brine (5 mL), dried with anhydrous sodium sulfate, spun off the solvent, and separated with column chromatography to obtain compound 15-A3 (900mg, 60%), a yellow solid. $^1$H-NMR (400 MHz, DMSO-*d6*): δ9.07 - 9.06 (dd, *J* = 4.0, 1.6 Hz, 1H), 9.00 (dd, *J* = 8.8, 1.6 Hz, 1H), 8.37 (s, 2H), 7.82 (dd, *J* = 8.8, 4.0 Hz, 1H), 2.78 (s, 3H). MS: Calculated: 216.1, found: 217.1([M+H]$^+$).

Compound 15-A3 (800 mg, 3.70 mmol) was dissolved in THF (5 mL). After cooled to 0 °C, $NaBH_4$ (280 mg, 7.40 mmol) was added in batches. 30 minutes later, the reaction was completed. At 0 °C, $H_2O$ (5 mL) was added dropwise, and the mixture was stirred for 20 minutes. The mixture was extracted with DCM (10 mL ×3), washed with water (5 mL), washed with brine (5 mL), dried with anhydrous sodium sulfate, evaporated the solvent, and separated with column chromatography to obtain compound 15-A4 (400 mg, 49.6%), a yellow solid. $^1$H-NMR (400 MHz, DMSO-*d6*): δ9.03 (dd, *J* = 4.0, 1.2 Hz, 1H), 8.82 (dd, *J*= 8.8, 1.2 Hz, 1H), 8.23 (d, *J*= 7.6 Hz, 1H), 7.84 (d, *J*= 8.0 Hz, 1H), 7.73 (dd, *J* = 8.8, 4.0 Hz, 1H), 5.67 (d, *J* = 4.4 Hz, 1H), 5.60-5.48 (m, 1H), 1.48 (d, *J* = 6.4 Hz, 3H). MS: Calculated 218.1, found 219.2 ([M+H]$^+$).

Under nitrogen protection, compound 15-A4 (300 mg, 1.37 mmol) and Br-IPM (1.06 g, 3.43 mmol) were added to anhydrous THF. Triphenylphosphine (900 mg, 3.43 mmol) was added, and the mixture was cooled to 0 °C. Then, a THF solution (2mL) of di-tert-butyl azodicarboxylate (790 mg, 3.43 mmol) was added. The mixture was kept at 0 °C for 0.5 hours, and then the mixture was stirred at room temperature for 2.5 hours. After the reaction was completed, water (5 mL) was added dropwise at 0 °C. The mixture was extracted with DCM (5 mL×3), washed with water (2 mL×2), dried with anhydrous sodium sulfate, and evaporated the solvent to obtain compound 15-A5 (450 mg, 64.2%), a yellow solid. $^1$H-NMR (400 MHz, $CDCl_3$): δ9.07 (dd, *J* = 4.0, 1.2 Hz, 1H), 8.63 (dd, *J* = 8.8, 1.2 Hz, 1H), 8.00 (d, *J* = 7.8 Hz, 1H), 7.77 (d, *J* = 7.8 Hz, 1H), 7.61 (dd, *J* = 8.7, 4.1 Hz, 1H), 6.24-6.21 (m, 1H), 3.53-3.23 (m, 8H), 1.77 (d, *J*= 6.4 Hz, 3H). MS: Calculated 507.9, 509.9, 511.9, found 509.0, 511.0, 513.0 ([M+H]$^+$).

Compound 15-A5 was dissolved in THF (10 mL). $Ag_2O$ (454 mg, 1.96 mmol) and DIEA (253 mg, 1.96 mmol) were added. The mixture was heated to reflux, and the reaction was completed 2 hours later. After cooling to room temperature, the mixture was suction filtered with diatomite. The solid was washed with THF (3mL×3). The original liquid was concentrated and separated with HPLC to obtain compound 15 (19 mg, 13.9 %), a white solid. $^1$H-NMR (400 MHz, $CDCl_3$): δ9.08-9.07 (dd, *J* = 4.4, 1.6 Hz, 1H), 8.57 (dd, *J* = 8.8, 1.6 Hz, 1H), 8.01 (d, *J* = 7.6 Hz, 1H), 7.81 (d, *J* = 8.0 Hz, 1H), 7.60 (dd, *J* = 8.8, 4.4 Hz, 1H), 6.38-6.32 (m, 1H), 2.23-1.97 (m, 8H), 1.79 (d, *J*= 6.8 Hz, 3H). MS: Calculated 348.1, found 349.1 ([M+H]$^+$).

**Preparation of Example 16: preparation of compound 16**

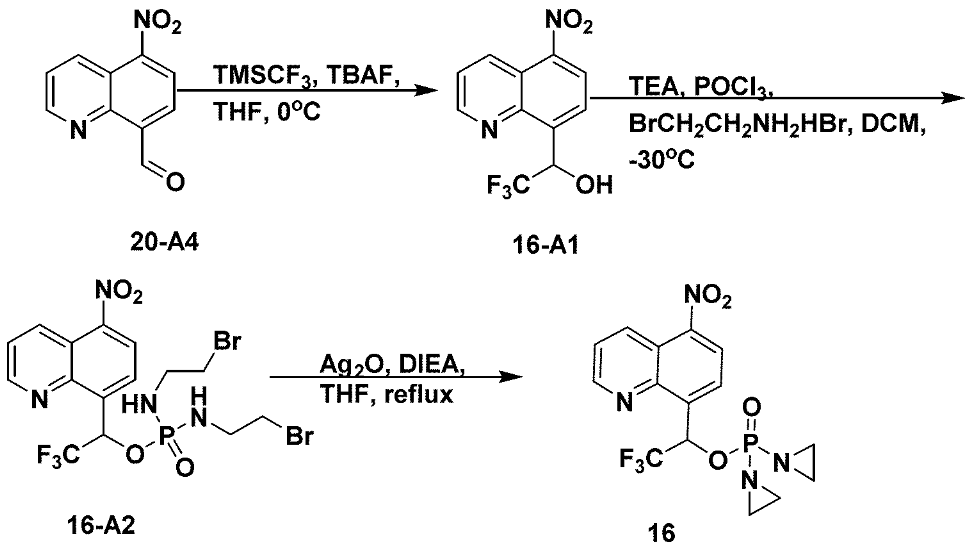


20-A4 16-A1

16-A2 16

Under nitrogen protection, compound 20-A4 (200 mg, 1.0mmol) was dissolved in THF (5 mL). Then, $TMSCF_3$ (226 mg, 1.5 mmol) was added. At 0 °C, a THF solution (0.02 mL, 1.0 M in THF, 0.02 mmol) of TBAF was added, and the reaction was completed 2 hours later. 3 N of hydrochloric acid (4 mL) was added, and the mixture was stirred for 15 minutes. The mixture was extracted with DCM (10 mL×3), washed with water (5 mL×2), dried with anhydrous sodium sulfate, evaporated the solvent, and purified with column chromatography to obtain compound 16-A1 (230 mg, 85.4%), a yellow solid. $^1$H-NMR (400 MHz, $CDCl_3$): $\delta$9.10-9.01 (m, 2H), 8.38 (d, *J= 7.8* Hz, 1H), 8.13 (m, 1H), 7.81 (d, *J* = 7.8 Hz, 1H), 5.60 (m, 1H). MS: Calculated 272.0, found 273.1 ([M+H]$^+$).

Under nitrogen protection, compound 16-A1 (100 mg, 0.4 mmol) and $POCl_3$ (113 mg, 0.7 mmol) were dissolved in DCM (2 mL). TEA (93 mg, 0.9 mmol) was added dropwise. One hour later, the disappearance of raw materials was monitored by liquid chromatography. Then, 2-bromoethylamine hydrobromide (614 g, 2.9 mmol) was added, and TEA (297 mg, 2.9 mmol) was added dropwise. 0.5 hours later, the end of the reaction was monitored by liquid chromatography. Water (3 mL) was added to quench. The mixture was extracted with DCM (8 mL×2), washed with water (4 mL), dried with anhydrous sodium sulfate, evaporated the solvent, and separated by column chromatography to obtain compound 16-A2 (100 mg, 48.2%), a yellow oily substance. $^1$H-NMR (400 MHz, $CDCl_3$): $\delta$9.10 (s, 1H), 9.00 (d, *J*= 8.8 Hz, 1H), 8.43 (d, *J* = 7.6 Hz, 1H), 8.13 (d, *J*= 7.2 Hz, 1H), 7.73 - 7.70 (m, 1H), 7.49 - 7.47 (m, 1H), 3.52-3.05 (m, 10H). MS: Calculated 561.9, 563.9, 565.9, found 563.0, 565.0, 567.0 ([M+H]$^+$).

Under nitrogen protection, compound 16-A2 (100 mg, 0.18 mmol) was dissolved in anhydrous THF (5mL). Silver oxide (123 mg, 0.53 mmol) and DIEA (115 mg, 0.89 mmol) were added. The mixture was heated to reflux to react for 1 hour, and suction filtered with diatomite. The original liquid was evaporated dry and prepared with HPCL to obtain compound 16 (10 mg, 14.1%), a white solid. $^1$H-NMR (400 MHz, $CD_3OD$): $\delta$9.11 (dd, *J* = 4.0, 1.2 Hz, 1H), 8.97 (dd, *J* = 8.8, 1.6 Hz, 1H), 8.52 (d, *J* = 8.0 Hz, 1H), 8.25 (d, *J* = 8.0 Hz, 1H), 7.82 (dd, *J* = 8.8, 4.0 Hz, 1H), 7.61-7.54 (m, 1H), 2.26-2.05 (m, 8H). MS: Calculated 402.1, found 403.1 ([M+H]$^+$).

**Preparation of Example 17: preparation of compound 18**

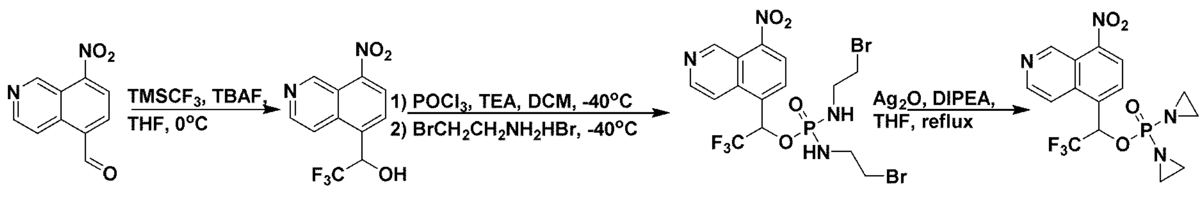


21-A3 18-A1 18-A2 18

Under nitrogen protection, compound 21-A3 (300 mg, 1.5 mmol) was dissolved in anhydrous THF (6 mL). Then, (trifluoromethyl)trimethylsilane (318 mg, 2.2 mmol) was added dropwise. After cooled to 0 °C, TBAF (0.03 mL, 1M in THF, 0.03 mmol) was added dropwise. The mixture was kept at 0 °C for 1.5 hour, and the reaction was completed. 3 N of hydrochloric acid (1.2 mL) was added, and the mixture was raised to room temperature naturally and stirred for 1 hour. Then, water (5 mL) was added. The mixture was extracted with DCM (10 mL×3), washed with water (5 mL×3), dried with anhydrous sodium sulfate, and evaporated the solvent to obtain a crude product, compound 18-A1 (180 mg, 44.6%, content 70%), an off-white solid. Compound 18-A1 was used in the next reaction.

Under nitrogen protection, $POCl_3$ (118 mg, 0.78 mmol) was dissolved in DCM (4 mL). Compound 18-A1 (105 mg, 0.39 mmol) was added, and the mixture was cooled to - 40 °C. A DCM solution (1 ml) of TEA (250 mg, 32.5 mmol) was added dropwise, and the mixture was kept at -40 °C to react. The progress of the reaction was monitored by HPLC, and raw materials were disappeared after reacting for 3 hours. At the same temperature, 2-bromoethylamine hydrobromide (627 mg, 3.1 mmol) and TEA (303 mg, 3.1 mmol) were added. The mixture was kept at -40 °C for 30 minutes, and the reaction was completed. $H_2O$ (2 mL) was added, and the mixture was stirred for 15 minutes. The mixture was extracted with DCM (8 mL×3), washed with water (5 mL×2), dried with anhydrous $Na_2SO_4$, evaporated the solvent, and purified with column chromatography to obtain compound 18-A2 (120 mg, 55.8%, content about 73%), a yellow oily substance. Compound 18-A2 was directly used in the next reaction after MNR and MS assay.

Compound 18-A2 (120 mg, 0.21 mmol) was dissolved in THF (7 mL). $Ag_2O$ (246 mg, 1.1 mmol) and DIEA (142 mg, 1.1 mmol) were added. The mixture was heated to reflux and reacted for 5 hours. After cooling to room temperature, the mixture was suction filtered with diatomite. The solid was washed with THF (3 mL×3). The original liquid was evaporated to remove the solvent and separated with HPLC to obtain compound 18 (15 mg, 17.9 %), an off-white solid. $^1$H-NMR (400 MHz, $CD_3OD$): $\delta$9.89 (s, 1H), 8.80 (d, *J* = 6.0 Hz, 1H), 8.53 (d, *J* = 8.4 Hz, 1H), 8.41-8.36 (m, 2H), 6.89-6.85 (m, 1H), 2.31-2.00 (m, 8H). MS: Calculated 402.1, found 403.1 ([M+H]$^+$).

**Preparation of Example 18: preparation of compound 19**

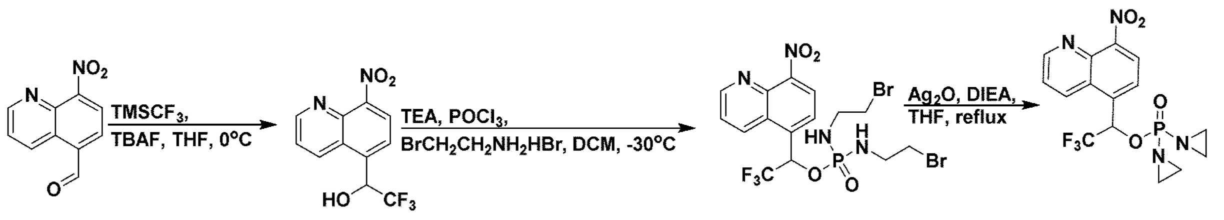


19-A1 19-A2 19-A3 19

Under nitrogen protection, compound 19-A1 (500 mg, 2.5 mmol) was dissolved in anhydrous THF (10mL). Then, (trifluoromethyl)trimethylsilane (530mg, 3.7 mmol) was added dropwise. After cooled to 0 °C, TBAF (0.05mL, 1 M in THF, 0.05 mmol) was added dropwise. The mixture was kept at 0 °C for 1.5 hours, and the reaction was completed. 3 N of hydrochloric acid (2 mL) was added dropwise. The mixture was raised to room temperature and stirred for 1 hour. Then, water (5 mL) was added. The mixture was extracted with DCM (10 mL×3), washed with water (5 mL×3), dried with anhydrous sodium sulfate, evaporated the solvent, and separated with column chromatography to obtain compound 19-A2 (400 mg, 59.3%), an off-white solid. $^1$H-NMR (400 MHz, $CD_3OD$): $\delta$ 8.99 (dd, *J* = 4.0, 1.6 Hz, 1H), 8.88 (d, *J* = 8.8 Hz, 1H), 8.13 (d, J = 8.0 Hz, 1H), 8.04 (d, *J* = 8.0 Hz, 1H), 7.72 (dd, *J* = 8.8, 4.0 Hz, 1H), 5.99-5.94 (m, 1H). MS: Calculated 272.0, found 273.1 ([M+H]$^+$).

Under nitrogen protection, $POCl_3$ (394 mg, 2.6 mmol) was dissolved in DCM (10 mL). Compound 19-A2 (350 mg, 1.3 mmol) was added, and the mixture was cooled to - 30 °C. A DCM solution (1mL) of TEA (326 mg, 3.2 mmol) was added dropwise, and the mixture was kept at -30°C. The progress of the reaction was monitored by HPLC, and raw materials were disappeared after reacting for 2 hours. After the mixture was cooled to -40 °C, 2-bromoethylamine hydrobromide (2.1 g, 10.2 mmol) and TEA (1.0 g, 10.0 mmol) were added. The reaction was completed after kept at -40 °C for 30 minutes. $H_2O$ (5 mL) was added, and the mixture was stirred for 15 minutes. The mixture was extracted with DCM (10 mL ×3), washed with water (5 mL ×2), dried with anhydrous $Na_2SO_4$, evaporated the solvent, and purified with column chromatography to obtain compound 19-A3 (405 mg, an off-white solid, 55.6%). $^1$H-NMR (400 MHz, $CD_3OD$): $\delta$9.04 (d, *J* = 3.2 Hz, 1H), 8.89 (d, *J* = 8.8 Hz, 1H), 8.19 (d, *J* = 8.0 Hz, 1H), 8.09 (d, *J* = 8.0 Hz, 1H), 7.81-7.78 (m, 1H), 6.61-6.57 (m, 1H), 3.50-3.34 (m, 4H), 3.15-3.02 (m, 4H). MS: Calculated 561.9, 563.9, 565.9, found 563.0, 565.0, 567.0 ([M+H]$^+$).

Compound 19-A3 (200 mg, 0.4 mmol) was dissolved in THF (10 ml). $Ag_2O$ (411 mg, 1.8 mmol) and DIEA (230

mg, 1.8 mmol) were added. The mixture was heated to 65 °C and reacted for 3.5 hours. After cooling to room temperature, the mixture was suction filtered with diatomite. The solid was washed with THF (3 ml ×3). The original liquid was evaporated to remove the solvent and separated with HPLC to obtain compound 19 (51.3 mg, 35.9%), a white solid. $^1$H-NMR (400 MHz, $CDCl_3$): δ9.12 - 9.11 (m, 1H), 8.59 (d, *J* = 8.4 Hz, 1H), 8.05 (d, *J* = 8.0 Hz, 1H), 7.98 (d, *J* = 8.0 Hz, 1H), 7.66 (dd, *J* = 8.8, 4.0 Hz, 1H), 6.50 (m, 1H), 2.29-1.96 (m, 8H). MS: Calculated 402.1, found 403.1 ([M+H]$^+$).

**Preparation of Example 19: preparation of compound 20**

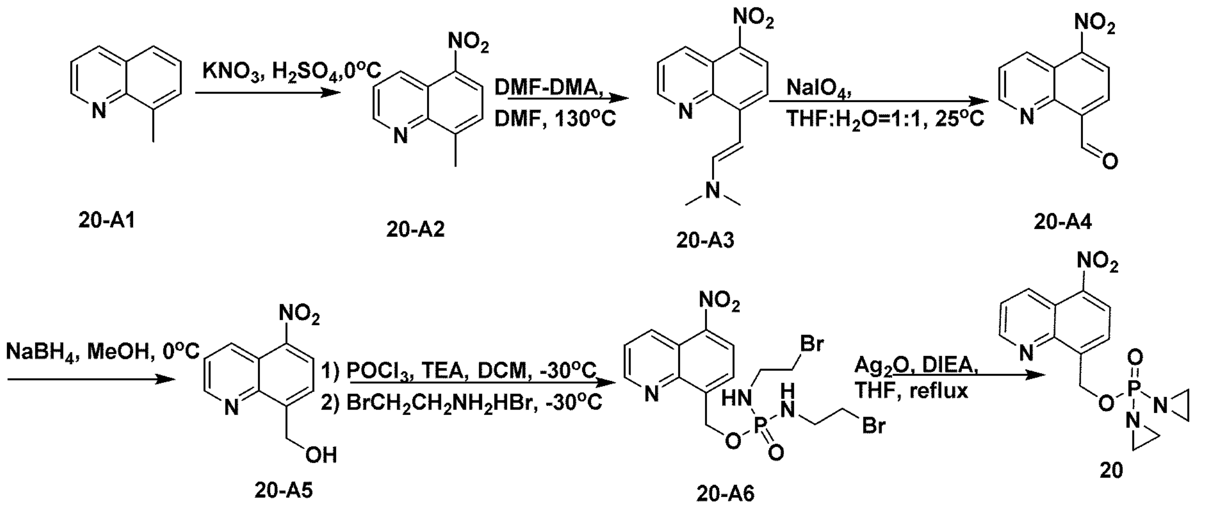


20-A1 20-A2 20-A3 20-A4 20-A5 20-A6 20

Under nitrogen protection, compound 20-A1 (1.0 g, 7.0 mmol) was dissolved in concentrated sulfuric acid (4 mL). After cooled to 0 °C, potassium nitrate (847 mg, 8.4 mmol) was added in batches. The mixture was raised to room temperature and stirred. One hour later, the end of the reaction was monitored by liquid chromatography. The reacted mixture was added dropwise to ice water (20 mL) and adjusted the pH value to 9 with sodium carbonate aqueous solution. The reaction mixture was extracted with EA (10 mL×4), dried with anhydrous sodium sulfate, and evaporated the solvent to obtain compound 20-A2 (700 mg, 53.3%), a yellow solid. $^1$H-NMR (400 MHz, $CDCl_3$): δ9.07-9.04 (m, 2H), 8.31 (d, *J* = 8.0 Hz, 1H), 7.66-7.63 (m, 2H), 2.91 (s, 3H). MS: Calculated 188.1, found 189.1 ([M+H]$^+$).

Under nitrogen protection, compound 20-A2 (1.0 g, 5.3 mmol) was dissolved in DMF (8 mL). DMF-DMA (1.9 g, 15.9 mmol) was added, and the mixture was heated to 130 °C and stirred to react for 1 hour. The end of the reaction was monitored by liquid chromatography. Most of DMF in the mixture was evaporated, suction filtered, washed with water (40 mL), and dried to obtain a crude product, compound 20-A3 (1.1 g, 85.2%), a black solid. $^1$H-NMR (400 MHz, $CDCl_3$): δ9.30 *(dd, J*= 8.8, 1.6 Hz, 1H), 8.87 (dd, J= 4.0, 1.6 Hz, 1H), 8.38 (d, *J* = 8.8 Hz, 1H), 7.58-7.49 (m, 3H), 6.53 (d, *J* = 13.6 Hz, 1H), 3.09 (s, 6H).

Under nitrogen protection, compound 20-A3 (300 mg, 1.2 mmol) and sodium periodate (791 mg, 3.7 mmol) were added to a mixed solution of THF and water (1:1, 6 mL) at 0 °C. After the mixture was raised to room temperature and stirred for 0.5 hours, the end of the reaction was monitored by liquid chromatography. The mixture was suction filtered, and the solid was washed with EA (15 mL). Most of the solvent was evaporated. The mixture was extracted with EA (10 mL×3), washed with water (5 mL), dried with anhydrous sodium sulfate, and distilled to obtain compound 20-A4 (170 mg, 68.2%), a yellow solid. $^1$H-NMR (400 MHz, $CDCl_3$): δ11.47 (s, 1H), 9.17 (dd, *J* = 4.0, 1.6 Hz, 1H), 8.97 (dd, *J*= 8.8, 1.6 Hz, 1H), 8.41-8.36 (m, 2H), 7.75 (dd, *J* = 8.8, 4.0 Hz, 1H). MS: Calculated 202.0, found 203.1 ([M+H]$^+$).

Compound 20-A4 (200 mg, 0.99 mmol) was dissolved in methanol (5 mL). After cooled to 0 °C, sodium borohydride (56 mg, 1.5 mmol) was added in batches. After the addition, the mixture was reacted for 15 minutes. Water (3 mL) was added to quench. The mixture was extracted with DCM (10 mL×2), washed with 5 % of citric acid aqueous solution (5 mL×2), washed with water (5 mL×2), washed with brine (5 mL), dried, and distilled to obtain compound 20-A5 (150 mg, 74.3%), a yellow solid. $^1$H-NMR (400 MHz, $CDCl_3$): δ9.10-9.07 (m, 1H), 8.99 (dd, *J* = 4.0, 1.6 Hz, 1H), 8.36 (d, *J* = 8.0 Hz, 1H), 7.75 (d, *J* = 7.6 Hz, 1H), 7.69 (dd, *J* = 8.8, 4.0 Hz, 1H), 5.28 (d, *J* = 8.8 Hz, 2H). MS: Calculated 204.1, found 205.1 ([M+H]$^+$).

Under nitrogen protection, compound 20-A5 (150 mg, 0.7 mmol) and $POCl_3$ (225 mg, 1.5 mmol) were dissolved in DCM (4 mL). After cooling to -30 °C, TEA (186 mg, 1.8 mmol) was added dropwise. One hour later, the disappearance of raw materials was monitored by liquid chromatography. Then, 2-bromoethylamine hydrobromide (1.2 g, 5.9 mmol),

and TEA (595 mg, 5.9 mmol) was added dropwise. 0.5 hours later, the end of the reaction was monitored by liquid chromatography. Water (3 mL) was added. The mixture was extracted with DCM (10 mL×2), washed with water (5 mL), dried anhydrous sodium sulfate, evaporated the solvent, and separated with column chromatography to obtain compound 20-A6 (230 mg, 63.1%), a yellow oily substance. $^1$H-NMR (400 MHz, $CDCl_3$): $\delta$9.06-9.03 (m, 2H), 8.39 (d, $J$ = 8.0 Hz, 1H), 7.94 (d, $J$ = 8.0 Hz, 1H), 7.70 (dd, $J$ = 8.8, 4.4 Hz, 1H), 5.76 (d, $J$ = 6.8 Hz, 2H), 3.52-3.40 (m, 10H). MS: Calculated 493.9, 495.9, 497.9, found 494.9, 496.9, 498.9 ($[M+H]^+$).

Under nitrogen protection, compound 20-A6 (200 mg, 0.4 mmol) was dissolved in THF (6 mL). DIEA (261 mg, 2.0 mmol) and silver oxide (280 mg, 1.2 mmol) were added, and the mixture was heated to reflux to react overnight. The mixture was suction filtered with diatomite. The original liquid was dried, and a product, compound 20 (41 mg, 30.4%), a white solid, was obtained by neutral preparation with HPLC. $^1$H-NMR (400 MHz, $CD_3OD$): $\delta$9.04 (dd, $J$ = 4.0, 1.2 Hz, 1H), 8.99 (d, $J$ = 8.8 Hz, 1H), 8.47 (d, $J$ = 8.0 Hz, 1H), 8.03 (d, $J$ = 8.0 Hz, 1H), 7.79 (dd, $J$ = 8.8, 4.0 Hz, 1H), 5.97 (d, $J$ = 7.2 Hz, 2H), 2.31-2.24 (m, 8H). MS: Calculated 334.1, found 335.1 ($[M+H]^+$).

**Preparation of Example 20: preparation of compound 21**

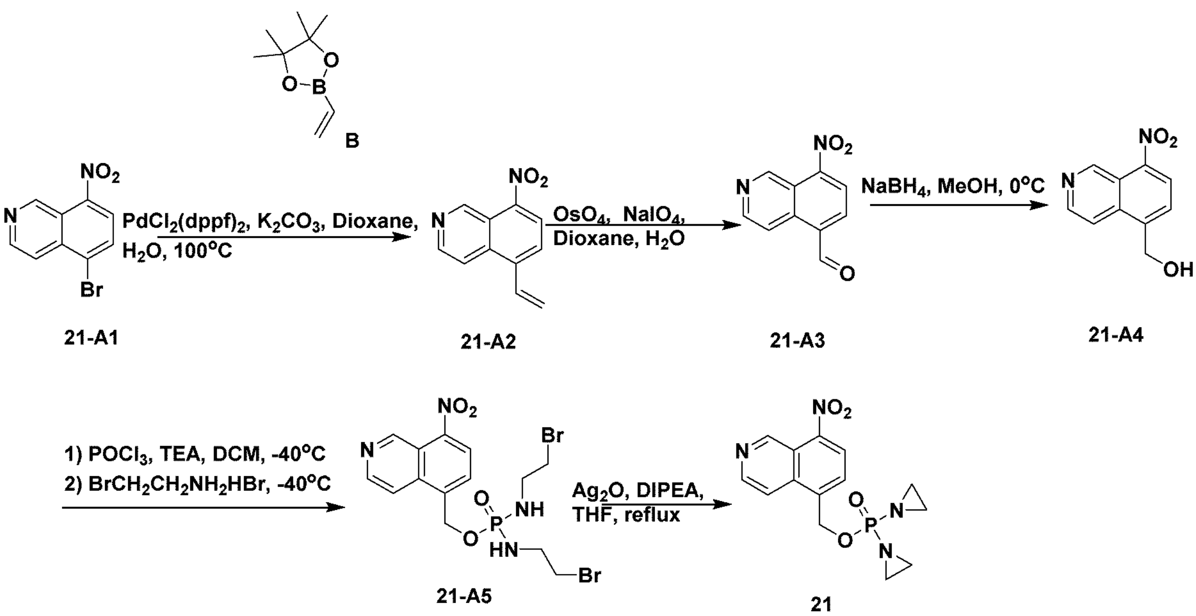


21-A1 21-A2 21-A3 21-A4

21-A5 21

Under nitrogen protection, compound 21-A1 (2.0 g, 7.9 mmol) was dissolved in dioxane (20 mL) and water (2.0 mL). Then, Pd (dppf) $Cl_2$ (280 mg, 0.3 mmol) and $K_2CO_3$ (580 mg, 15.9 mmol) were added, and compound B (2.2 g, 7.9 mmol) was added. The mixture was heated to 100 °C and stirred for 5 hours. After the reaction was completed, the mixture was cooled to room temperature. Saturated ammonium chloride (5 mL) was added. The mixture was extracted with EA (20 mL×3). The organic phase wad combined, which was dried with anhydrous sodium sulfate, evaporated the solvent, and purified with column chromatography to obtain a crude product, compound 21-A2 (1.3 g, 81.2 %, content about 80%), a yellow oily substance. Compound 21-A2 was directly used in the next reaction after NMR and MS assay.

Under nitrogen protection, compound 21-A2 (800 mg, 4.0 mmol) was dissolved in dioxane (10 mL) and water (1 mL). $NaIO_4$(2.6 g, 12.0 mmol) was added. Then, $OsO_4$ (50.8 mg, 0.20 mmol) was added, and the mixture was stirred at room temperature for 24 hours. The mixture was extracted with EA (10 mL×3). The organic phase was dried with anhydrous sodium sulfate, evaporated the solvent, and purified with column chromatography to obtain compound 21-A3 (280 mg, 35.0 %), a white solid. $^1$H-NMR (400 MHz, $CDCl_3$): $\delta$10.50 (s, 1H), 9.89 (s, 1H), 9.08 (d, $J$ = 6.0 Hz, 1H), 8.90 (d, $J$ = 6.0 Hz, 1H), 8.33-8.32 (m, 2H). MS: Calculated 202.1, found 203.1 ($[M+H]^+$).

Compound 21-A3 (400 mg, 2.0 mmol) was dissolved in MeOH (10 mL). After cooled to 0 °C, $NaBH_4$ (91.0 mg, 2.4 mmol) was added in batches. The mixture was stirred at 0 °C for 0.5 hours. Water (5 mL) was added to quench the reaction, which was extracted with EA (10 mL ×3). The organic phase was dried with anhydrous sodium sulfate, evaporated the solvent, and purified with column chromatography to obtained compound 21-A4 (210 mg, 51.5 %), an off-white solid. $^1$H-NMR (400 MHz, $CDCl_3$): $\delta$10.50 (s, 1H), 8.76 (d, $J$ = 6.0 Hz, 1H), 8.34 (d, $J$= 8.0 Hz, 1H), 7.94 (d, $J$= 8.0 Hz, 1H), 7.89 (d, $J$= 6.0 Hz, 1H), 5.26 (d, $J$ = 5.2 Hz, 2H). MS: Calculated 204.1, found 205.1 ($[M+H]^+$).

Under nitrogen protection, compound 21-A4 (210 mg, 1.0 mmol) was dissolved in DCM (20 mL). After cooling

to -40 °C, $POCl_3$ (317 mg, 2.1 mmol) and TEA (525 mg, 5.2 mmol) were added. The mixture was stirred at -40 °C for 2 hours. Then, at the same temperature, bromoethylamine hydrobromide (1.3 g, 6.3 mmol) and TEA (2.1 g, 20.8 mmol) were added. The mixture was kept the temperature to react for 1 hour. After completed, saturated ammonium chloride aqueous solution (5 mL) was added. The mixture was extracted with DCM (10 mL×3) and washed with water (10 mL×2). The organic phase was dried with anhydrous sodium sulfate, evaporated the solvent, and purified with column chromatography to obtain compound 21-A5 (280 mg, 54.9 %, content about 70%), a yellow oily substance. MS: Calculated 493.9, 495.9, 497.9, found 494.9, 496.9, 498.9 ($[M+H]^+$).

Under nitrogen protection, compound 21-A5 (280 mg, 0.6 mmol) was dissolved in THF (25 mL). Silver oxide (784 mg, 3.4 mmol) and DIPEA (437 mg, 3.4 mmol) were added. The mixture was heated to reflux to react for 3 hours. After the reaction was completed, the mixture was cooled to room temperature and suction filtered with diatomite. The solid was washed with DCM. The original liquid was evaporated the solvent, and prepared with HPLC to obtain a pure product, compound 21 (40.6 mg, 29.7 %), an off-white solid. $^1$H-NMR (400 MHz, $CD_3OD$): $\delta$9.89 (s, 1H), 8.74 (d, *J*= 6.0 Hz, 1H), 8.43 (d, *J* = 8.0 Hz, 1H), 8.19 (d, *J* = 6.0 Hz, 1H), 8.06 (d, *J* = 8.0 Hz, 1H), 5.73 (d, *J* = 8.0 Hz, 2H), 2.24-2.19 (m, 8H). MS: Calculated 334.1, found 335.1 ($[M+H]^+$).

**Preparation of Example 21: preparation of compound 22**

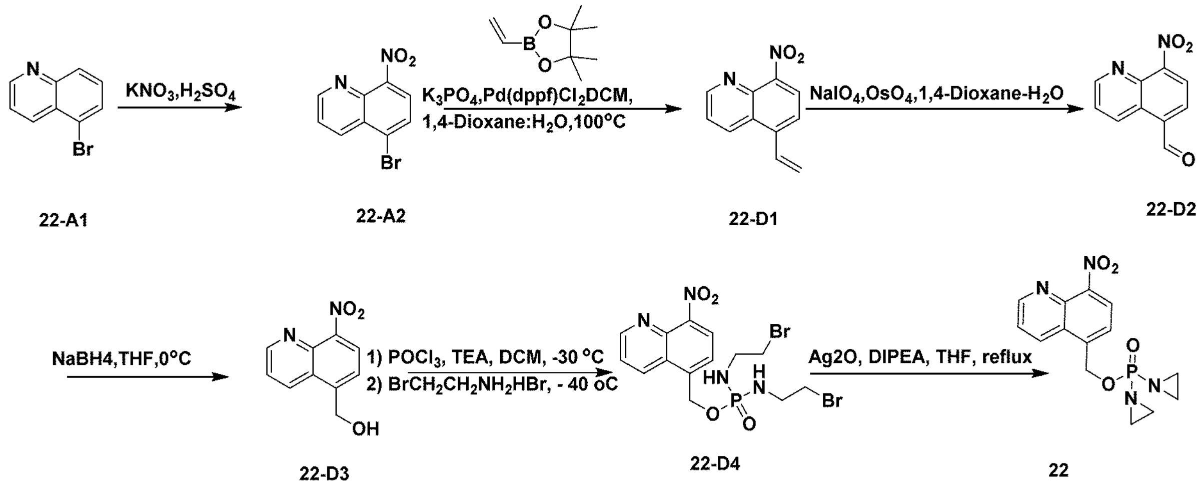


22-A1 22-A2 22-D1 22-D2

22-D3 22-D4 22

Compound 22-A1 (8.0 g, 38.4 mmol) was concentrated sulfuric acid (30 ml). After cooled to 0 °C, potassium nitrate (4.7g, 46.1 mmol) was added in batches. The mixture was stirred at room temperature for 1.5 hours. Then, the reaction system was slowly poured into ice water (80 mL). After the mixture was stirred for 30 minutes, the mixture was extracted with DCM (50 mL×3), washed with water (15mL×3), washed with brine, and evaporated the solvent to obtain compound 22-A2 (7.2 g, 74.2%), a light yellow solid. $^1$H-NMR (400 MHz, DMSO-*d6*): $\delta$9.11 (dd, *J* = 4.0, 1.6 Hz, 1H), 8.66 (dd, *J* = 8.8, 1.6 Hz, 1H), 8.24 (d, *J* = 8.0 Hz, 1H), 8.16 (d, *J* = 8.0 Hz, 1H), 7.89 (dd, *J* = 8.8, 4.0 Hz, 1H). MS: Calculated 252.0 and 254.0, found 253.0 and 255.0 ($[M+H]^+$).

Under nitrogen protection, compound 22-A2 (3.0 g, 11.9 mmol) and vinylboronic acid pinacol cyclic ester (2.2 g, 14.3 mmol) were dissolved in a mixed solution of dioxane and water (30 mL, 10:1). Potassium orthophosphate (6.3 g, 29.8 mmol) was added. After nitrogen was sucked and refilled three times, 1,1'-bis(diphenylphosphino)ferrocenepalladium(II)dichloride dichloromethane complex (2.9 g, 3.6 mmol) was added. The mixture was heated to 100 °C and stirred for 1.5 hours, and the reaction was completed. After cooled to room temperature, saturated ammonium chloride aqueous solution (15mL) was added to quench. The mixture was extracted with EA (20 mL×3), washed with water (10 mL), washed with saturated brine (10 mL), evaporated the solvent, and purified with column chromatography to obtain compound 22-D1 (1.3g, 54.6%), a yellow solid. $^1$H-NMR (400 MHz, $CD_3OD$): $\delta$8.97 *(dd, J*= 4.0, 1.6 Hz, 1H), 8.74 (dd, *J* = 8.8, 1.6 Hz, 1H), 8.09 (d, *J* = 8.0 Hz, 1H), 7.86 (d, *J* = 8.0 Hz, 1H), 7.69 (dd, *J* = 8.8, 4.0 Hz, 1H), 7.54 (dd, *J* = 17.2, 11.2 Hz, 1H), 6.01 (dd, *J* = 17.2, 0.8 Hz, 1H), 5.71 (dd, *J* = 11.2, 0.8 Hz, 1H). MS: Calculated 200.1, found 201.0 ($[M+H]^+$).

Compound 22-D1 (1.3 g, 6.5 mmol) was dissolved in 1, 4-dioxane. Sodium periodate (4.2 g, 19.5 mmol) aqueous solution (15 mL) was added. Then, a tert-butanol aqueous solution (3.5 g) with 2.4 % of $OsO_4$ was added dropwise. The mixture was stirred at room temperature overnight. After the reaction was completed, the solid was suction filtered out. The solid was washed with EA (20 mL), and the liquid was separated. The organic phase was washed with water (5 mL×2), dried with anhydrous sodium sulfate, and evaporated the solvent to obtain a crude product, compound 22-D2

(1.3 g). Compound 22-D2 was used in the next step.

Compound 22-D2 (800 mg, 4.0 mmol) was dissolved in THF (5 mL). After cooled to 0 °C, $NaBH_4$ (225 mg, 5.9 mmol) was added in batches. 30 minutes later, the reaction was completed. At 0 °C, $H_2O$ (5 mL) was added dropwise, and the mixture was stirred for 20 minutes. The mixture was extracted with DCM (10 mL×3). The organic phase was washed with water (5 mL×2), washed with brine (5 mL), dried with anhydrous sodium sulfate, evaporated the solvent, and purified with column chromatography to obtain a pure product, compound 22-D3 (470 mg, two-step yield 58.1 %), a yellow solid. $^1$H-NMR (400 MHz, $CD_3OD$): δ 8.97 (dd, *J* = 4.0, 1.6 Hz, 1H), 8.69 (dd, *J* = 8.8, 1.6 Hz, 1H), 8.09 (d, *J* = 7.6 Hz, 1H), 7.78 (d, *J* = 7.6 Hz, 1H), 7.69 (dd, *J* = 8.8, 4.0 Hz, 1H), 5.14 (s, 2H). MS: Calculated 204.1, found 205.2 ($[M+H]^+$).

$POCl_3$ (600 mg, 3.9 mmol) was DCM (10 mL), and compound 22-D3 (400 mg, 2.0 mmol) was added thereto. After cooled to -30 °C, a DCM solution (1mL) of TEA (500 mg, 4.9 mmol) was added dropwise. The mixture was kept at -30 °C to react. The progress of the reaction was monitored by HPLC, and raw materials were disappeared after reacting for 2 hours. After cooled to -40 °C, 2-bromoethylamine hydrobromide (3.2 g, 15.7 mmol) and TEA (1.6 g, 15.7 mmol) were added. The mixture was kept at -40 °C, and the reaction was completed 30 minutes later. After the temperature was raised to 5 °C, $H_2O$ (5 mL) was added dropwise. The mixture was stirred for 15 minutes, which was extracted with DCM (10 mL×3), dried with anhydrous sodium sulfate, evaporated the solvent, and purified with column to obtain compound 22-D4 (350 mg, 36.0%), a yellowish-brown solid. $^1$H-NMR (400 MHz, $CD_3OD$): *δ*9.01 (d, *J*= 2.8 Hz, 1H), 8.73 (d, *J*= 8.4 Hz, 1H), 8.10 (d, *J* = 7.6 Hz, 1H), 7.83 (d, *J* = 7.6 Hz, 1H), 7.75 (dd, *J* = 8.4, 4.0 Hz, 1H), 5.53 (d, *J* = 7.8 Hz, 2H), 3.46-3.41 (m, 4H), 3.29-3.26 (m, 4H). MS: Calculated 493.9, 495.9, 497.9, found 495.0, 497.0, 499.0 ($[M+H]^+$).

Compound 22-D4 (200 mg, 0.4 mmol) was dissolved in THF (10 mL). $Ag_2O$ (470 mg, 2.0 mmol) and DIPEA (261 mg, 2.0 mmol) were added, and the mixture was raised to 65 °C. The progress of the reaction was monitored by HPLC, and the reaction was completed 2 hours later. After cooled to room temperature, the mixture was suction filtered with diatomite. The solid was washed with THF (3mL×3). The mixture was evaporated the solvent, and separated with HPLC to obtain compound 22 (14.4 mg, 10.7 %), a white solid. $^1$H-NMR (400 MHz, $CDCl_3$): *δ*9.11 (dd, *J* = 4.4, 1.6 Hz, 1H), 8.57 (dd, *J* = 8.8, 1.6 Hz, 1H), 8.00 (d, *J* = 7.6 Hz, 1H), 7.74 (d, *J* = 7.6 Hz, 1H), 7.63 (dd, *J* = 8.4, 4.4 Hz, 1H), 5.64 (d, *J* = 7.6 Hz, 2H), 2.22-2.02 (m, 8H). MS: Calculated 334.1, found 335.1 ($[M+H]^+$).

**Preparation of Example 22: preparation of compound 26**

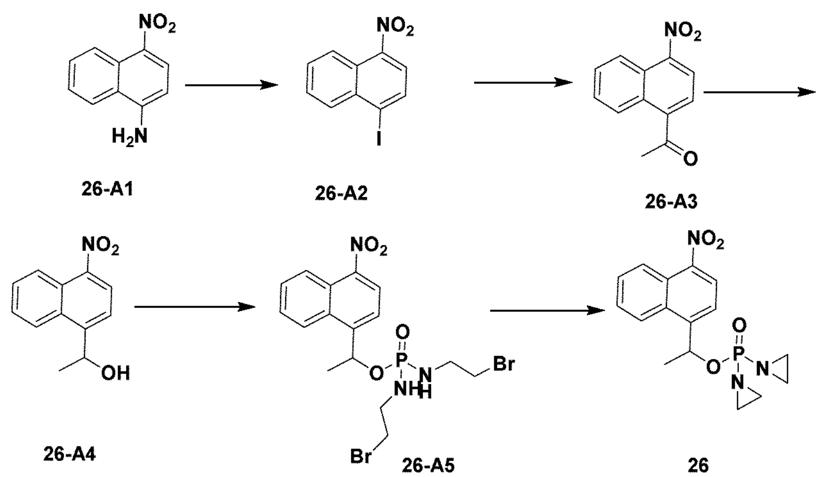

26-A1 26-A2 26-A3

26-A4 26-A5 26

Para-toluenesulfonic acid (20.0 g, 112.0 mmol) added to acetonitrile (160 ml), and then compound 26-A1 (7.4 g, 39.32 mmol) was added to the reaction system. After cooled to 0 °C, a solution (24 ml) of KI (16 g, 98.30 mmol) and $NaNO_2$ (5.4 g, 78.64 mmol) was added to the system. The reaction was completed 2 hours later. $H_2O$ (600 ml) was added to diluted. The pH value of the mixture was adjusted to 9-10 with 1 N of $NaHCO_3$. Next, 2 M of disodium thiosulfate solution (50 ml) was added to precipitate a large amount of solid. After suction filtered, the solid was dissolved with ethyl acetate, dried to concentrated, and separated with column chromatography (200-300 mesh silica, n-heptane: ethyl acetate = 20:1) to obtain a product (11.0 g, yield 93.5%), compound 26-A2, a yellow solid. $^1$H-NMR (400 MHz, $CDCl_3$): δ 8.50 (d, *J* = 8.0 Hz, 1H), 8.24 (d, *J* = 1.2 Hz, 1H), 8.20 (d, *J* = 8.4 Hz, 1H), 7.86 (d, *J* = 8.0 Hz, 1H), 7.76-7.70 (m, 2H).

Under $N_2$, compound 26-A2 (3.0 g, 10.00 mmol) and 1-ethoxyvinyltri-n-butyltin (5.0 g, 14.00 mmol) were added to dioxane (40 ml). After nitrogen was sucked and refilled three times, the mixture was heated to 105 °C overnight and the reaction was completed. After cooled to room temperature, 50 ml of ammonium chloride was added to quench the system. The mixture was extracted with ethyl acetate (50 ml×4). After concentrated, 3N of HCl (100 ml) was added.

After the mixture was stirred for 12 hours, extracted with DCM (50 ml×4), washed with water, washed with brine, dried to concentrated, and separated by column chromatography (200-300 mesh silica, n-heptane: ethyl acetate = 20:1) to obtain a product, compound 26-A3 (1.1 g, yield 51.5%), a yellow solid. $^1$H-NMR (400 MHz, $CDCl_3$): δ 8.55 (d, *J*= 6.4 Hz, 1H), 8.45 (d, *J* = 6.4 Hz, 1H), 8.10 (d, *J*= 7.6 Hz, 1H), 7.83 (d, *J* = 7.6 Hz, 1H), 7.78-7.69 (m, 2H), 2.77 (s, 3H). MS: Calculated 215.1, found 216.0 ($[M+H]^+$).

Under nitrogen protection, compound 26-A3 (200 mg, 0.93 mmol) was dissolved in THF (5 mL). After cooled to 0 °C, $BH_3$-THF (4.7 mL, 4.65mmol) was added to the system. The reaction was completed after 30 minutes. After cooled to 0 °C, MeOH (6 mL) was added dropwise to quench. After concentrated, DCM was used to dissolve. The mixture was washed with 1 N of HCl three times, and dried and concentrated to obtain a product, compound 26-A4 (185 mg, yield 91.6%), a yellow oily substance. $^1$H-NMR (400 MHz, $CDCl_3$): δ 8.55 (d, *J* = 6.4 Hz, 1H), 8.20-8.11 (m, 2H), 7.79 (d, *J* = 6.4 Hz, 1H), 7.73-7.62 (m, 2H), 5.75-5.70 (m, 1H), 1.66 (d, *J* = 6.4 Hz, 3H). MS: Calculated 217.1, found 218.0 ($[M+H]^+$)

Under nitrogen protection, compound 26-A4 (650 mg, 2.99 mmol), Br-IPM (1.4 g, 4.49 mmol), and $PPh_3$ (1.8 g, 5.99 mmol, commercially purchased) were added to THF (50 ml). After cooled to 0 °C, DIAD (1.2 g, 5.99 mmol, commercially purchased) was added dropwise to the system. The mixture was kept at 0 °C for 30 minutes, and the reaction was completed. Water (50 ml) was added to the system, which was extracted with DCM (30 ml ×3). The organic phase was washed with water, washed with brine, dried and concentrated, and separated with column chromatography (200-300 mesh silica, n-heptane: ethyl acetate = 1:1) to obtain a product, compound 26-A5 (400 mg, yield 26.3%), a yellow oily substance. $^1$H-NMR (400 MHz, $CDCl_3$): *δ* 8.52 (d, *J* = 6.4 Hz, 1H), 8.26-8.15 (m, 2H), 7.75-7.68 (m, 2H), 7.54-7.52 (m, 1H), 6.35-6.28 (m, 1H), 3.49-3.15 (m, 8H), 1.79 *(d, J*= 6.4 Hz, 3H).

Under nitrogen protection, compound 26-A5 (250 mg, 0.49 mmol) was dissolved in THF (25 ml). Then, silver oxide (569 mg, 2.46 mmol) and DIEA (217 mg, 2.46 mmol) were added to the system. The mixture was heated to 65 °C and stirred overnight, and the reaction was completed. The mixture was suction filtered, and the solid was washed with DCM. The original liquid was concentrated and prepared with HPLC to obtain compound 26 (30.7 mg, 18.1%), a yellow oily substance. $^1$H-NMR (400 MHz, $CDCl_3$): δ 8.56 - 8.54 (m, 1H), 8.20 8.17 (m, 2H), 7.81 (d, *J* = 8.0 Hz, 1H), 7.74-7.67 (m, 2H), 6.57-6.50 (m, 1H), 2.25-1.99 (m, 8H), 1.79 (d, *J*= 6.4 Hz, 3H). MS: Calculated 347.1, found 348.0 ($[M+H]^+$).

**Example 23: Resolution of compounds and determination of absolute configuration**

The pure product of compound 01 was subjected to chiral analysis.

HPLC chiral analysis conditions were:

| | |
|---|---|
| Analytical column | CHIRALPAK AD-3(AD30CD-SK010) column |
| Column size | 0.46 cm internal diameter, 15 cm long |
| Injection volume | 0.5 μl |
| Mobile phase | n-hexane: ethanol = 60/40 (volume ratio) |
| Flow rate | 1.0 ml/min |
| Detection wavelength | UV 210 nm |
| Column temperature | 35 °C |

The HPLC spectrum obtained from the assay is shown in FIG. 11 (named as No. 1 peak and No. 2 peak based on the time of peak appearance). The specific results were listed in the following table.

| Separation peak | Retention time (Ret. Time) | Area | Area ratio (%) |
|---|---|---|---|
| No. 1 | 2.908 | 5110974 | 47.096 |
| No. 2 | 3.766 | 5741368 | 52.904 |

4.9971 g of compound 01 was taken and dissolved in chromatography grade ethanol. The following chiral separation preparation method and chiral separation preparation conditions were used to carry out chiral separation preparation.

HPLC chiral separation preparation method: HPLC method was used. HPLC preparation equipment and chiral

columns were used to separate the preceding chiral isomers, and the corresponding components were collected. The solvent was removed by rotary evaporation to obtain pure optical isomers.

HPLC chiral separation preparation conditions were as follow.

| | |
|---|---|
| Resolution Column | CHIRALPAK AD column |
| Column size | 5.0 cm internal diameter, 25 cm long, 10mm particle size |
| Mobile phase | n-hexane : ethanol = 60/40 (volume ratio) |
| Flow rate | 60 ml/min |
| Detection wavelength | UV 254 nm |
| Column temperature | 38 °C |

After separation and preparation, 2.5377 g of the pure optical isomer corresponding to the No. 1 peak was obtained, and a measured enantiomeric excess value (ee value) was 99.7 % thereof. As well, 2.4652 g of the pure optical isomer corresponding to No. 2 peak was obtained, and a measured ee value was 99.4 %.

Further, the optical isomers corresponding to the No. 1 peak and the No. 2 peak prepared by the chiral separation were analyzed using HPLC chiral analysis conditions, and the results obtained was shown in FIG. 12 and FIG. 13.

The HPLC analysis results of the optical isomer corresponding to No. 1 peak were as the following table.

| Separation peak | Retention time (Ret. Time) | Area | Area ratio (%) |
|---|---|---|---|
| No. 1 | 2.908 | 7125381 | 99.837 |
| No. 2 | 3.758 | 11633 | 0.163 |

The HPLC analysis results of the optical isomer corresponding to No. 2 peak were as the following table.

| Separation peak | Retention time (Ret. Time) | Area | Area ratio (%) |
|---|---|---|---|
| No. 1 | 2.897 | 20323 | 0.320 |
| No. 2 | 3.765 | 6340121 | 99.680 |

Determination of the absolute configuration of the compounds

An absolute configuration of compound 01 was resolved by single X-ray diffraction results.

Single crystal culture

About 5 mg of the optical isomer sample corresponding to the No. 1 peak was weighed, and dissolved in methanol (2 mL, analytical grade). At room temperature, the mixture was sonicated for 1 minute, filtered through an organic filter membrane, and stayed at room temperature for 2 days to obtain colorless, crystals.

Single crystal structure analysis

The colorless crystals were single crystal diffracted using the following parameter with X-ray single crystal diffractometer (D8 Venture, Bruker).

| | |
|---|---|
| Light source: Mo target | X-ray: Mo-K (=0.71073 Å) |
| Detector: CMOS area detector | Resolution: 0.80 Å |
| Current, Voltage: 50 kV, 1.2A | Exposure time: 30 s |
| Distance from area detector to sample: 50 mm | Test temperature: 298K (25°C) |

After data were collected, the diffraction data were integrated and restore with SAINT program. Then, the data

were empirically corrected for absorption with SADABS program. The single crystal structure was analyzed by a direct method with SHELXT2014, and the structure was refined by a least square method. Isotropic calculation was adopted in the refining process of hydrogen atom. The hydrogen atoms on C-H were hydrogenated by calculation and refined by riding model. A flack constant obtained from the data was 0.03 (10), and the absolute configuration may be determined. The C5 configuration in the structure was an S-configuration as shown in FIG. 14.

It may be seen that the optical isomer corresponding to No. 1 peak is the S-configuration, and the structure formula is as follow.

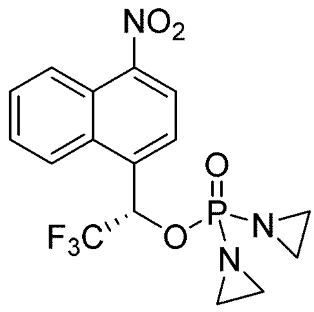

, this compound was hereafter referred to as "compound 01-S-configuration."

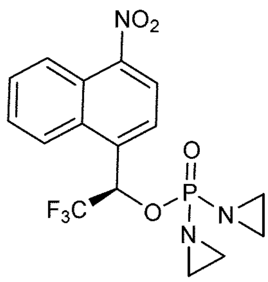

,

this compound was hereafter referred to as "compound 01-R-configuration."

Based on the similar operation steps in the preceding embodiments, different starting substrates may be selected to synthesize a series of similar compounds.

Similarly, isotopic variants of a series of compounds may be synthesized by similar operations using isotopic variants of different starting substrates or isotopic variant reagents.

The compound of control 1 was TH-302, which was purchased commercially.

**Control Example: Preparation of Compound (D2) of control 2**

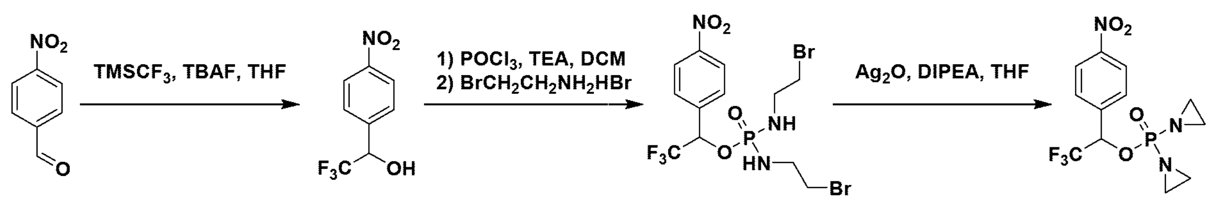


D2-A1 D2-A2 D2-A3 D2

Under nitrogen protection, compound D2-A1 (0.5 g, 3.3 mmol, purchased commercially) and $TMSCF_3$ (0.7 g, 4.9 mmol, purchased commercially) were dissolved in THF (10 mL). After cooled to 0 °C, TBAF (0.13ml, 0.07 mmol, 1M in THF, purchased commercially) was added dropwise to the system. After the system was kept at 0 °C for 30 minutes, all compound D2-A1 disappeared. 3N of HCl (2 ml) was added dropwise to the system, and the system became clear. The system was stirred at 0 °C for 1 hour, all of which became products. The system was extracted with DCM (10 ml×3). The organic phase was washed with water (10 ml×3), dried and concentrated, and separated with column chromatography (200-200 mesh silica gel, n-heptane : ethyl acetate = 12:1 - 10:1) to obtain a product, compound D2-A2 (0.7 g, yield 95.7 %), a yellow oily substance. $^1$H-NMR (400 MHz, $CDCl_3$): $\delta$ 8.27 (d, *J* = 8.8 Hz, 2H), 7.70 (d, *J* = 8.8 Hz, 2H), 5.16- 5.20 (m, 1H).

Under nitrogen protection, $POCl_3$ (972 mg, 6.34 mmol) was dissolved in DCM (10 ml), and the system was cooled to -40 °C. Then, compound D2-A2 (0.7 g, 3.17 mmol) was dissolved in DCM (20 ml), which was added dropwise to the system. Compound D2-A3 was all converted into intermediates after being kept at -40 °C for 2 hours. Then, Bromoethylamine bromide (4.06g, 25.36 mmol) and TEA (5.1 g, 0.05 mol) were added to the system. The system was monitored by HPLC. 30 minutes later, the reaction was completed. At 0 °C, saturated $NH_4Cl$ (10 ml) was added, and the system was extracted with DCM (10 ml×3). The organic phase was washed with water, washed with brine, dried and concentrated, and separated with column chromatography (200-200 mesh silica gel, n-heptane : ethyl acetate = 5:1 - 1:1) to obtain a product, compound D2-A3 (420 mg, yield 26.3 %), a yellow oily substance. $^1$H-NMR (400 MHz, $CDCl_3$): $\delta$ 8.31 (d, *J* = 8.4 Hz, 2H), 7.70 (d, *J* = 8.4 Hz, 2H), 5.78 - 5.82 (m, 1H), 3.13 -3.54(m, 10H). MS: Calculated 510.9, found 513.9 ($[M+1]^+$).

Under nitrogen protection, compound D2-A3 (400 mg, 0.8 mmol) was dissolved in tetrahydrofuran (20 mL). Then, silver oxide (1.1 g, 4.7 mmol) and DIPEA (0.6 g, 4.7 mmol) were added. The system was raised to 65 °C and stirred for 1.5 hours. After the reaction was completed, the system was cooled to room temperature and suction filtered with diatomite. The solid was washed with dichloromethane. The original liquid was concentrated, and prepared with HPLC to obtain a pure product, compound D2 (127mg, 46.3%), a white solid. $^1$H-NMR (400 MHz, DMSO-*d6*): $\delta$ 8.34 (d, *J* = 8.8 Hz, 2H), 7.89 (d, *J* = 8.8 Hz, 2H), 6.37- 6.33 (m, 1H), 2.18-1.98 (m, 8H). MS: Calculated 352.1, found 352.0 ($[M+H]^+$).

## Effect Examples

Descriptions of animal experiments were as follow. All animal studies in this disclosure were carried out by professional CRO (Contract research organization) companies. The experimental protocols were approved by the Institutional Animal Care and Use Committee within the institution or similar institutions, and the handling of animals met the requirements.

Effect embodiment 1: Cytotoxicity test of human tumor cell line ***in vitro***

The inhibitory effect of each compound on cancer cell proliferation was compared using $IC_{50}$ (50 % growth inhibitory concentration in nanomolar (nM)), a quantified value of cytotoxicity against human tumor cell line *in vitro.* In vitro proliferation data of human tumor cell line of H460 non-small cell lung cancer were reported in Table 1 below.

$IC_{50}$ values were obtained by the following methods. The cancer cells were exposed to various concentrations of compounds for 72 hours. Following by washing steps, fresh medium was added to allow the growth of the cancer cells. The cancer cells were stained to obtain cell viability. Then, comparisons were made with the medium-only control.

Specifically, the exponentially growing cells were seeded in 96-well plates at a density of $2\times10^3$ cells/well, and 100 μL/well. The cells were incubated for 24 hours at 37 °C, in 5 % $CO_2$, in 95 % air, and 100 % relative humidity. Next, 99 μL of culture medium was added. The compounds were dissolved in 100 % DMSO at 200 times the desired final test concentration. Upon addition of compounds, the compounds were further diluted to 4 times the desired final concentration with a complete medium. Aliquots of 1 μL of compounds at specific concentration were added to the micro-wells containing 199 μL of medium to obtain the final compound concentrations as reported. After the compounds were added, the plates were incubated for an addition 72 hours at 37 °C, in 5 % $CO_2$, in 95 % air, and 100 % relative humidity.

The plates to be test containing the cells were equilibrated at room temperature for 30 minutes, and 100 μL of medium per well was discarded. 100 μL of CTG reagent (CelltiterGlo Kit) was added to each well. The plates were placed in a fast-shaker to shake for 2 minutes, and placed at room temperature for 30 minutes in the dark. Chemoluminescence signal values were read with Envision instrument. $IC_{50}$ of the drug concentrations resulting in 50 % growth inhibition was calculated with computer software, and the results were listed in Table 1 below.

Table 1: $IC_{50}$ values of compounds

| Number | Compound structure | $IC_{50}$ (nM) | Number | Compound structure | $IC_{50}$ (nM) |
|---|---|---|---|---|---|
| Control 1 | 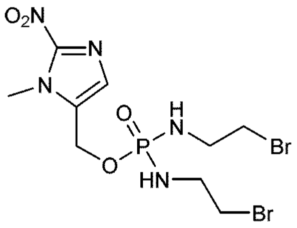 | 800* | 12 | 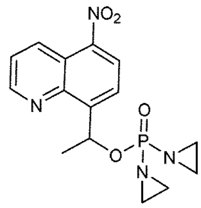 | 35.21 |
| Control 2 | 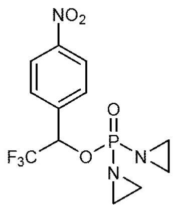 | 658.9 | 13 | 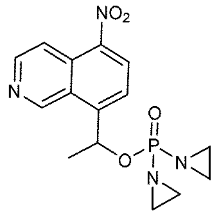 | 12.86 |
| 26 | 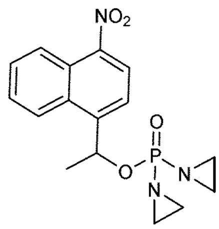 | 92 | 14 | 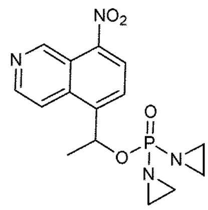 | 3.818 |
| 01 | 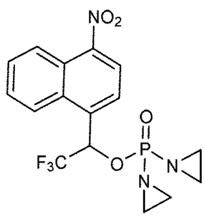 | 128 | 15 | 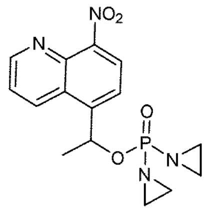 | 145.30 |
| 02 | 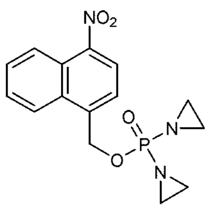 | 755 | 16 | 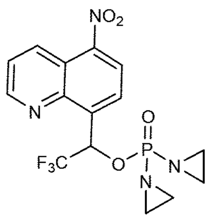 | 34.88 |
| 03 | 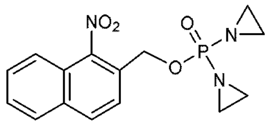 | 805.9 | 17 | 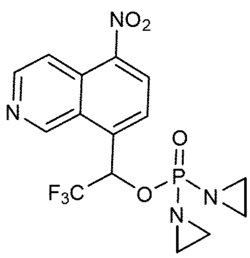 | / |

(continued)

| Number | Compound structure | $IC_{50}$ (nM) | Number | Compound structure | $IC_{50}$ (nM) |
|---|---|---|---|---|---|
| 04 | 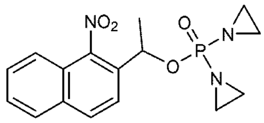 | 9283 | 18 | 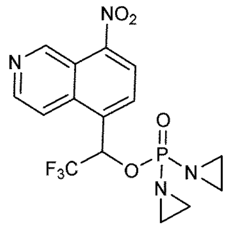 | 7.88 |
| 05 | 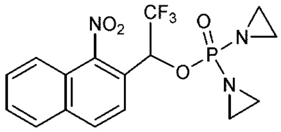 | 9022 | 19 | 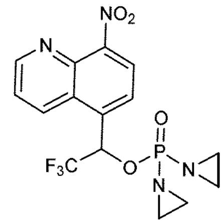 | 311.2 |
| 06 | 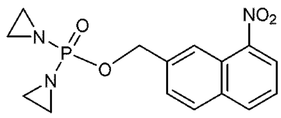 | 172.2 | 20 | 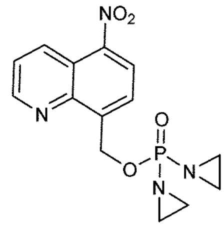 | 41.55 |
| 07 | 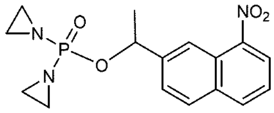 | 168.3 | 21 | 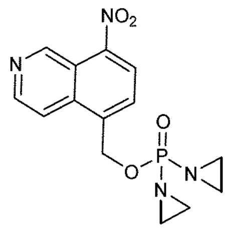 | 7.174 |
| 08 | 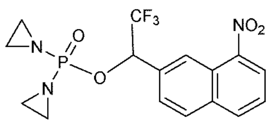 | 3836 | 22 | 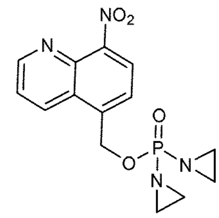 | 199.6 |
| 09 | 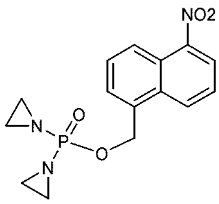 | 1653 | 23 | 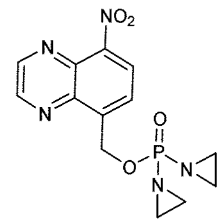 | / |

(continued)

| Number | Compound structure | $IC_{50}$ (nM) | Number | Compound structure | $IC_{50}$ (nM) |
|---|---|---|---|---|---|
| 10 | 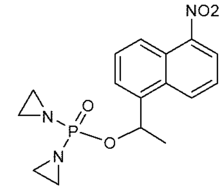 | 691.9 | 24 | 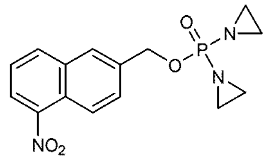 | 478.1 |
| 11 | 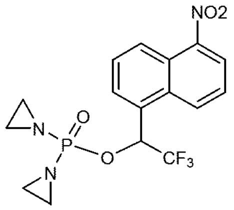 | 7647 | 25 | 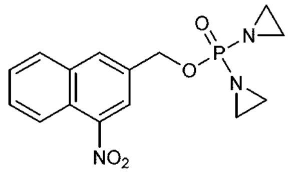 | 106 |
| TH2565 (Control) | 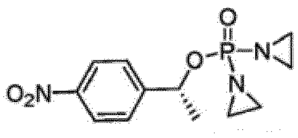 | 100 | TH2566 (Control) | 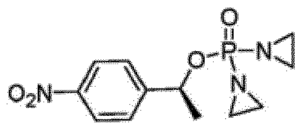 | 100 |

The test result of TH-302 was the values in the published literature tested and published by Fanying Meng, one of the inventors of the present disclosure. The published literature is Molecular and Cellular Pharmacology of the Hypoxia-Activated Prodrug TH-302, Mol Cancer Ther, 2012; 11:740-751. Fanying Meng, James W. Evans, Deepthi Bhupathi, et al.

The test results of TH2565 and TH2566 were the test results in the literatures (WO2016210175A1 and CN108024974A).

By comparing the anticancer activity of an aziridine compound with a parallel ring structure (naphthalene ring, benzopyrazine, benzopyridine) provided by the present disclosure and the anticancer activity of an aziridine compound with the following benzene ring structure provided in the previous literatures (WO2016210175A1 and CN108024974A), the anticancer activity may become stronger (the compound of control 2 compared with compound 01, compound 26 and TH2565/TH2566). In general, the new structural compounds (having a core structure of naphthalene ring, benzopyrazine, benzopyridine with different relative positions of the nitro group and the aziridinyl side chain), provided by the present disclosure, have stronger or roughly equivalent anticancer activity compared to the compound of control 2 (except for compound 04 and compound 05, the anticancer activity of the compound 04 and compound 05 is obviously worse than that of the compound of control 2).

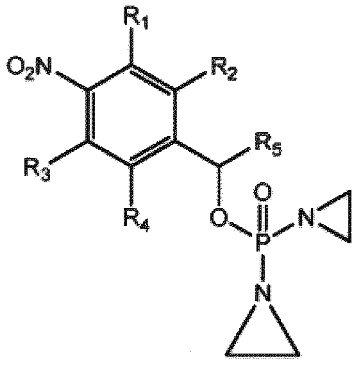

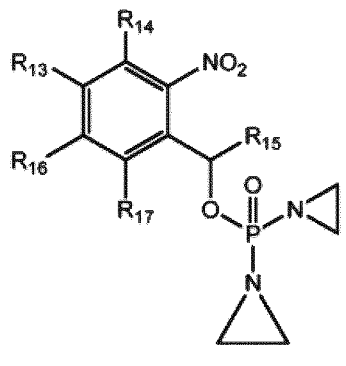

## Effect Example 2: Hypoxia-dependent cytotoxicity experiment

To determine the effect of the compounds of the present disclosure on cancer cell proliferation, these compounds were tested for cancer cell anti-proliferative activity in a porous CTG-based assay. Cell growth was compared in the

present (treatment groups) and the absence of the test compound. 10,000 cells/well/495 μL of medium was used to test the following cell line, a human tumor cell line of H460 non-small cell lung cancer. The cells were seeded on glass-inserts located in each well of 24-well plates with a density and medium as specified above. 24 hours later, the plates were divided into 2 groups, hypoxia (using $N_2$) group and air group. TH-302 at the concentrations from 100, 10, 0.1 to 0.01 μM, and the test compounds at the concentrations from 10, 1, 0.1, 0.01, 0.001 to 0.0001 were added to each well (500 μL volume) of the treatment groups. The final DMSO concentration in each well was lower than or equal to 1%. The cells in the hypoxia group were incubated for 2 hours in an ELECTROTEK hypoxia workstation. The cells in the air group were incubated for 2 hours in a standard culture incubator. 2 hours later, the test compounds were removed from each well. The cells were washed with 500 μL of medium and incubated in 500 μL of fresh medium for 3 days. 3 days later, 200 μL of medium per well was discarded. 60 μL of CTG was added to the wells, and the plates were placed at room temperature for 15 minutes in the dark. 100 μL of medium from each well of the 24-well plates was transferred to 96-well plates. The chemilumiescense signal values were read with an M5 full-band multi-modefunction microplate reader, and integration = 750 ms. The 50% growth inhibitory concentration, $IC_{50}$, of the test compounds was calculated and listed in Table 2 below. The compounds of control 1 and control 2 were used as controls.

Table 2: Inhibitory effects of the compound on H460 cell line under normoxic and hypoxic conditions

| Code | Compound structure | Normoxia: $IC_{50}$ (μM) | $N_2$: $IC_{50}$ (μM) |
|---|---|---|---|
| Control 1 | 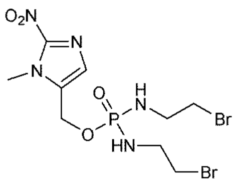 | 7.98 | 0.0479 |
| Control 2 | 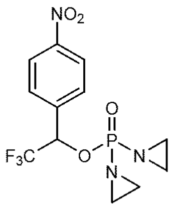 | 15.58 | 7.079 |
| 26 | 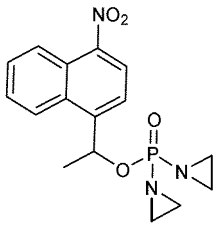 | 0.03761 | <0.0098 |
| 01 | 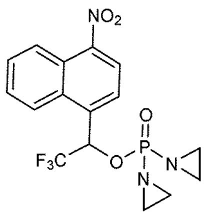 | 0.3313 | 0.02389 |
| 03 | 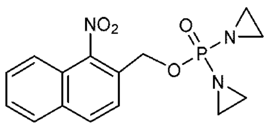 | 10.02 | 0.6335 |
| TH2565 | 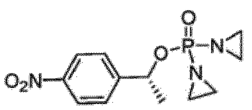 | 0.1 | 18 |

(continued)

| Code | Compound structure | Normoxia: $IC_{50}$ ($\mu$M) | $N_2$: $IC_{50}$ ($\mu$M) |
|---|---|---|---|
| TH2566 | 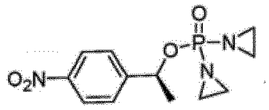 | 0.1 | 23 |

The test results of TH2565 and TH2566 were the test results in the literatures (WO2016210175A1 and CN108024974A).

Besides, inhibitory curves of different concentrations of TH-302 and compound 01 of the present disclosure on H460 cells under the normoxic air and the hypoxia nitrogen conditions are shown in FIG. 1.

The results indicates that the aziridine compounds with the parallel ring structure (naphthalene ring, benzopyrazine, benzopyridine) provided by the present disclosure has the same hypoxia activation mechanism as TH-302. That is, under hypoxia condition, the aziridine compounds of the present disclosure are more cytotoxic to cancer cells than under normoxic condition.

**Effect Example 3: P-gP inhibitory assay**

P-glycoprotein (P-gP, also known as multidrug resistance protein) is a high molecular weight protein found on the plasma membrane of multidrug-resistant tumor cells with a transport pump-like structure. P-gP pumps out a variety of chemotherapeutic drugs out of cells and reduced the intracellular drug concentration, which is closely relative to the drug resistance of clinical chemotherapy.

The action of chemotherapy drugs in treating tumors or cancers of the central nervous system (CNS), such as brains, is limited. It is mainly due to the blood brain barrier (BBB), which makes the chemotherapy drugs less permeable to tumor system or tumor tissue. That is, the chemotherapy drugs cannot cross the blood brain barrier and enter the brains to kill cancer cells.

The process of small molecules, such as chemotherapy drugs, crossing the blood brain barrier is more complicated. The blood brain barrier is located between the systemic blood circulation and the cerebrospinal fluid. The blood brain barrier is formed by specialized brain microvascular endothelial cells, along with surrounding cells and perivascular astrocytes through tight junctions between adjacent cells. Rather than forming a physical barrier around vascular endothelial cells, the blood brain barrier forces most molecules to pass through as a selective barrier. The transport system of the membrane of the blood brain barrier allows the passage of hydrophilic small molecules. However, large hydrophilic molecules, comprising many chemotherapeutic and macromolecular drugs, are excluded from the central nervous system unless the large hydrophilic molecules can be actively transported by certain proteins. More importantly, the blood brain barrier has a "drug efflux pump," for example P-glycoprotein, to protect brain tissue, which may actively exclude some chemotherapeutic and macromolecular drugs from the brains. Therefore, even if the small molecule chemotherapeutic drugs (comprising small molecular targeted antitumor drugs molecules) with a certain hydrophilicity may cross the blood brain barrier and enter the brain to play a role. Yet, under the action of P-glycoprotein, the small molecule chemotherapeutic drugs will still be excluded from the central nervous system and cannot really work. In other words, the transmembrane structure of P-glycoprotein has an energy-dependent "drug pump" function, which may pump lipophilic drugs, such as Vincristine (VCR), Doxorubicin (Dox), or Vepesid (VP-16), out of the cell, resulting in a decrease of intracellular drug concentration and a reduction or complete loss of cytotoxicity. Accordingly, experimental data on the interaction of P-glycoprotein with these compounds may be used to evaluate the real degree of efficacy of these compounds on the inhibition of CNS tumor cell proliferation. If a compound is experimentally proven to be a substrate of P-gp glycoprotein, then the compound may bind to P-gp and be excluded from the central nervous system without actually working. If a compound is experimentally proven not to be a substrate of P-gp glycoprotein, then the compound either may not bind to P-gp or binds weakly, so as to not be excluded from the central nervous system. Hence, the compound may accumulate sufficient concentration in the central nervous system and take effect.

MDCKII-MDR1 cells were used as a model and verapamil was used as an inhibitor to evaluate the potential of the compounds as P-glycoprotein substrates.

**Preparation of cells for penetration assay:**

MDCKII-MDR1 cells were incubated in cell culture flasks. The incubator was set at 37 °C, 5% $CO_2$, and a guaranteed relative humidity of 95 %. When the cells reached 70 to 90 % confluence, the cells may be seeded to transwells.

Before the cells were seeded, 50 $\mu$L of cell culture medium was added to apical chambers of each well of the

transwells, and 25 mL of cell culture medium was added to basolateral chambers of each well of the transwells. The transwells may be used to seed the cells after 1 hour of incubation in the 37 °C and 5 % $CO_2$ incubator.

After cell incubation, the cell suspensions were transferred to a round-bottom centrifuge tube and centrifuged at 120 g for 5 minutes.

The medium was used to resuspend the cells to a final concentration of $1.56\times10^6$ cells/mL. 50 μL of the cell suspensions were added to the apical chambers of each well of the 96-well transwells at a final seeding density of $5.45\times10^5$ cells/$cm^2$.

The medium was changed at 48 hours after seeded, and the medium was changed every other day to incubate for 4 to 8 days.

The procedure of replacing the medium was as follows. The apical chambers were separated from the basolateral chambers. The medium in the acceptor chambers was discarded first, and then the medium in the donor chambers was discarded. Finally, 75 μL of fresh medium was added to the donor chambers of each well, and 25 mL of the fresh medium was added to the acceptor chambers of each well.

**Evaluation of cell monolayer membrane integrity:**

MDCKII-MDR1 cells may be fully confluent and differentiated after 4 to 8 days of incubation. At this time, the cells may be applied to penetration test. The monolayer membrane resistance was measured with a resistance meter (Millipore), and the resistance per well was recorded. After the test, the transwells were returned to the incubator.

Calculation of resistance value: measured resistance value (ohms) × membrane area ($cm^2$) = TEER value (ohm·$cm^2$). If the TEER value was less than 42 ohm·$cm^2$, the well cannot be used for penetration test.

**Drug penetration test:**

The transwells with MDCKII-MDR1 cells were taken from the incubator. The cell monolayers were rinsed twice with buffer and incubated at 37 °C for 30 minutes.

The rate of the compounds from an apical end to a basolateral end was determined. 75 μL of the buffer containing the test sample was added to each well of the apical chambers (apical end), and 235 μL of the buffer was added to each well of the basolateral chambers (basolateral end).

The rate of the compounds transporting from the basolateral end to the apical end was determined. 75 μL of the buffer was added to the apical chambers (apical end), and 235 μL of the buffer containing the test sample was added to the basolateral chamber (basolateral end).

50 μL of sample from a working solution preparation plate was transferred to a plate with 200 μL of acetonitrile (100 nM of Alprazolam, 200 nM of Labetalol, 200 nM of caffeine, and 2 μM of Ketoprofen) as an internal standard at 0 minute for administering sample for detection.

In order to determine the transport of the test samples under the condition of adding the P-glycoprotein inhibitor, Verapamil, it is necessary to add Verapamil to both the buffers in the donor end and the acceptor end of the MDCKII-MDR1 transwells, and the final concentration was 100 μM.

After the apical chambers and the basolateral chambers were combined respectively, the transwells were incubated at 37 °C for 2 hours.

After incubation, 50 μL of the sample from each well of the apical chambers and the basolateral chambers of the transwells was transferred to new sample tubes respectively. 200 μL of acetonitrile (100 nM of Alprazolam, 200 nM of Labetalol, 200 nM of caffeine, and 2 μM of Ketoprofen) as an internal standard was added to the sample tubes, vortexed for 10 minutes, and centrifuged at 3220 g for 30 minutes. 100 μL of the supernatant was taken and diluted with an equal volume of water for LC-MS/MS analysis. Each sample was incubated in triplicate.

Leakage evaluation of fluorescent yellow was used to estimate the integrity of cell monolayer membrane after 2 hours of incubation. The fluorescent yellow stock solution was diluted with buffer to a final concentration of 100 μM/L. 100 μL of the fluorescent yellow solution was added to each well of the apical chambers of the transwells, and 300 μL of buffer was added to each well of the basolateral acceptor chambers of the transwells. After 30 minutes of incubation at 37 °C, 80 μL of the solution from each well of the apical chambers and the basolateral chambers was transferred to a new 96-well plate, respectively. Fluorescence was measured by the microplate reader with an excitation wavelength of 480 nm and an emission wavelength of 530 nm.

**Data analysis**

Peak-areas were calculated from ion chromatography results. The apparent permeability coefficient (Papp, unit: cm/s×10-6) of the compounds was calculated by the following formula.

$$P_{app} = \frac{V_A}{Area \times time} \times \frac{[drug]_{acceptor}}{[drug]_{initial,donor}}$$

In the formula, $V_A$ was the volume of the solution at the acceptor end (Ap→B1 was 0.235 mL, and B1→Ap was 0.075 mL), Area was the membrane area (0.143 $cm^2$) of 96-well transwell, time was the incubation time (unit: s), $[drug]_{acceptor}$ referred to the drug concentration of the acceptor end after incubation, and $[drug]_{initial,donor}$ referred to the initial concentration of the donor end before incubation.

Efflux ratio was calculated with the following formula.

$$Efflux\ Ratio = \frac{P_{app\ (B-A)}}{P_{app\ (A-B)}}$$

In the formula, $Papp_{(B-A)}$ was the apparent permeability coefficient from the basolateral end to the apical end, and $Papp_{(A-B)}$ was the apparent permeability coefficient from the apical end to the basolateral end.

The recovery rate was calculated with the following formula.

$$Re\,cov\,ery\% = \frac{[drug]_{acceptor} \times V_A + [drug]_{donor} \times V_D}{[drug]_{initial,donor} \times V_D} \times 100$$

In the formula, $V_A$ was the solution volume of the acceptor end (unit: mL), $V_D$ was the solution volume of the donor end (unit: mL), $[drug]_{acceptor}$ referred to the drug concentration of the acceptor end after incubation, and $[drug]_{initial,donor}$ referred to the initial concentration of the donor end before incubation.

The fluorescence value of the cell monolayer (LY Leakage) was calculated with the following formula.

$$\text{LY Leakage} = \left( \frac{I_{acceptor} \times 0.3}{I_{acceptor} \times 0.3 + I_{donor} \times 0.1} \right) \times 100$$

In the formula, Iacceptor referred to a fluorescence density of the accepter well (0.3 mL), Idonor referred to a fluorescence density of the donor well (0.1 mL), expressed in %LY. LY<1.5% indicates that the monolayer membrane is intact.

Parts of the prepared compounds were tested to obtain the efflux ratio of different compounds in the presence or absence of the P-glycoprotein inhibitor, Verapamil. The experimental data were shown in Table 3 below.

Table 3: Experimental results of compound efflux ratio

| Compound | Verapamil (μM) | Papp (A-B) ($10^{-6}$, cm/s) | Papp (B-A) ($10^{-6}$, cm/s) | Efflux ratio | Recovery (%) | |
|---|---|---|---|---|---|---|
| | | | | | AP-BL | BL-AP |
| Metoprolol | 0 | 26.01 | 18.93 | 0.73 | 84.87 | 89.45 |
| Prazosin | 0 | 13.96 | 28.23 | 2.02 | 81.88 | 75.47 |
| Prazosin | 100 | 28.47 | 14.14 | 0.50 | 84.58 | 81.33 |
| Imatinib | 0 | 2.24 | 24.07 | 10.76 | 67.41 | 58.70 |
| Imatinib | 100 | 21.94 | 18.69 | 0.85 | 77.02 | 65.23 |
| 14 | 0 | 12.94 | 26.31 | 2.03 | 91.14 | 103.86 |
| 14 | 100 | 16.68 | 14.10 | 0.85 | 80.80 | 90.81 |
| 20 | 0 | 33.72 | 21.76 | 0.65 | 92.71 | 96.26 |
| 20 | 100 | 34.16 | 22.71 | 0.66 | 87.60 | 96.15 |
| 01 | 0 | 18.56 | 11.95 | 0.64 | 58.56 | 58.59 |

(continued)

| Compound | Verapamil (μM) | Papp (A-B) ($10^{-6}$, cm/s) | Papp (B-A) ($10^{-6}$, cm/s) | Efflux ratio | Recovery (%) | |
|---|---|---|---|---|---|---|
| | | | | | AP-BL | BL-AP |
| 01 | 100 | 22.19 | 12.47 | 0.56 | 63.37 | 67.28 |

The experiments indicate that Metoprolol is a known compound with high passive permeability, while Prazosin and Imatinib are known P-gp substrates. The experiments used these three compounds as controls to verify the reliability of the experimental data.

**Experimental conclusion**

The 3 critical parameters of Papp(A-B), Papp(B-A), and Efflux Ratio of compounds 01, 14, and 20 are very close in the presence or absence of the P-glycoprotein inhibitor, Verapamil. As a result, the 3 compounds are not P-glycoprotein substrates.

The experimental data previously tested by the inventor's team, the subsequent clinical trials of TH-302 drugs (the clinical trial registration numbers in US: NCT01497444, NCT02712567, NCT01403610, NCT00495144, NCT01746979, NCT02093962, NCT02342379, NCT02047500, NCT01381822, NCT02076230, NCT01440088, NCT03098160, NCT02598687, NCT02402062, NCT01144455, NCT01833546, NCT00743379, NCT01864538, NCT02433639, NCT00742963, NCT01149915, NCT02020226, NCT01485042, NCT01522872, NCT02255110), and the related literatures of clinical trials (Chawla S P, Cranmer L D, Van Tine B A, et al. Phase II Study of the Safety and Antitumor Activity of the Hypoxia-Activated Prodrug TH-302 in Combination With Doxorubicin in Patients With Advanced Soft Tissue Sarcoma[J]. Journal of Clinical Oncology, 2014, 32 (29): 3299-3306 ; Weiss G J, Infante J R, Chiorean E G, et al. Phase 1 Study of the Safety, Tolerability, and Pharmacokinetics of TH-302, a Hypoxia-Activated Prodrug, in Patients with Advanced Solid Malignancies[J]. Clinical Cancer Research, 2011, 17(9): 2997-3004 ; Borad M J, Reddy S G, Bahary N, et al. Randomized Phase II Trial of Gemcitabine Plus TH-302 Versus Gemcitabine in Patients With Advanced Pancreatic Cancer [J]. Journal of Clinical Oncology, 2015, 33(13): 1475-1481 ; Brenner A, Reardon D A, Wen P Y, et al. ACTR-17. Evophosphamide (th-302) for recurrent gbm following bevacizumab failure, final results of a multicenter phase ii study [J]. Neuro-oncology, 2018.) have all proven that TH-302 is a P-gp substrate. The compound cannot effectively enter to the central nervous system, such as the brain, and does not easily or basically unable to accumulate enough concentration to exert the drug effect on the central nervous system.

The above experiments indicate that the anticancer compounds of the new structure with the naphthalene ring provided by the present disclosure were not the P-gp substrates. Therefore, the anticancer compounds provided by the present disclosure are expected to be drugs for treating primary brain cancers or tumors, metastatic cancers or tumors that metastasizes to the brains, or used for treating primary brain cancers or tumors, metastatic cancers or tumors that metastasizes to the brains.

**Effect Example 4: Cytotoxicity comparison of TH-302 and compound 01**

To further compare the cytotoxicity of TH-302 with compound 01, a comparison of their cytotoxicity in different cell lines was further provided.

**(1) H460 cell line**

The cytotoxicity test results of TH-302 and compound 01 on H460 cell line under different test times in the same laboratory, the same operator, and the same experimental conditions. The results were shown in Table 4.

Table 4: Comparison data of test results of the same cell line at different times

| The same laboratory, the same operator, different test times with the same experimental conditions | Exposure for 2hours; air; $IC_{50}$ (μM) | | Multiple |
|---|---|---|---|
| | TH-302 | Compound 01 | TH-302/ Compound 01 |
| Time 1 | 23 | 0.7 | 32.9 |
| Time 2 | 8 | 0.3 | 26.7 |

(continued)

| The same laboratory, the same operator, different test times with the same experimental conditions | Exposure for 2hours; air; $IC_{50}$ ($\mu$M) | | Multiple |
|---|---|---|---|
| | TH-302 | Compound 01 | TH-302/ Compound 01 |
| Time 3 | 3.3 | 0.5 | 6.6 |

**(2) Different species cell lines**

The cytotoxicity test results of TH-302 and compound 01 on cell lines of different species were shown in Table 5.

Table 5: Comparison data of test results of cell lines of different species

| Cell line | Species | $IC_{50}$ ($\mu$M) | |
|---|---|---|---|
| | | Compound 01 (Exposure time: 3 days) | TH-302 (Exposure time: 72h) |
| H460 | Human | 0.1 | 20 |
| AA8 | Rat | 11 | 830 |
| Multiple : Rat/Human | | 110 | 41.5 |

The preceding cytotoxicity test results indicate that compound 01 of the present disclosure is more toxic to cancer cells than TH-302. That is, compound 01 of the present disclosure has a stronger inhibitory effect on the proliferation of cancer cells.

**Effect Example 5: Cytotoxicity of compound 01 on UV41** cell line **with DNA repair gene mutation**

UV41 cell line is a derivative of CHO-AA8 cell line, derived from the UV-sensitive line of AA8. The cell line is a cell line with damaged homologous recombination DNA repair enzymes and damaged nucleotide excision repair enzymes caused by mutations in ERCC4/XPF gene of DNA.

It has been reported that the UV41 cell line is deficient in nucleotide excision repair enzymes relative to AA8 (referred to Thompson LH, et al. Repair of DNA adducts in asynchronous CHO cells and the role of repair in cell killing and mutation induction in synchronous cells treated with 7-bromomethylbenz[a]anthracene. Somatic Cell Mol. Genet. 10: 183-194, 1984. PubMed: 6584989 ; Thompson LH, et al. Genetic diversity of UV-sensitive DNA repair mutants of Chinese hamster ovary cells. Proc. Natl. Acad. Sci. USA 78: 3734-3737, 1981. PubMed: 6943579 ; Hoy CA, et al. Defective DNA cross-link removal in Chinese hamster cell mutants hypersensitive to bifunctional alkylating agents. Cancer Res. 45: 1737-1743, 1985. PubMed: 3919945 ; Busch D, et al. Summary of complementation groups of UV-sensitive CHO cell mutants isolated by large-scale screening. Mutagenesis 4: 349-354, 1989. PubMed: 2687628 ; Bessho T, et al. Initiation of DNA interstrand cross-link repair in humans: the nucleotide excision repair system makes dual incisions 5" to the cross-linked base and removes a 22- to 28-nucleotide-long damage-free strand. Mol. Cell. Biol. 17: 6822-6830, 1997. PubMed: 9372913 ; Thompson LH, et al. Hypersensitivity to mutation and sister-chromatid-exchange induction in CHO cell mutants defective in incising DNA containing UV lesions. Somatic Cell Genet. 8: 759-773, 1982. PubMed: 7163954).

The damage of DNA repair enzymes in the UV41 cell line may result in the mutation of any one or more genes corresponding to BRCA1, BRCA2, FANCA, FANCD1, FANCD2, ATM, ATR, CHEK1, CHEK2, CTP, BARD1, BRIP1, PALB2, RAD51D, RAD51C, RAD52, RAD54, RAD55, RAD57, FAM175, NBN, Rad50, MRE11, p53, NBS1, XRS2, XRCC2, XRCC3, XRCC4/XPF, ERCC1, ERCC2/XPD, ERCC3/XPB, ERCC4/XPF, XRCC1, Ku80, MHS6, MGMT, PARP, ERCC5/XPG, CCNH, CDK7, CETN2, DDB1, DDB2, ERCC5 /XPG, ERCC6/CSB, ERCC8/CSA, LIG1/DNA Ligase I, MMS19, MNAT1, RAD23A, RAD23B, RPA1, RPA2, TFIIH, XAB2, XPA, XPC, MBD4, NEIL1, BAP1, CDK12, EXO1, FAAP20, FAN1, FANCE, FANCM, MDC1, NONO, POLQ, RAD51B, RBBP8, SMC5, USP11, WRN and AP endonucleases, End processing enzymes, DNA polymerase, Flap endonuclease.

Specifically, the exponentially growing cells were seeded in 96-well plates at a density of $4\times10^3$ cells/well and incubated at 37 °C, in 5 % $CO_2$, 95 % air, and 100 % relative humidity. Next, 24 hours later, the test compounds were added. The test compounds were dissolved in 100 % DMSO at 200 times the desired final test concentration. Upon addition of the test compounds, the test compounds were further diluted with the complete medium to 4 times the desired

final concentration. Aliquots of 50 μL of the compounds at specific concentrations were added to the microwells containing 150 μL of medium to obtain the final compound concentrations as reported. After the test compounds were added, the plates were incubated for an addition 2 hours at 37 °C, in 5 % $CO_2$, 95 % air, and 100 % relative humidity. Then, the compounds were washed off, and fresh medium was added to the plates. The plates were incubated for an addition 70 hours at 37 °C, in 5 % $CO_2$, 95 % air, and 100 % relative humidity. At the end of this incubation, AlamarBlue reagent was used to quantify viable cells. The concentration of the drugs causing 50 % growth inhibition (IC 50) was calculated with computer software. The results showed that the $IC_{50}$ of compound 01 on AA8 cell line was 10.85 μmol/L, while the $IC_{50}$ of UV41 cell line with DNA gene repair mutation was 0.34 μmol/L, as shown in FIG. 2.

The results indicate that the compounds of the present disclosure have higher sensitivity to the cell lines with mutation in DNA repair genes. Specifically, compound 01 was more sensitive to the cell lines with damaged homologous recombination DNA repair enzymes and damaged nucleotide excision repair enzymes. As well, compound 01 has a stronger proliferation inhibitory effect on cell lines with damaged homologous recombination DNA repair enzymes and damaged nucleotide excision repair enzymes.

**Effect Example 6: Hypoxia-dependent reduction experiment of compound 01 and TH-302**

Ingredients were added to each tube without NADPH. Next, 50 μL of the condition B samples were transferred into 100 μL of acetonitrile (T = 0). For the samples to be tested under hypoxia condition, the tubes were placed in a hypoxia chamber (BACTRON anaerobic/environmental chamber). The reactions were initiated by adding NADPH to tube A and tube C. Then, the samples with NADPH were placed at 37 °C. For the samples to be tested in air condition, the tubes were placed in a hot plate at 37 °C. After 30 minutes and 2 hours of incubation, 50 μL of samples were taken from each tube and mixed with 100 μL of acetonitrile. All tubes were spun to remove any protein precipitate and placed in HPLC vials for analysis. The results were compared with the standard curve of the authentic standard. The specific composition of the reagents in each sample reaction tube was shown as follow.

| Sample tube | PBS solution | Test drug | CYP450R enzyme solution | NADPH solution |
|---|---|---|---|---|
| A | 450 μL | 10 μL | 20 μL | 20 μL |
| B | 470 μL | 10 μL | 20 μL | 0 μL |
| C | 470 μL | 10 μL | 0 μL | 20 μL |

The drugs relative to the initial amount of the test drug (TH-302, compound 01) of the 3 different sample reaction tubes of A/B/C that have been reacted for different times (0/ 30 min/ 120 min). The results of adding different concentrations of human NADPH coenzyme were shown in FIG.3 and FIG. 4, respectively.

**Experimental Conclusion:**

In the present of the NADPH and CYP450R enzymes, compound 01 showed the hypoxia-dependent reduction reaction. Also, the reduction ratio of compound 01 and TH-302 was similar.

**Effect Example 7: Metabolic stability evaluation experiment**

Two independent experiments were performed.

**7.1 Hepatomicrosome stability experiment**

1a) With NADPH: 10 μL of 20 mg/mL of hepatomicrosome and 40 μL of 10 mM of NADPH were added to medium. The final concentrations of hepatomicrosome and NADPH were 0.5 mg/mL and 1 mM, respectively.

1b) Without NADPH: 10 μL of 20 mg/mL hepatomicrosome and 40 μL of μltra-pure $H_2O$ were added to medium. The final concentration of hepatomicrosome was 0.5 mg/mL.

2) Reactions were started by adding 4 μL of 200 μM test compound solution or control compound solution (verapamil) at a final concentration of 2 μM and incubated at 37°C.

3) 3) Aliquots of 50 μL from the reaction solutions at 0 minute and 30 minutes were taken. The reaction solutions were stopped by adding 4 volumes of cold acetonitrile and 4 internal standards (IS internal standard, 100 nM of Alprazolam, 200 nM of caffeine, 200 nM of Labetalol, and 2 μM of Ketoprofen) to stop the reaction solution. The samples were

centrifuged at 3,220 g for 40 minutes. Aliquots of 100 μL of the supernatants were mixed with 100 μL of μltra-pure $H_2O$. Then, the mixtures were used for LC-MS/MS analysis. All experiments were performed in duplicate. The test results were as follow.

| Compound | Remaining percentage after 30 minutes | | | | | | | | | |
|---|---|---|---|---|---|---|---|---|---|---|
| | Human | | Monkey | | Dog | | Rat | | Mouse | |
| | With NADPH | Without NADPH | With NADPH | Without NADPH | With NADPH | Without NADPH | With NADPH | Without NADPH | With NADPH | Without NADPH |
| Verapamil | 15.55 | 98.27 | 0.19 | 92.44 | 15.73 | 97.58 | 1.13 | 89.24 | 2.91 | 93.95 |
| Compound 01 | 96.39 | 82.15 | - | - | 31.16 | 101.62 | 71.05 | 111.15 | 102.89 | 103.07 |

The test data of compound 01 was prepared as a graph, as shown in FIG. 5.
Conclusion: Compound 01 is relatively stable in the hepatomicrosomes of human and mice, but unstable in the hepatomicrosomes of rat and dog.

7.2 **Plasma stability experiment**

1) **Plasma preparation**

Frozen plasma was melted immediately in a water bath at 37 °C. The plasma was centrifuged at 3,220 g for 10 minutes to remove blood clots. The supernatants were collected into new tubes, respectively. pH values of the plasma were checked and recorded. Note: a, no more 2 times melted plasma was used since the sample was received; b, the plasma in pH 7 to pH 8 was used.

**2) Experimental solution preparation**

1 mM of working solution of the test compounds and the control compound, propantheline, were prepared in DMSO. 1 mM of working solution of the control compound in propane was prepared in acetonitrile. 4 μL of the working solutions were added to 796 μL of pre-incubated plasma respectively to achieve a final concentration of 5 μM. The final concentration of solvent was 0.5%.

3) **Test protocol for plasma stability**

Aliquots of 50 μL of labelled plasma were transferred to new tubes and incubated in a water bath at 37 °C at approximately 60 rpm for 2 hours. The test was performed in duplicate. After 2 hours of incubation, 300 μL of quench solution (acetonitrile with internal standard (acetonitrile, 100 nM of Alprazolam, 500 nM of Labetalol, and 2 μM of Ketoprofen)) of room temperature was added to stop the reaction. 50 μL of the labeled plasma was added to new tubes containing 300 μL of the quench solution of room temperature, i.e., the sample with a preparation time of 0. The samples were vortexed to mix for 5 minutes. The samples in the plates were centrifuged at 3,220 g for 30 minutes at 4 °C to precipitate the proteins. 100 μL of the supernatant was transferred to new plates. The supernatant was diluted with 100 μL of water, mixed well, and analyzed by LC-MS/MS.
The test results after 30 minutes were as follow.

| Compound | Species | Remaining percentage |
|---|---|---|
| Propantheline | Human | 0.07 |
| Lovastatin | Rat | 0.21 |
| Propantheline | Mouse | 5.33 |
| Lovastatin | Dog | 37.6 |
| Propantheline | Monkey | 48.11 |
| Compound 01 | Human | 68.68 |
| Compound 01 | Rat | 52.11 |
| Compound 01 | Mouse | 55.07 |
| Compound 01 | Dog | 85.04 |

In the table, Propantheline and Lovastatin are commonly used as control drugs in this experiment.
The test data of compound 01 were prepared as a graph, as shown in FIG. 6.
Conclusion: Compound 01 is relatively stable in the plasma of dog, followed by the plasma of human. Yet, compound 01 is relatively unstable on the plasma of rat and mouse.

**Effect Example 8: Human-derived xenografts (patient-derived xenografts, PDX) model**

BALB/c nude mice were subcutaneously inoculated with GA6201 tumor of HuPrime® model to establish a subcutaneously transplanted tumor model of human gastric cancer. The experiment was divided into a vehicle control group 1 with normal saline (pH 7.0-7.6), a control group 2 with ifosfamide (60 mg/kg), and an Experimental group (Exp.

group) with compound 01 (5-50 mg/kg), and 5 mice per group. Besides, the vehicle control group 1 with normal saline (pH 7.0-7.6) was administered by tail vein injection (i.v.) once a week for 3 weeks and observed for 4 weeks.

The control group 2 with ifosfamide (60 mg/kg) was administered by intraperitoneal injection (i.p.) for 5 consecutive days and stopped for 2 days a week, administered for 2 weeks, and observed for 5 weeks. The Exp. group with compound 01 (5-50 mg/kg) was administered by intraperitoneal injection for 5 consecutive days and stopped for 2 days a week, and administered for 2 weeks. Then, the dose was changed to 10 mg/kg by intraperitoneal injection, administered daily for 4 days. After that, the dose and administration method were changed. The dose was 20 mg/kg by tail vein injection for 1 day. After 10 days of drug withdrawal, the dose was changed again. The dose was 50 mg/kg by tail vein injection, once a week, for 2 weeks, and one week of observation. The efficacy was evaluated according to the relative tumor inhibition rate TGI (%). The safety was evaluated according to the changes in animal body weight and death.

The tumor volume of each group of mice was measured on different days, and the average value was obtained. The results were shown in the following table and FIG. 7.

| Group | Changes in tumor volume on different days of inoculation; the unit of the volume was $mm^3$ | | | | | | | | | | | | | | |
|---|---|---|---|---|---|---|---|---|---|---|---|---|---|---|---|
| | 0 | 3 | 7 | 10 | 14 | 17 | 21 | 24 | 28 | 31 | 35 | 38 | 42 | 45 | 49 |
| Control 1 | 94.22 | 123.26 | 161.08 | 182.31 | 242.26 | 297.28 | 398.37 | 479.52 | 579.90 | 648.12 | 831.15 | 905.75 | 1026.01 | 1130.43 | 1283.11 |
| Control 2 | 94.15 | 122.22 | 129.39 | 175.00 | 190.74 | 189.23 | 217.96 | 225.66 | 247.04 | 265.76 | 324.51 | 371.02 | 403.47 | 484.83 | 578.60 |
| Exp. | 94.24 | 121.79 | 123.19 | 153.46 | 191.17 | 219.91 | 246.57 | 271.72 | 280.86 | 270.68 | 299.31 | 277.17 | 300.46 | 342.08 | 287.47 |

Additionally, the body weight change rates of each group of mice on different days were calculated. The results were shown in FIG. 8.

The above-mentioned dosing regimen, ifosfamide, was designed according to the best dosing regimen and the maximum safe dosage in the literature (Jessica D. Sun, Qian Liu, Dharmendra Ahluwalia, Damien J. Ferraro, Yan Wang, Don Jung, Mark D. Matteucci,and Charles P. Hart. Comparison of hypoxia-activated prodrug evofosfamide (TH-302) and ifosfamide in preclinical non-small cell lung cancer models [J]. Cancer Biology & Therapy, 2016, 17(4): 371-380.). The dosing regimen of compound 01 with dose-increasing dosing regimen was designed by experience based on previous cytotoxicity experiments and MTD experiment.

Conclusion: The results indicate that compound 01 of the present disclosure has obvious advantages in inhibiting tumor growth compared with the classical chemotherapeutic drug, ifosfamide (which is also a drug with a mechanism of a DNA alkylating agent).

**Effect Example 9: Experiment of human-derived xenograft animal model (cell-linederived xenograft, CDX)**

BALB/c nude mice were subcutaneously inoculated with human lung cancer cells, NCI-H460 cells, to establish a subcutaneously transplanted tumor model of human lung cancer. The experiment was divided into a 20 mg/kg of the test drug compound 01 group, a 40mg/kg of the test drug compound 01 group, and a vehicle control group with 10 % of ethanol + 10 % of polyoxyethylene (35) castor oil, Cremophor EL + 80% of 5 % of glucose injection (pH7.4), and 6 mice in each group. Besides, the vehicle control group with 10 % of ethanol + 10 % of polyoxyethylene (35) castor oil, Cremophor EL + 80% of 5 % of glucose injection (pH7.4) was administered by tail vein injection once a week for three weeks and observed for once a week. The 40 mg/kg of the test drug compound 01 group was administered by tail vein injection, once every three weeks, for a total of 2 administrations, and observed for a week after the second administration. The 20 mg/kg of the test drug compound 01 group was administered by intraperitoneal injection once a day for 5 consecutive days and observed for 24 days. The efficacy was evaluated based on the relative tumor inhibition rate TGI (%). The safety evaluation was performed based on changes in animal body weight and mortality. The administered doses of compound 01 were determined based on the MTD experiment.

The tumor volume of each group of mice was measured on different days respectively, and the average value was obtained. The results were shown in the following table and FIG. 9.

| Group | The change of tumor volume on different days of inoculation, the unit of volume was $mm^3$ | | | | | | | | |
|---|---|---|---|---|---|---|---|---|---|
| | 7 | 10 | 14 | 17 | 21 | 24 | 28 | 31 | 35 |
| Control group | 144.68 | 226.31 | 339.60 | 493.01 | 774.49 | 977.77 | 1370.471 | 1818.22 | 2299.37 |
| Exp. Group 7 | 143.84 | 184.06 | 153.75 | 159.72 | 218.13 | 291.07 | 520.17 | 580.70 | 447.32 |
| Exp. Group 8 | 144.63 | 173.09 | 152.99 | 130.06 | 129.66 | 164.63 | 272.20 | 447.50 | 651.58 |

Additionally, the body weight change rate of each group of mice on different days was calculated. The results were shown in FIG. 10.

Conclusion: The treatment group of single-drug of 40mg/kg of compound 01 (Q3Wx2, IV, Exp. group 7) shows a significant tumor inhibitory effect on the 31$^{st}$ day after tumor cell inoculation. Compared with the control group, there is a statistically significant difference (p= 0.00561) and relative tumor inhibition rate TGI (%) is 66.87 %. The treatment group of single-drug of 20mg/kg of compound 01 (QDx5xlweek, IP, Exp. group 8) shows a significant tumor inhibitory effect on the 31$^{st}$ day after tumor cell inoculation. Compared with the control group, there is a statistically significant difference (p= 0.000464) and relative tumor inhibition rate TGI (%) is 76.22 %.

**Effect Example 10: Experiment of Maximal tolerance dose, MTD**

**1. S-configuration of compound 01**

**Experiment procedure**

18 ICR mice, 9 females/9 males, were selected for the experiment. According to gender and body weight, the mice were randomly divided into a 10mg/kg group, a 20mg/kg group, and a 40mg/kg group of the S-configuration of compound 01, with 3 females/ 3 males in each group. By intravenous injection, the 10 mg/kg group of the S-configuration of compound 01 was administered with 1 mg/ml of the administration concentration, 10 ml/kg of the administration volume, and 10 mg/kg of the dose. The 20 mg/kg group containing the S-configuration of compound 01 was administered

with 2 mg/ml of the administration concentration, 10 ml/kg of the administration volume, and 20 mg/kg of the dose. The 40mg/kg group containing the S-configuration of compound 01 was administered with 4 mg/ml of the administration concentration, 10 ml/kg of the administration volume, and 40 mg/kg of the dose. The mice was administered once a day for 5 consecutive days, and recovered for 2 weeks after the last administration. The mice were closely observed for 2 hours after administration of the daily drug. The mice were observed twice a day in the morning and afternoon on the non-administration day. During the experiment, body weight and food intake were measured twice a week. The dead animals and all the survival animals were subjected to gross necropsy on the $14^{th}$ day after the last administration. The diseased organic were grossly necropsied for histopathological examination.

**Experimental results**

(1) Animals death conditions

During the experiment, 3 of the 6 animals in the 40 mg/kg group of the S-configuration of compound 01 died. The death time was mainly during the recovery period of 2 to 6 days.

(2) Clinical symptoms of animals after administration

During the experiment, the general state of the animals of the 10 mg/kg group and the 20 mg/kg group of the S-configuration of compound 01 was in good condition. The animals of the 40 mg/kg group containing the S-configuration of compound 01 intermittently appeared unsteady gait, deepened breathing, accelerated breathing, and decreased spontaneous activity. During the recovery period of 2 to 6 days, individual dying animals appeared symptoms of decreased spontaneous activity, erect hair, arched back, and spasm.

(3) Effect on animal body weight

After intravenous injection of the S-configuration of compound 01 in mice, the body weight of the animals increased slowly. On the $3^{rd}$ day of administration and the $2^{nd}$ day of recovery, the body weight of the female animals decreased compared with that before administration. After $5^{th}$ day of recovery, the body weight of the female animals recovered to increase. After 5 days of recovery in the 40 mg/kg group, the body weight of the surviving male animals decreased.

(4) Effect on animal food intake

There was no significant difference between the average food intake of animals on each administration group and the average food intake of normal mice (the background data in our laboratory).

(5) Pathological examination results

Tissues from dead animals would be harvested for examination. After the observation of the recovery period, the gross necropsy of the surviving animals showed on obvious gross pathological changes.

**Experimental conclusion**

Under the experimental conditions, the maximum tolerated dose of the S-configuration of compound 01 in ICR mice was 20 mg/kg by intravenous injection in a single cycle.

**2. R-configuration of compound 01**

Experiment procedure

18 ICR mice, 9 females/9 males, were selected for the experiment. According to gender and body weight, the mice were randomly divided into a 10mg/kg group, a 20mg/kg group, and a 40mg/kg group of the R-configuration of compound 01, with 3 females/ 3 males in each group. By intravenous injection, the 10 mg/kg group of the R-configuration of compound 01 was administered with 1 mg/ml of the administration concentration, 10 ml/kg of the administration volume, and 10 mg/kg of the dose. The 20 mg/kg group of the R-configuration of compound 01 was administered with 2 mg/ml of the administration concentration, 10 ml/kg of the administration volume, and 20 mg/kg of the dose. The 40mg/kg group of the R-configuration of compound 01 was administered with 4 mg/ml of the administration concentration,

10 ml/kg of the administration volume, and 40 mg/kg of the dose. The mice was administered once a day for 5 consecutive days, and recovered for 1 week after the last administration. After the animals were continuously administered for 5 consecutive days, there no animal death in each dose group. To find the maximum tolerated dose, a second cycle of dosing was required. The doses were adjusted and administered for 5 consecutive days. The animals were observed for 14 consecutive days after the last administration. The doses were adjusted as shown in the following table.

| Original dose group | Dose adjustment for the 2nd cycle group | Group identification |
|---|---|---|
| 10 mg/kg group of R-configuration of compound 01 | 80 mg/kg | 80 mg/kg group of R-configuration of compound 01 |
| 20 mg/kg group of R-configuration of compound 01 | 60 mg/kg | 60 mg/kg group of R-configuration of compound 01 |
| 40 mg/kg group of R-configuration of compound 01 | 40 mg/kg | 40 mg/kg group of R-configuration of compound 01 |

The mice were closely observed for 2 hours after administration of the daily drug. The mice were observed twice a day in the morning and afternoon on the non-administration day. During the experiment, body weight and food intake were measured twice a week. The dead animals and all the survival animals were subjected to gross necropsy on the 14th day after the last administration. The diseased organic were grossly necropsied for histopathological examination.

**Experimental results**

(1) Animals death conditions

During the experiment, 1 of the 6 animals in the 10/80 mg/kg group of the R-configuration of compound 01 (indicating that the 1st cycle was administered with 10mg/kg, and continued to be administered with 80 mg/kg in the 2nd cycle) died. 3 of the 6 animals in the 20/60 mg/ml group of the R-configuration of compound 01 died. 1 of the 6 animals in 40/40 mg/kg group of the R-configuration of compound 01 died. The death time was mainly during the recovery period of 3 to 7 days.

(2) Clinical symptoms of animals after administration

In the first cycle of administration, the general state of the animals with the 10/80 mg/kg group of the R-configuration of compound 01 was in good condition. The animals with the 20/60 mg/kg group of the R-configuration of compound 01 appeared unsteady gait after administration. The animals with 40/40 mg/kg appeared intermittent gait unsteady, accelerated breathing, and increased spontaneous activity immediately after administration to 5 minutes after administration.

In the second cycle of administration, the animals with 10/80 mg/kg of the R-configuration of compound 01 intermittently appeared abnormal gait, decreased muscle tension, decreased spontaneous activity, irregular breathing, unsteady gait, accelerated breathing, erect hair, spasm, deepened breathing, prone, and arched back immediately after administration to 1 hour after administration. The animals with 20/60 mg/kg of the R-configuration of compound 01 appeared intermittent deepened breathing, decreased spontaneous activity, unsteady gait, irregular breathing, increased spontaneous activity, accelerated breathing, unsteady gait, and prone immediately after administration to 30 minutes after administration. The animals with 40/40 mg/kg of the R-configuration of compound 01 appeared intermittent unsteady gait, deepened breathing, accelerated breathing, increased spontaneous activity, and abnormal gait immediately after administration to 10-30 minutes after administration. In the recovery period of 3 to 4 days, the animals in the 10/80 mg/kg group and the 20/60 mg/kg group of the R-configuration of compound 01 appeared salivation and wetness around the mouth.

(3) Effect on animal body weight

After intravenous injection of the R-configuration of compound 01 in mice, the body weight of the animals increased slowly.

For the male animals, it was found that on the 3rd day of administration in the 1st cycle: Compared with before

administration, the body weight of the 10 mg/kg group and the 40 mg/kg group decreased, and the 20 mg/kg group increased slowly.

For the female animals, compared with before administration, the body weight on the 3rd day of administration and 1st day of recovery in the first cycle decreased, and the body weight increased after 5 days of recovery.

For all animals in the administration group (regardless of male and female), compared with before administration, the body weight of the 20/60 mg/kg group and the 40/40 mg/kg group decreased after 5 days of recovery in the second cycle.

(4) Effect on animal food intake

There was no significant difference between the average food intake of animals on each administration group and the average food intake of normal mice (the background data in our laboratory).

(5) Pathological examination results

Tissues from dead animals would be harvested for examination. After the observation of the recovery period, the gross necropsy of the surviving animals showed on obvious gross pathological changes.

**Experimental conclusion**

Under the experimental conditions, in the first cycle, the maximum tolerated dose of the R-configuration of compound 01 in ICR mice was > 40 mg/kg by intravenous injection. In the second cycle, the maximum tolerated dose of the R-configuration of compound 01 in ICR mice was < 40 mg/kg by intravenous injection. To sum up, the maximum tolerated dose of the R-configuration of compound 01 was 20 to 40 mg/kg, which was similar to or slightly higher than that of the S-configuration of compound 01.

**3. Comparison of MTD of R/S configuration of compound 01 with TH-2565/2566**

The MTD experimental results in mice of TH-2565/2566 compounds disclosed in the previous literatures (WO2016210175A1, CN108024974A) were 2 mg/kg respectively. The list of MTD results for TH-2565/2566 was as follow.

| Compound code | Compound structure | MTD result (mg/kg) |
|---|---|---|
| TH2565 | 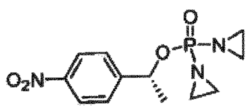 | 2 |
| TH2566 | 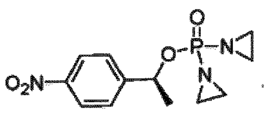 | 2 |
| S-configuration of compound 01 | 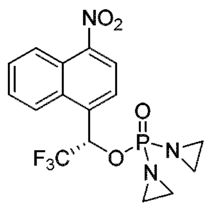 | 20 |
| R-configuration of compound 01 | 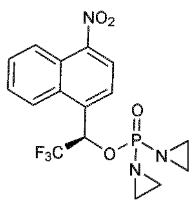 | 20-40 |

The results of the maximum tolerated dose assay show that the anticancer compound 01 with the new structure

of naphthalene ring provided by the present disclosure has stronger safety for animals. That is, it is possible to use a higher dose in clinical treatment to achieve a better therapeutic effect.

Further, as a comparison, the compounds TH-2565/2566 disclosed in the previous literature (WO2016210175A1, CN108024974A) are the most excellent compounds comprehensively among the anticancer compounds of benzene ring structure. By comparison, it may be speculated that the new structural compounds (the modification ideas comprise improving the core structure of the single benzene ring into a naphthalene ring, benzopyrazine, benzopyridine core structure, and different relative positions of the nitro and aziridine side chain structures) of the present disclosure obtained by the new structural modification has a higher MTD. Therefore, the safety and clinical therapeutic dose of the new compounds may be improved.

**Summary of preparation embodiments and effect examples**

Compared with the anticancer compounds of single benzene ring structure of the following structures disclosed in the previous literatures (WO2016210175A1, CN108024974A):

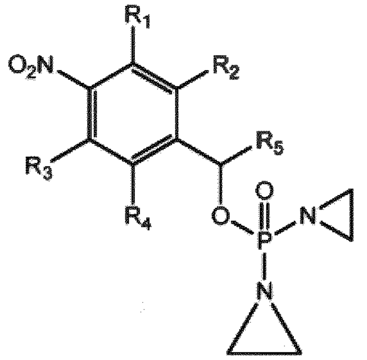
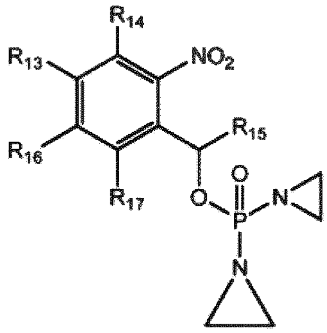

The present disclosure through: A, the single benzene ring core structure was improved into the naphthalene ring, benzopyrazine, benzopyridine, and other core structures. B, by changing the relative positions of the nitro and aziridine side chain structures, a series of new compounds were obtained and indeed synthesized. As well, the new compounds have been experimented to verify the following:

1. The new structural compounds still have good *in vitro* cell anticancer activity. Compared with the single benzene ring structure, the new structural compounds have stronger or roughly equivalent anticancer activity. For example, the anticancer activity of compound 01 is significantly stronger than that of single benzene ring structure compounds, and stronger than that of TH-302.
2. The new structural compounds are still activated by hypoxia. In other words, the new anticancer compounds provided by the present disclosure have the same hypoxia activation mechanism as the anticancer compounds with the single benzene ring structure. It is more cytotoxic to cancer cells under hypoxia conditions than under normoxic conditions. For example, compound 01 and TH-302 have comparable hypoxia reduction activity.
3. The new structural compounds are not P-gp substrates. In this way, the new structural compounds may effectively enter the central nervous system, such as the brain. The new structural compounds may accumulate sufficient concentration to exert the drug effect on the central nervous system. Therefore, the new structural compounds are expected to be drugs for treating primary brain cancers or tumors, metastatic cancers or tumors that metastasizes to the brains, or used for treating primary brain cancers or tumors, metastatic cancers or tumors that metastasizes to the brains. This characteristic is significantly better than that of TH-302, which is a P-gp substrate.

The new structural compounds are more sensitive to DNA repair gene mutations in cell lines. Specifically, compound 01 is more sensitive to cell lines with damaged homologous recombination DNA repair enzymes and damaged nucleotide excision repair enzymes. Compound 01 has a stronger inhibitory effect on the proliferation of cell lines with damaged homologous recombination DNA repair enzymes and damaged nucleotide excision repair enzymes.

5. The representative drug of the new structural compounds, compound 01, is relatively stable in human plasma and hepatomicrosome, and has the potential to be developed as drugs.

Further, through *in vivo* animal tests, it is proven that:

6. The new structural compounds are equivalent to the compounds disclosed in the previous literatures with higher safety and clinical therapeutic dose. As well, the therapeutic effect on cancers or tumors may be better.

7. As a comparison, the new structural compounds, compound 01, has obvious advantages in inhibiting tumor

growth compared with the classical chemotherapeutic drug, ifosfamide (also a drug with a mechanism of a DNA alkylating agent).

## Claims

1. A compound of formula (I), or a pharmaceutically acceptable salt, a solvate, an isotopic variant, or an isomer thereof:

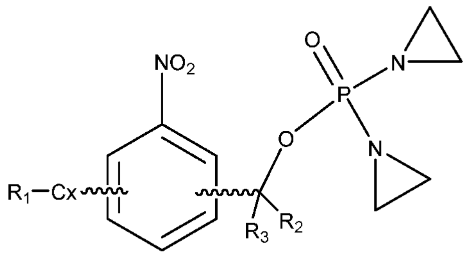

(I)

wherein

Cx is a 5- to 10-membered aryl ring or heteroaryl ring, a heteroaliphatic ring or a cycloalkane, and the Cx shares two carbons with the nitrobenzene ring to form a fused ring structure;
$R_1$ is attached to any skeleton atom of the Cx ring, and the $R_1$ is selected from a hydrogen, a halogen atom, a cyano or isocyano group, an hydroxyl group, an thiol group, an amine, an OTs, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4- to 15-membered heterocycle or a Z-substituted heterocycle, a 5- to 15-membered heteroaryl group or a Z-substituted heteroaryl group, an alkoxyl group with 1-6 carbon atoms or a Z-substituted alkoxyl group with 1-6 carbon atoms, $-CONR^6R^7$, - $SO_2NR^6R^7$, $-SO_2R^6$, $-OCOO-R^6$, $-COOR^6$, $-NR^6COR^7$, $-OCOR^6$, $-NR^6SO_2R^7$, $-NR^6SO_2NR^6R^7$;
$R_2$, $R_3$ are each respectively a hydrogen, a $C_1$-$C_6$ alkyl group or a Z-substituted alkyl group, a $C_2$-$C_6$ alkenyl group or a Z-substituted alkenyl group, a $C_2$-$C_6$ alkynyl group or a Z-substituted alkynyl group, a $C_3$-$C_8$ cycloalkyl group or a Z-substituted cycloalkyl group, a $C_6$-$C_{10}$ aryl group or a Z-substituted aryl group, a 4- to 15-membered heterocycle or a Z-substituted heterocycle, a 5- to 15-membered heteroaryl group or a Z-substituted heteroaryl group, or a 3- to 6-membered ring formed by $R_2$, $R_3$, and the bonded carbon atom at the benzylic position;

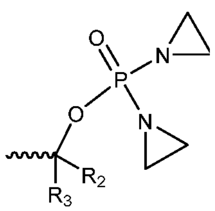

group replaces a hydrogen atom at any position on a carbon atom of the fused ring, and a number of substitutions is 1;
the Z-substituted group is a halogen atom, a cyano or isocyano group, a hydroxyl group, a thiol group, an amine group, a $C_1$-$C_3$ alkyl group or a substituted alkyl group, a $C_1$-$C_3$ alkoxyl group or a substituted alkoxyl group, a $C_2$-$C_3$ alkenyl group or a substituted alkenyl group, $C_2$-$C_3$ alkynyl group or a substituted alkynyl group, a $C_3$-$C_8$ cycloalkyl group or a substituted cycloalkyl group;
$R_6$, $R_7$ are each respectively a hydrogen, a $C_1$-$C_6$ alkyl group or a Z-substituted alkyl group, a $C_2$-$C_6$ alkenyl group or a $C_2$-$C_6$ Z-substituted alkenyl group, a $C_2$-$C_6$ alkynyl group or a Z-substituted alkynyl group, $C_3$-$C_8$ cycloalkyl group or a Z-substituted cycloalkyl group, a $C_6$-$C_{10}$ aryl group or a $C_6$-$C_{10}$ Z-substituted aryl group, a 4- to 15-membered heterocylic group or a Z-substituted 4- to 15-membered heterocyclic group, 5- to 15-membered heteroaryl group or a Z-substituted 5- to 15-membered heteroaryl group, or a 5- to 7-membered heterocyclic group or a Z-substituted 5- to 7-membered heterocyclic group formed by $R_6$, $R_7$, and an atom

bonded thereto.

2. The compound of claim 1, wherein the Cx is

a 5-, 6-, 8-membered aryl ring; or
a 5-, 6-, 7-, 8-membered heteroacryl ring or aliphatic heterocycle containing N, O, S atoms; or
a 5-, 6-, 7-, or 8-membered aliphatic ring.

3. The compound of claim 1, which is a compound of formula (II):

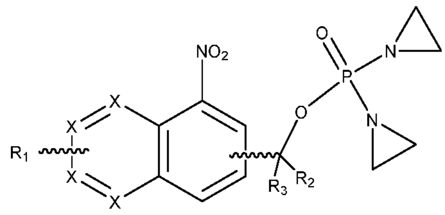

(II)

wherein

$R_1$ substitute a hydrogen atom at any position on carbon atom of a fused ring, and a member of the substituent $R_1$ is 1, 2, 3, 4, 5, or 6;
X is C or N.

4. The compound of claim 3, wherein:
only one or two of the four X atoms are N atoms.

5. The compound of claim 3, wherein,

$R_1$ is the hydrogen, the halogen atom, the $C_1$-$C_6$ alkyl group or the Z-substituted alkyl group, the $C_3$-$C_8$ cycloalkyl group or the Z-substituted cycloalkyl group, the $C_6$-$C_{10}$ aryl group or the Z-substituted aryl group, the 4- to 15-membered heterocycle or the Z-substituted heterocycle, the 5- to 15-membered heteroaryl group or the Z-substituted heteroaryl group; or
$R_2$, $R_3$ are each respectively the hydrogen, the $C_1$-$C_6$ alkyl group or the Z-substituted alkyl group, the $C_3$-$C_8$ cycloalkyl group or the Z-substituted cycloalkyl group, the $C_6$-$C_{10}$ aryl group or the Z-substituted aryl group, the 4- to 15-membered heterocycle or the Z-substituted heterocycle, the 5- to 15-membered heteroaryl group or the Z-substituted heteroaryl group.

6. The compound of claim 5, wherein,

$R_1$ is the hydrogen, the $C_1$-$C_6$ alkyl group or a halogen substituted $C_1$-$C_6$ alkyl group, the $C_3$-$C_8$ cycloalkyl group or a halogen substituted $C_3$-$C_8$ cycloalkyl group, the $C_6$-$C_{10}$ aryl group or a halogen substituted $C_6$-$C_{10}$ aryl group; or
$R_2$, $R_3$ are each respectively the hydrogen, the $C_1$-$C_6$ alkyl group or a halogen substituted $C_1$-$C_6$ alkyl group, the $C_3$-$C_8$ cycloalkyl group or a halogen substituted $C_3$-$C_8$ cycloalkyl group, the $C_6$-$C_{10}$ aryl group or a halogen substituted $C_6$-$C_{10}$ aryl group.

7. The compound of claim 6, wherein

$R_1$ is H, $-CH_3$, $-CF_3$;
$R_2$, $R_3$ are respectively H, D, $-CH_3$, $-CF_3$.

8. The compound of claim 3, wherein,
when X is C; $R_1$, $R_2$ are H; and $R_3$ is H, D, or $-CF_3$.

9. The compound of claim 1, wherein the compound is selected from compounds of the following structures:

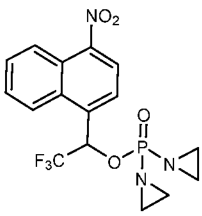,
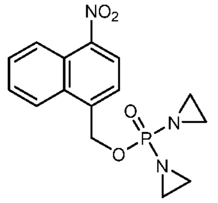,
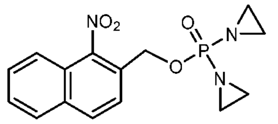,
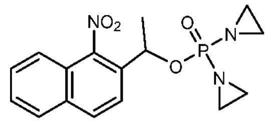,

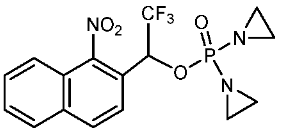,
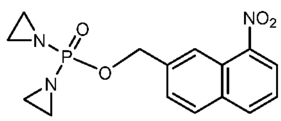,
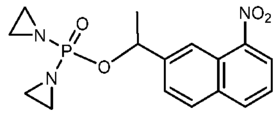,

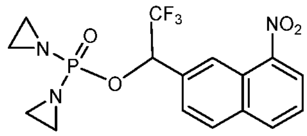,
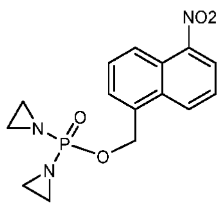,
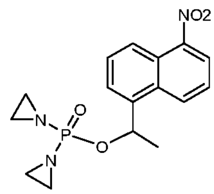,
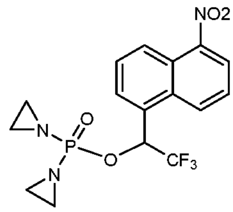,

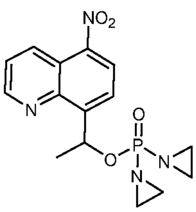,
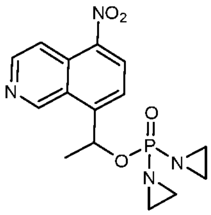,
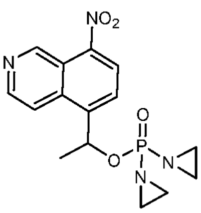,
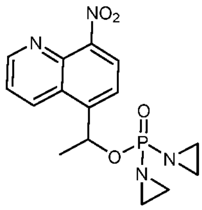,
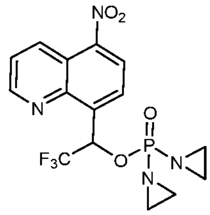,

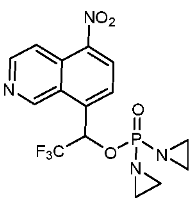,
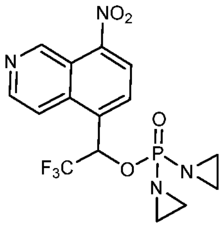,
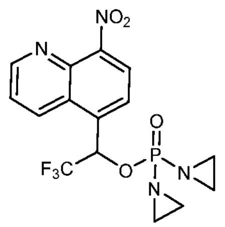,
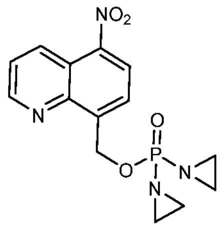,
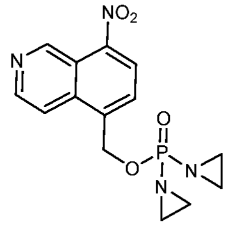,

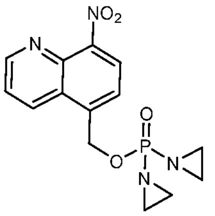,
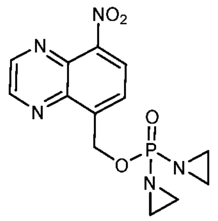,
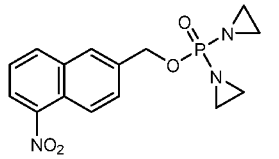,
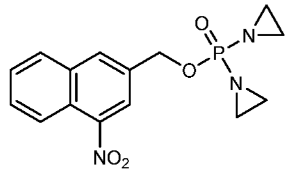,

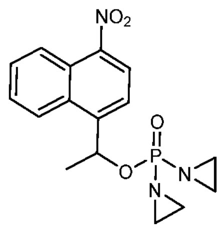

.

10. A drug or a formulation containing the compound, or the pharmaceutically acceptable salt, the solvate, the isotope variant, or the isomer thereof in any one of claims 1-9.

11. A use of the drug or the formulation of claim 10 for treating a tumor, a cancer, or a cell proliferative disease.

12. A use of the compound, or the pharmaceutically acceptable salt, the solvate, the isotope variant, or the isomer thereof described in any one of claims 1 to 9 in a preparation of a drug for treating tumors, cancers, or cell proliferative diseases.

13. The use of the claim 12, wherein the tumor, the cancer comprises:
lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous-cell cancer , basal cell carcinoma, adenocarcinoma, squamous-cell carcinoma, sebaceous carcinoma, papillary carcinoma, papillary adenocarcinoma, cystadenocarcinoma, cystic carcinoma, medullary carcinoma, bronchial carcinoma, bone cell carcinoma, epithelial carcinoma, cholangiocarcinoma, choriocarcinoma, embryonic carcinoma, seminoma, Wilms' carcinoma, glioblastoma, astrocytoma, medulloblastoma, craniopharyngioma, ependymoma, pineal tumor, hemoblastoma, neurogenic tumor of the larynx, meningiomas, neuroblastoma of optic nerve, neuroblastomas, retinoblastomas, neurofibromas, fibroma sarcomatosum, fibroblastomas, fibroma, fibroadenomas, fibrochondromas, fibrocystic tumors, fibrous myxoma, osterfibroma, myxofibrosarcoma, fibropapillary, myxofibrosarcoma, bursal tumor, myxonchondroma, myxonchondrosarcoma, myxonchondrosarcoma, myxedema, myxoblastoma, liposarcoma, lipoma , lipoadenoma, lipoblastoma, lipochondroma, lipofibroma, lipoangioma, myxolipoma, chondrosarcoma, chondroma, chondromyoma, chordoma, chorioadenoma, chorioepithelioma, chorioblastoma, osteosarcoma, osteoblastoma, osteochondrofibroma, osteochondrosarcoma, osteochondroma, osteocystoma, cementoma, osteofibroma, fibrosarcoma, angiosarcoma, hemangioma, angiolipoma, angiochondroma, hemangioblastoma, angiokeratoma, angioglioma, hemangioendothelioma, angiofibroma, angiomyoma, angiolipoma, angiolymphoma, angiolipiomyoma, angiomyolipomas, angiomyoneuroma, angiomyxoma, angioreticuloendothelioma, lymphangiosarcoma, lymphogranuloma, lymphangioma, lymphoma, lymphomyxoma, lymphosarcoma, lymphangial fibrom, lymphocytoma, lymphoepithelioma, lymphoblastoma, endothelioma, endothelioblastoma, synovialoma, synovial sarcoma, mesothelioma, desmoplastic tumor, Ewing's tumor, leiomyoma, leiomyosarcoma, leioblastoma, leiomyofibroma, rhabdomyoma, rhabdomyosarcoma, rhabdomyomatous myxoma, acute lymphoblastic leukemia, acute myeloid leukemia, chronic disease cells, polycythemia, endometrial cancer, glioma, colorectal cancer, thyroid cancer, urothelial cancer, or multiple myeloma.

14. The use of claim 13, wherein the tumor, the cancer is selected from the non-small cell lung cancer, the pancreatic cancer, the breast cancer, or the prostate cancer.

15. The use of claim 13, wherein the cancer, the tumor is primary brain cancers, brain tumors, or metastatic cancers or tumors which metastasize to brains.

16. A use of the compound, or the compound, or the pharmaceutically acceptable salt, the solvate, the isotope variant, or the isomer thereof described in any one of claims 1 to 9 in preparing of a drug for treating tumors, cancers in patients with a damaged DNA repair, wherein the damaged DNA repair is a damaged homologous recombination DNA repair enzyme, a damaged nucleotide excision repair enzyme.

17. A compound drug, comprising the compound, or the compound, or the pharmaceutically acceptable salt, the solvate, the isotope variant, or the isomer thereof described in any one of claims 1 to 9, and

a. a traditional chemotherapy drug;
b. an anti-angiogenic drug;

c. a cell checkpoint inhibitor; or
d. an immunosuppressant.

**18.** A combination therapy for treating a cancer and a tumor, comprising administering to a patient a drug or a formulation containing the compound, or the compound, or the pharmaceutically acceptable salt, the solvate, the isotope variant, or the isomer thereof described in any one of claims 1 to 9; and administering a traditional chemotherapy drug.

**19.** A combination therapy for treating a cancer and a tumor, comprising administering to a patient a drug or a formulation containing the compound, or the compound, or the pharmaceutically acceptable salt, the solvate, the isotope variant, or the isomer thereof described in any one of claims 1 to 9; and to administering an anti-angiogenic drug.

**20.** A combination therapy for treating cancers and tumors, comprising administering to a patient a drug or a formulation containing the compound, or the the compound, or the pharmaceutically acceptable salt, the solvate, the isotope variant, or the isomer thereof described in any one of claims 1 to 9; and administering a cell checkpoint inhibitor.

**21.** A combination therapy for treating cancers and tumors, comprising administering to a patient a drug or a formulation containing the compound, or the compound, or the pharmaceutically acceptable salt, the solvate, the isotope variant, or the isomer thereof described in any one of claims 1 to 9; and administering an immunosuppressant.

**22.** A method for preparing the compound of any one of claims 3 to 9, wherein,

the method is carried out by adopting a first scheme, the method comprising the following steps:
providing the compound of formula (II) by undergoing a condensation reaction of a compound A to close a ring:

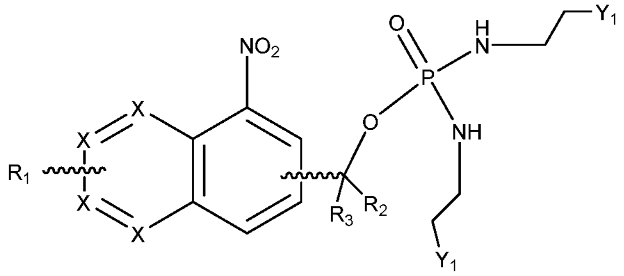

Compound A

or
the method is carried out by adopting a second scheme, the method comprising the following steps:
providing the compound of formula (II) by reacting a compound B with $R_1H$

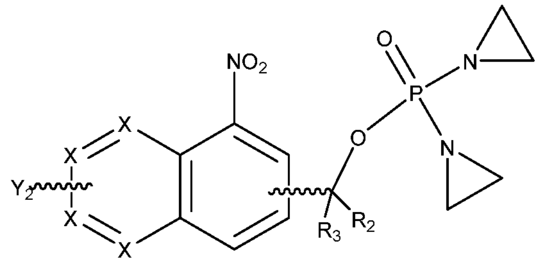

Compound B

wherein $Y_1$ and $Y_2$ are both leaving groups, and preferably, $Y_1$ and $Y_2$ are each respectively Cl, Br, I, -OTs, $-ONO_2$, -OMs, $-OT_f$, or $-OSO_2Cl$; more preferably, $Y_1$ is Br in the compound A, and $Y_2$ is F in the compound B.

**23.** The method of claim 22, wherein in the first scheme, DIPEA or TEA is used as an anti-acid agent, and silver oxide or sliver nitrate is used as catalyst; or
in the second scheme, an alkali is added in a reaction process.

**24.** A use of a compound of following formula in preparing of a drug for treating primary brain cancers, brain tumors, or metastatic cancers or tumors metastasizing to brains:

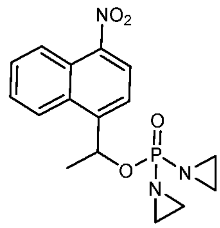

.

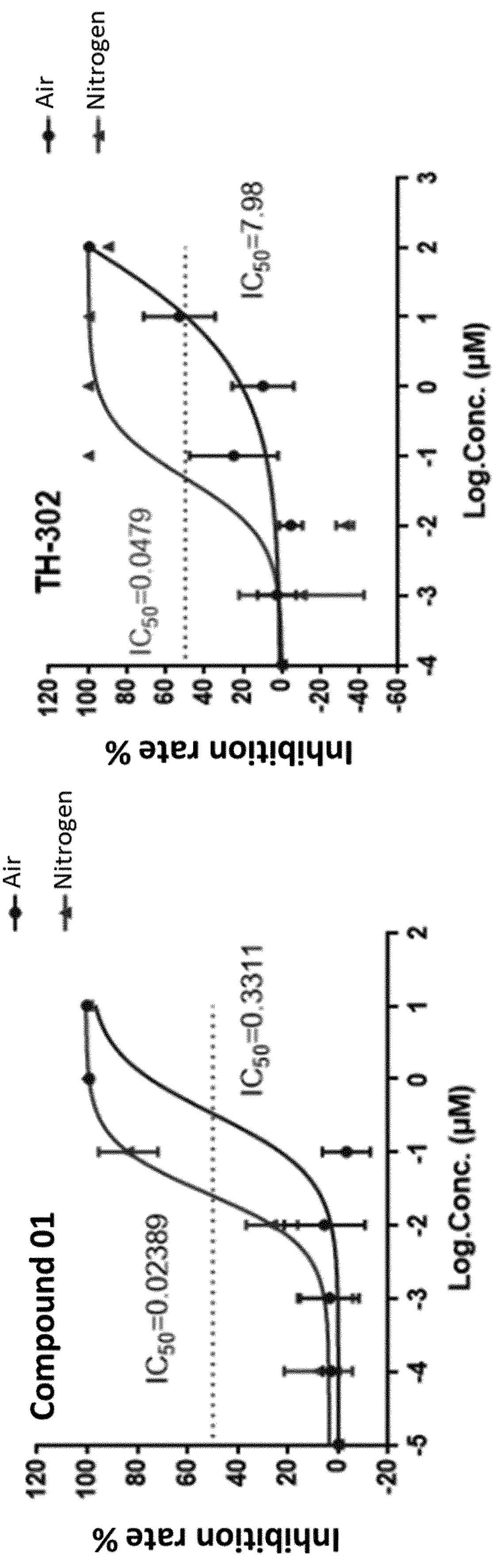
TH-302
Air
Nitrogen
$IC_{50}$=0.0479
$IC_{50}$=7.98
Inhibition rate %
120
100
80
60
40
20
0
-20
-40
-60
-4
-3
-2
-1
0
1
2
3
Log.Conc. (μM)
Compound 01
Air
Nitrogen
$IC_{50}$=0.02389
$IC_{50}$=0.3311
Inhibition rate %
120
100
80
60
40
20
0
-20
-5
-4
-3
-2
-1
0
1
2
Log.Conc. (μM)

FIG. 1

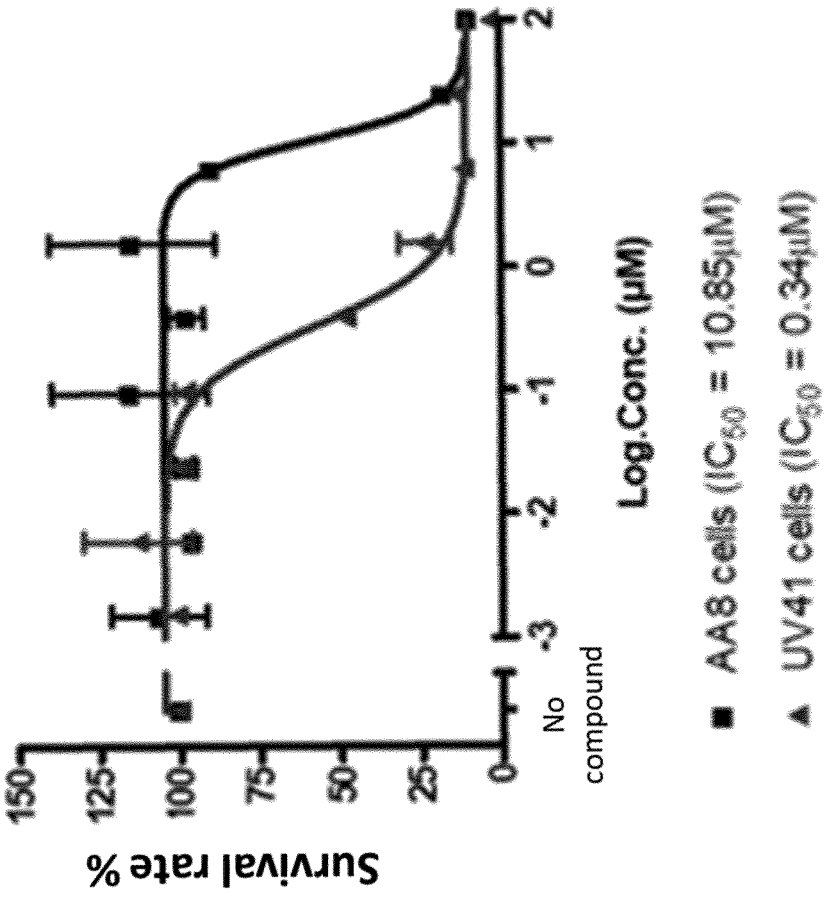
Survival rate %
150
125
100
75
50
25
0
No
compound
-3
-2
-1
0
1
2
Log.Conc. (µM)
AA8 cells (IC50 = 10.85µM)
UV41 cells (IC50 = 0.34µM)

FIG. 2

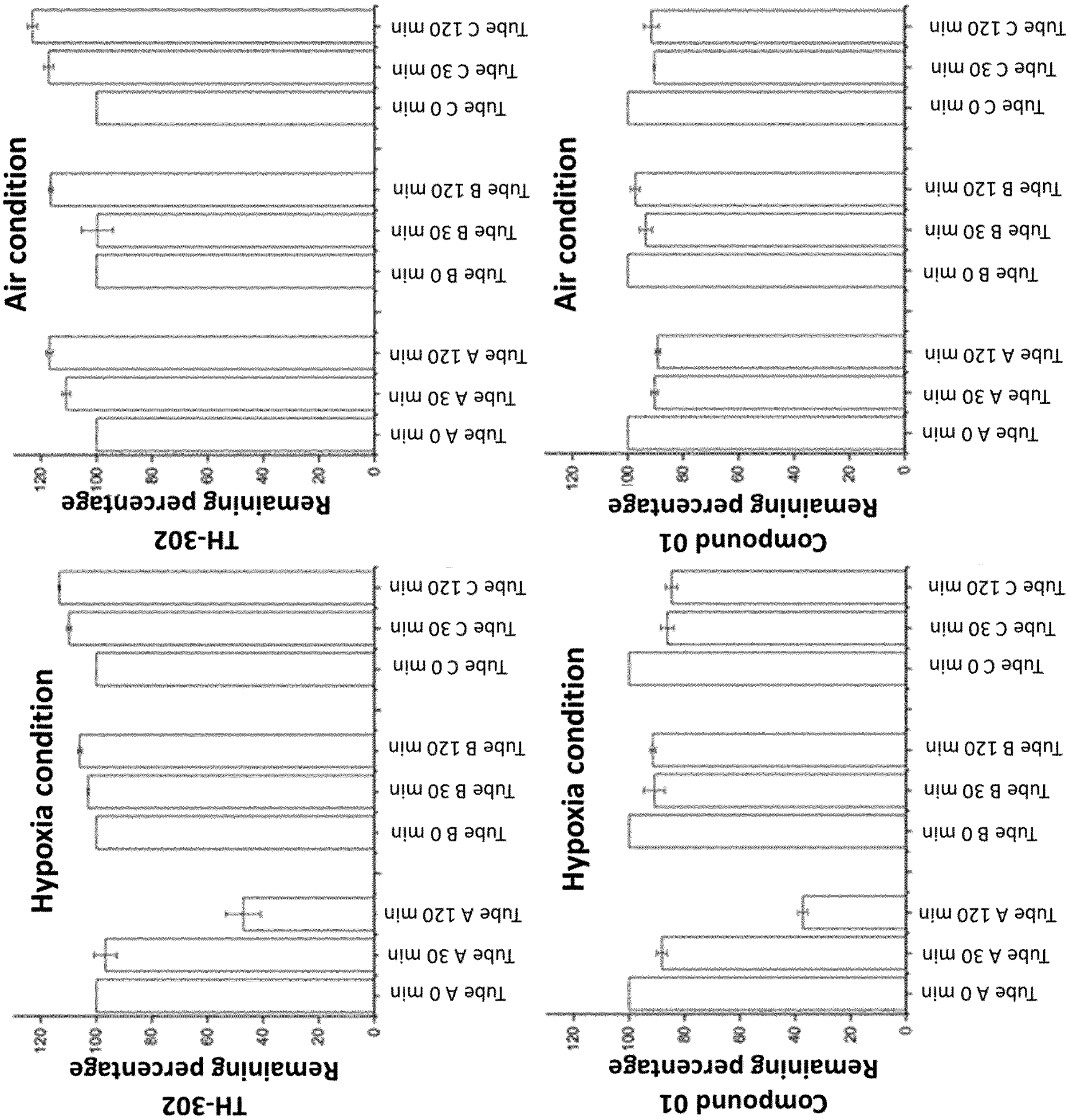
TH-302
Hypoxia condition
Air condition
Compound 01
Remaining percentage
0
20
40
60
80
100
120
Tube A 0 min
Tube A 30 min
Tube A 120 min
Tube B 0 min
Tube B 30 min
Tube B 120 min
Tube C 0 min
Tube C 30 min
Tube C 120 min

FIG. 3

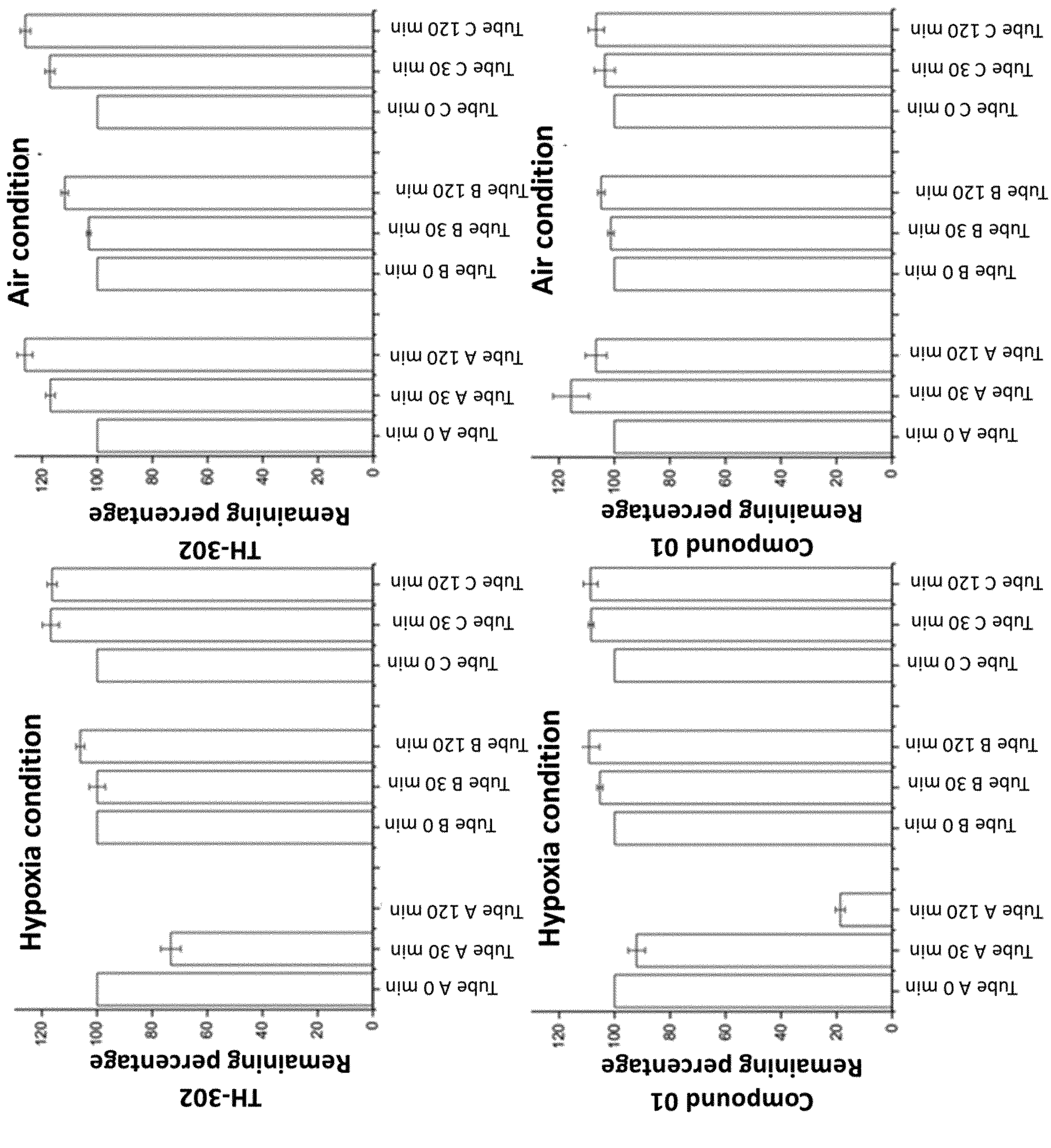
Hypoxia condition
Air condition
TH-302
Compound 01
Remaining percentage
0
20
40
60
80
100
120
Tube A 0 min
Tube A 30 min
Tube A 120 min
Tube B 0 min
Tube B 30 min
Tube B 120 min
Tube C 0 min
Tube C 30 min
Tube C 120 min

FIG. 4

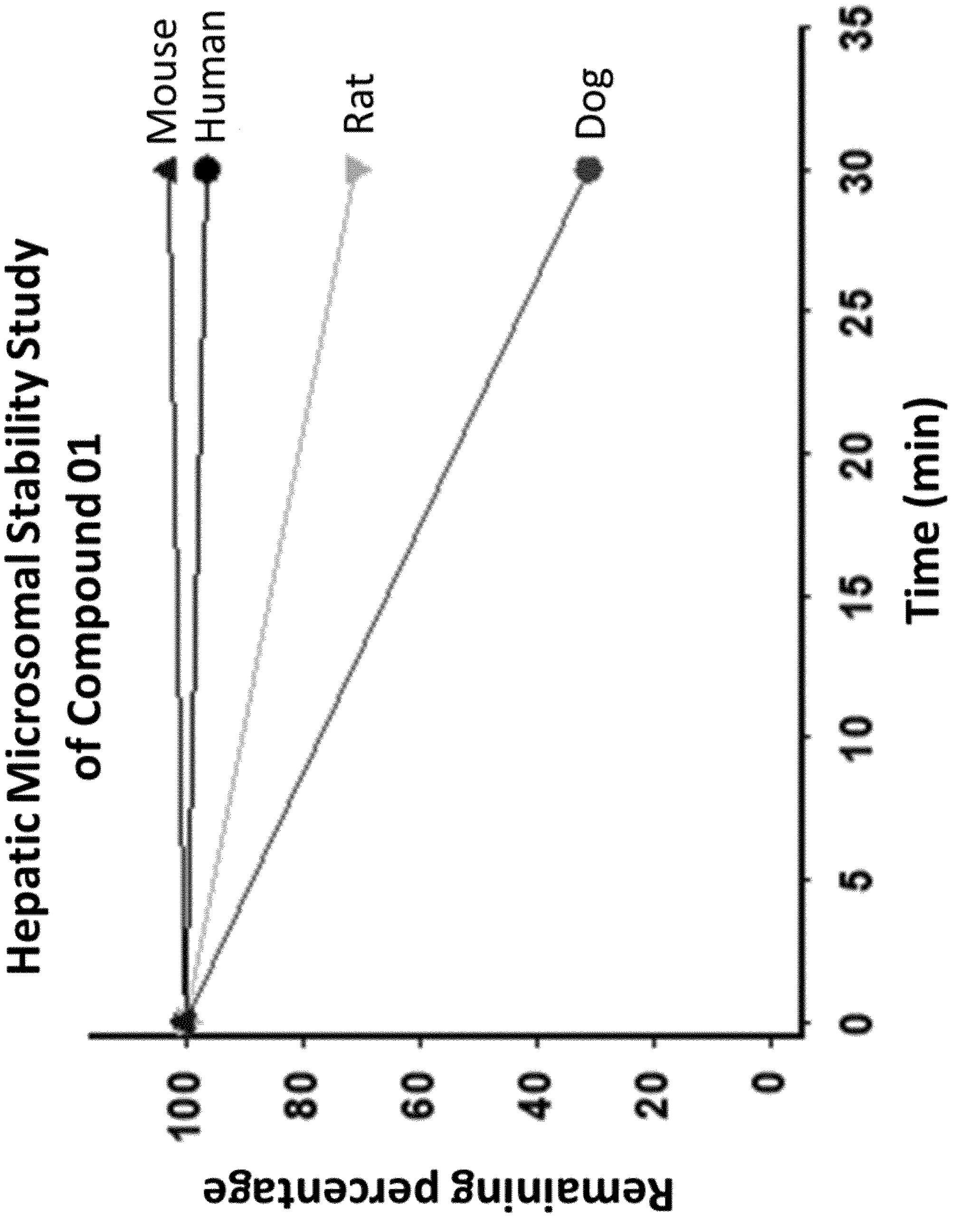

Hepatic Microsomal Stability Study
of Compound 01
Mouse
Human
Rat
Dog
Remaining percentage
100
80
60
40
20
0
0
5
10
15
20
25
30
35
Time (min)


FIG. 5

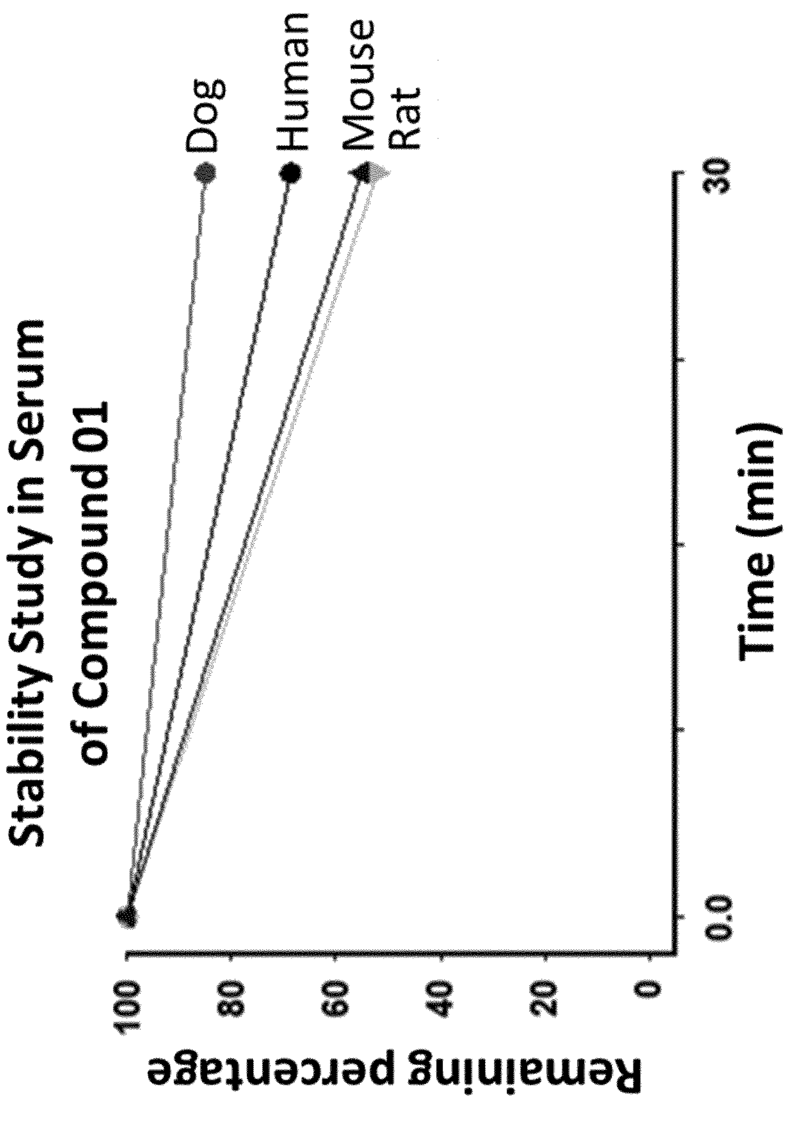
Stability Study in Serum
of Compound 01
Dog
Human
Mouse
Rat
Remaining percentage
100
80
60
40
20
0
0.0
30
Time (min)

FIG. 6

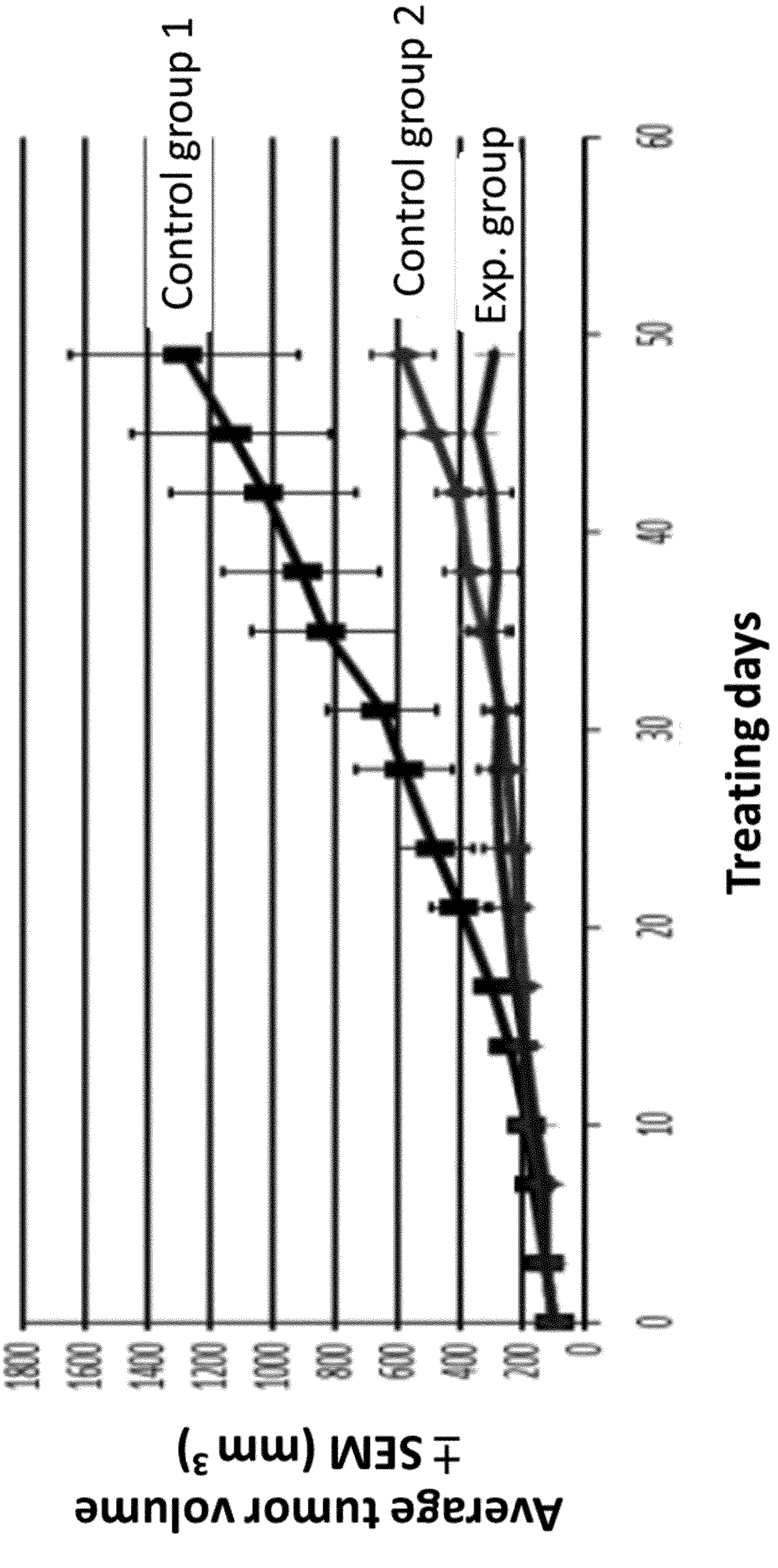
Control group 1
Control group 2
Exp. group
Treating days
Average tumor volume ± SEM (mm 3)
0
200
400
600
800
1000
1200
1400
1600
1800
0
10
20
30
40
50
60

FIG. 7

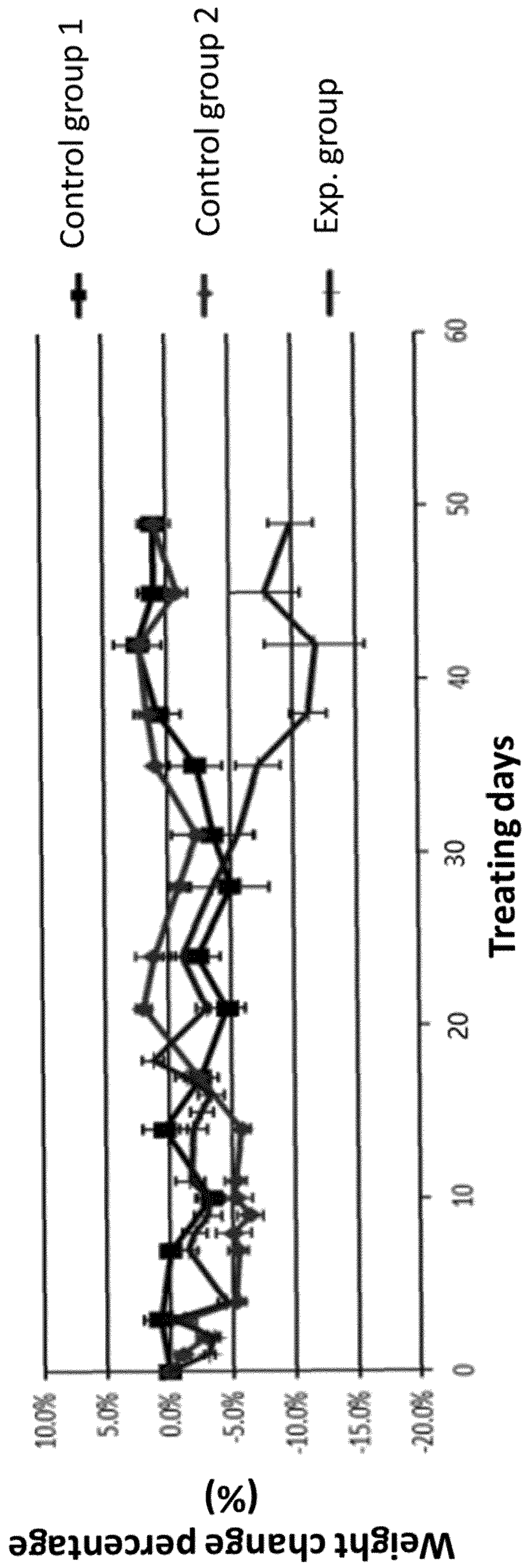
Control group 1
Control group 2
Exp. group
Weight change percentage (%)
10.0%
5.0%
0.0%
-5.0%
-10.0%
-15.0%
-20.0%
0
10
20
30
40
50
60
Treating days

FIG. 8

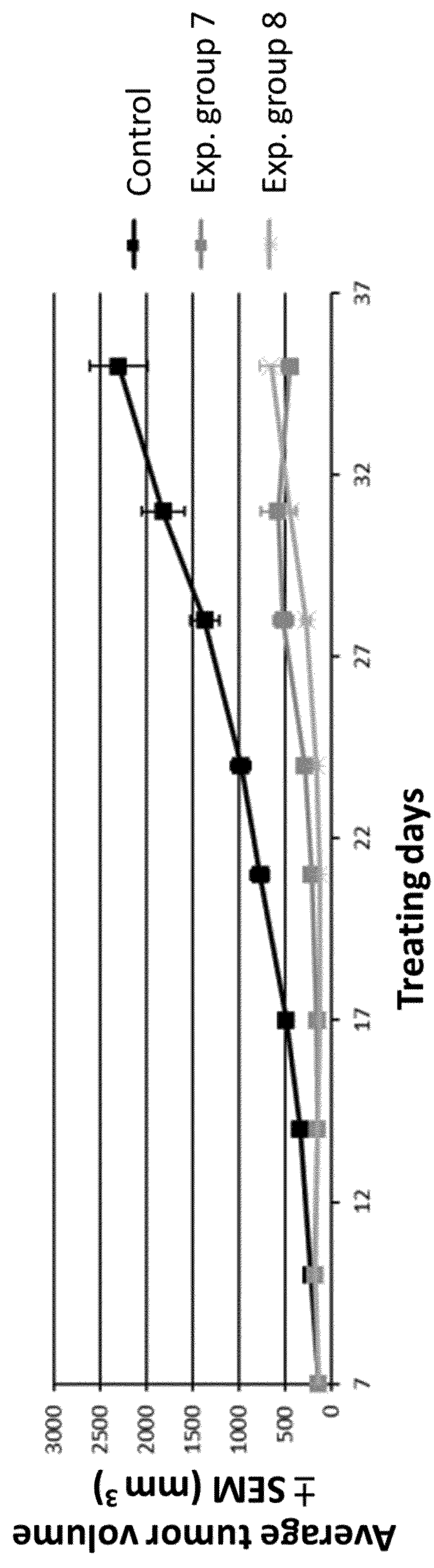

Control
Exp. group 7
Exp. group 8
Average tumor volume ± SEM (mm 3)
3000
2500
2000
1500
1000
500
0
7
12
17
22
27
32
37
Treating days


FIG. 9

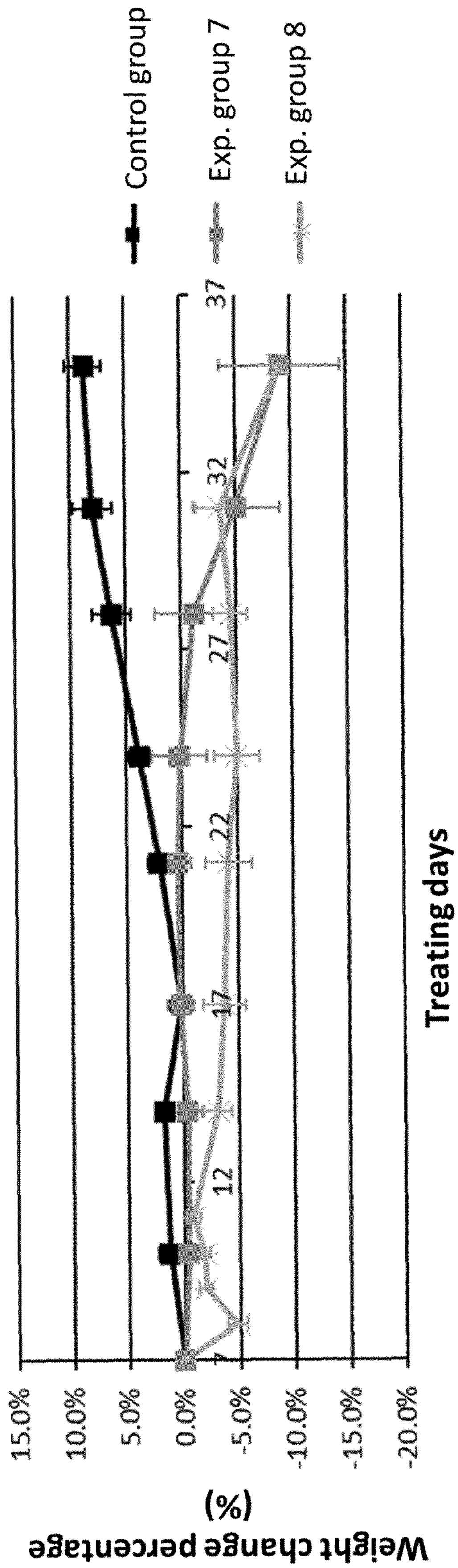
Control group
Exp. group 7
Exp. group 8
15.0%
10.0%
5.0%
0.0%
-5.0%
-10.0%
-15.0%
-20.0%
Weight change percentage (%)
7
12
17
22
27
32
37
Treating days

FIG. 10

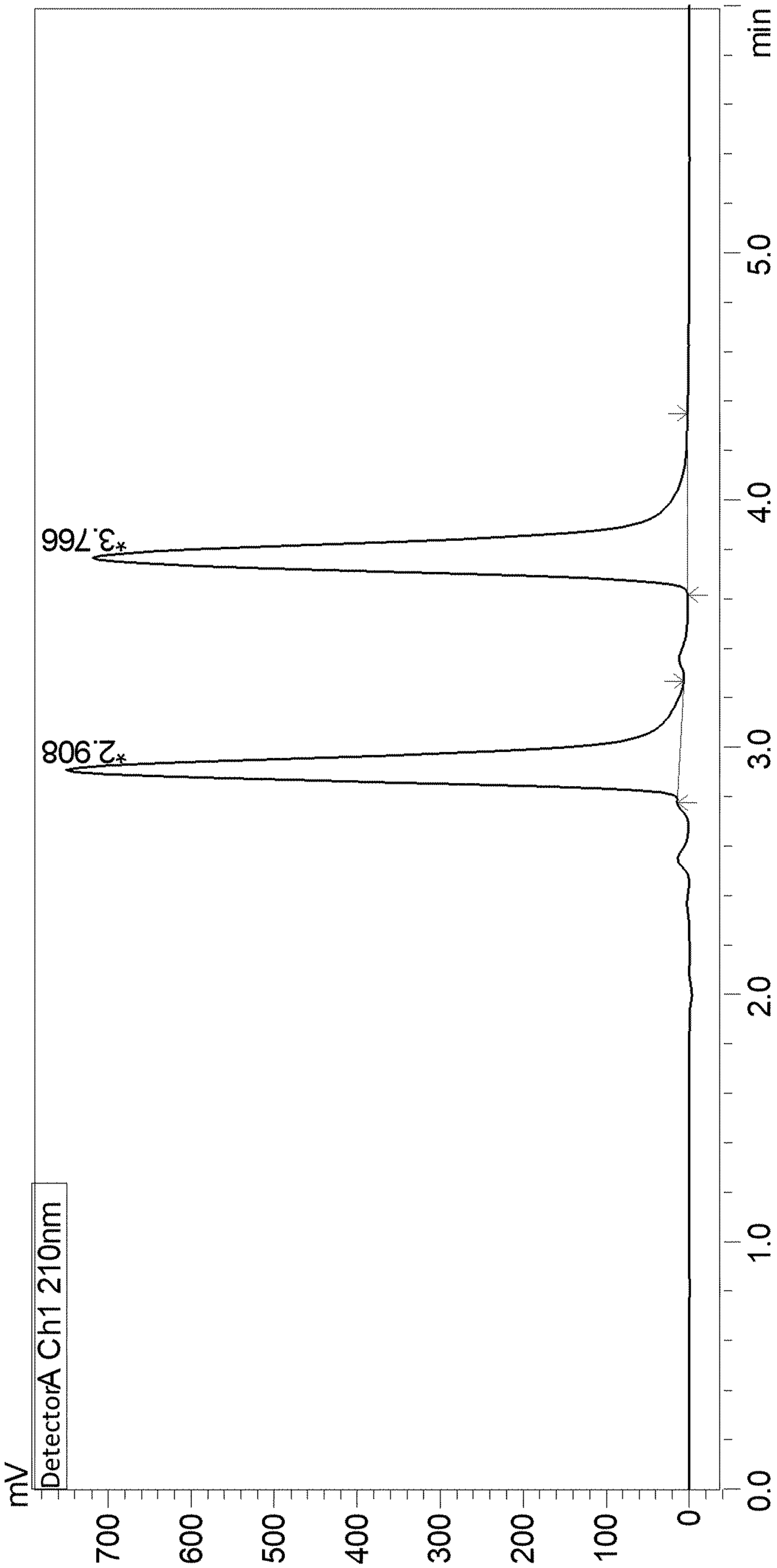
mV
Detector A Ch1 210nm
*2.908
*3.766
700
600
500
400
300
200
100
0
0.0
1.0
2.0
3.0
4.0
5.0
min

FIG. 11

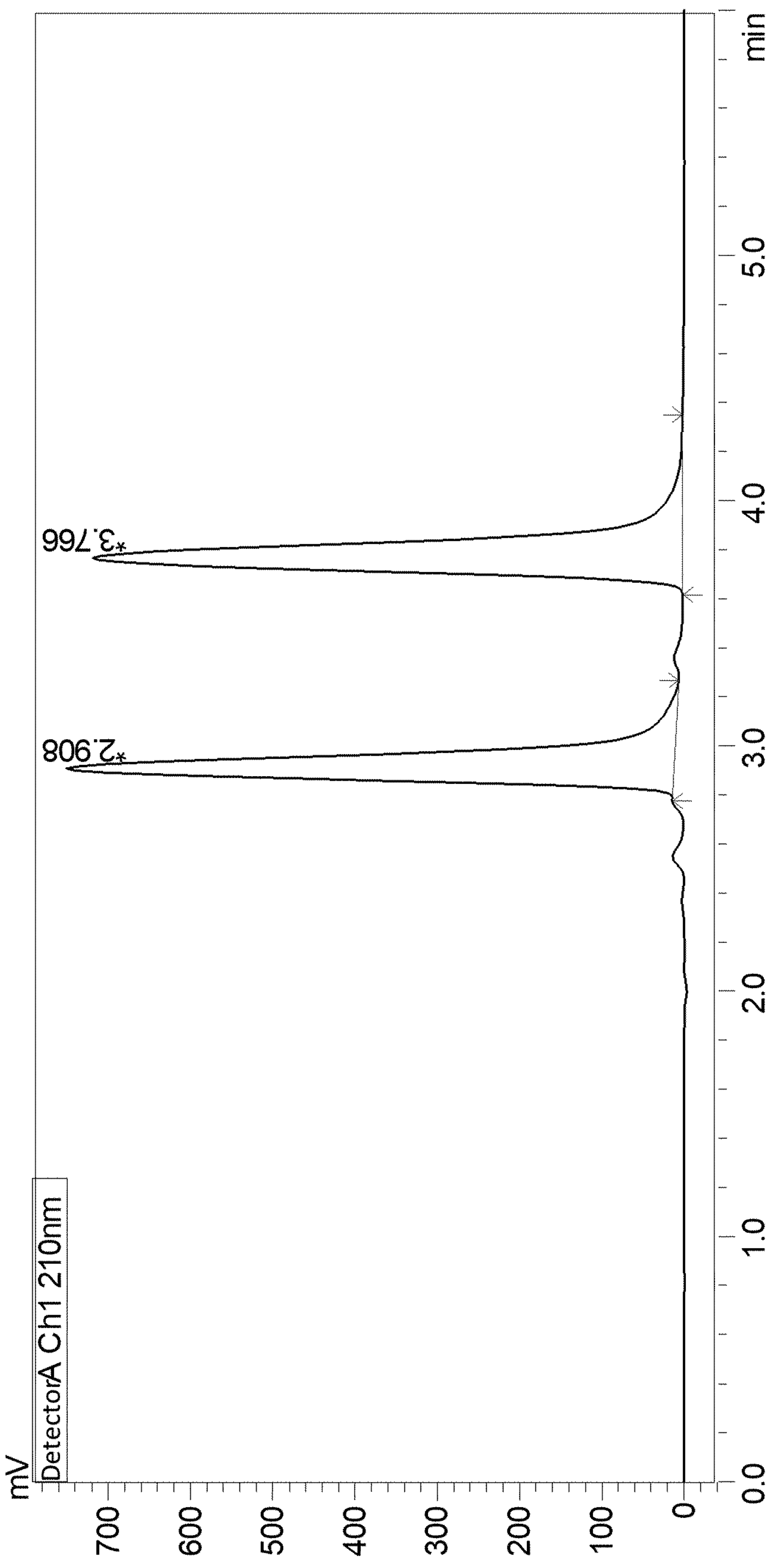
mV
700
600
500
400
300
200
100
0
Detector A Ch1 210nm
*2.908
*3.766
0.0
1.0
2.0
3.0
4.0
5.0
min

FIG. 12

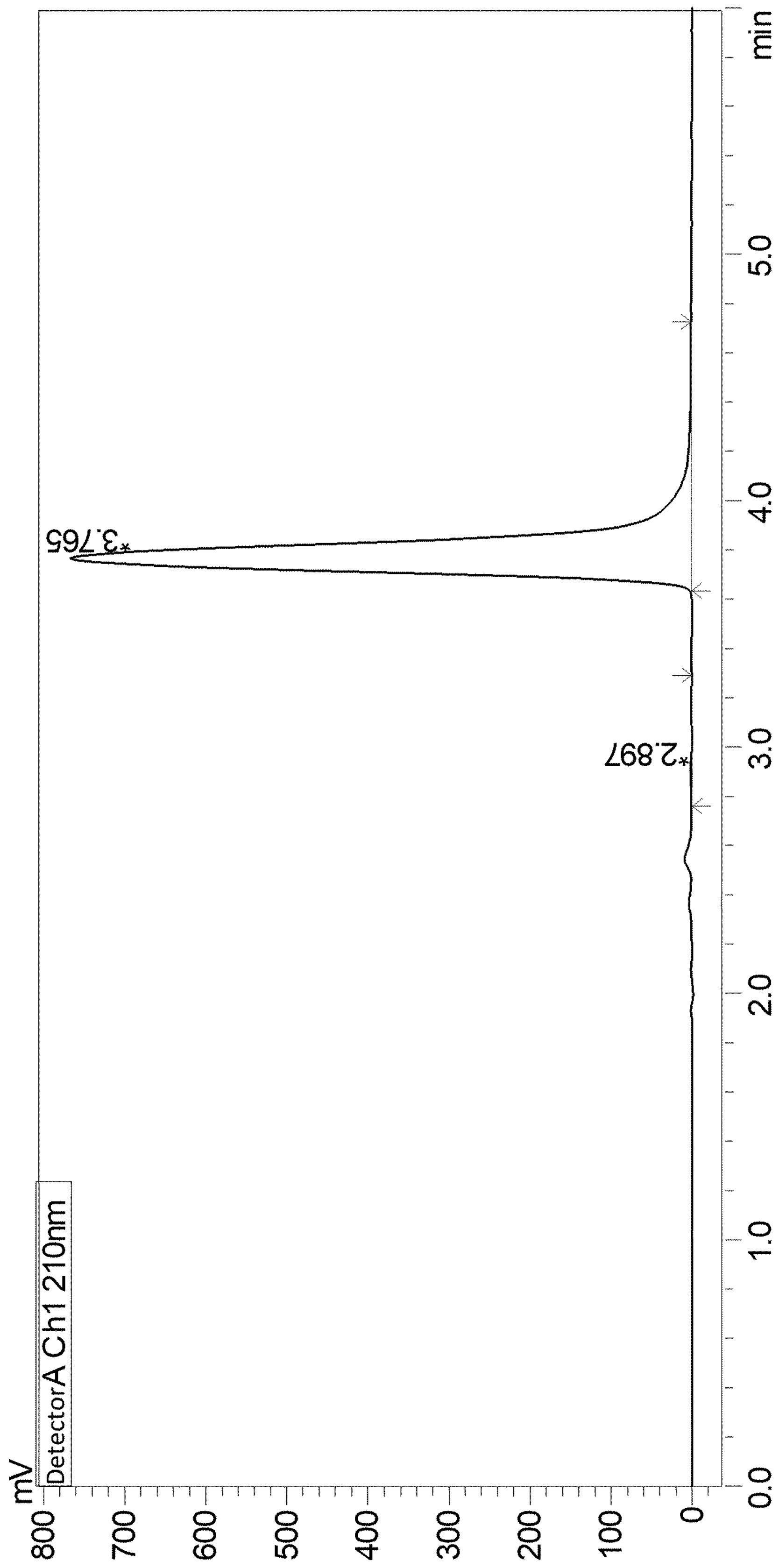
mV
800
700
600
500
400
300
200
100
0
Detector A Ch1 210nm
*3.765
*2.897
0.0
1.0
2.0
3.0
4.0
5.0
min

FIG. 13

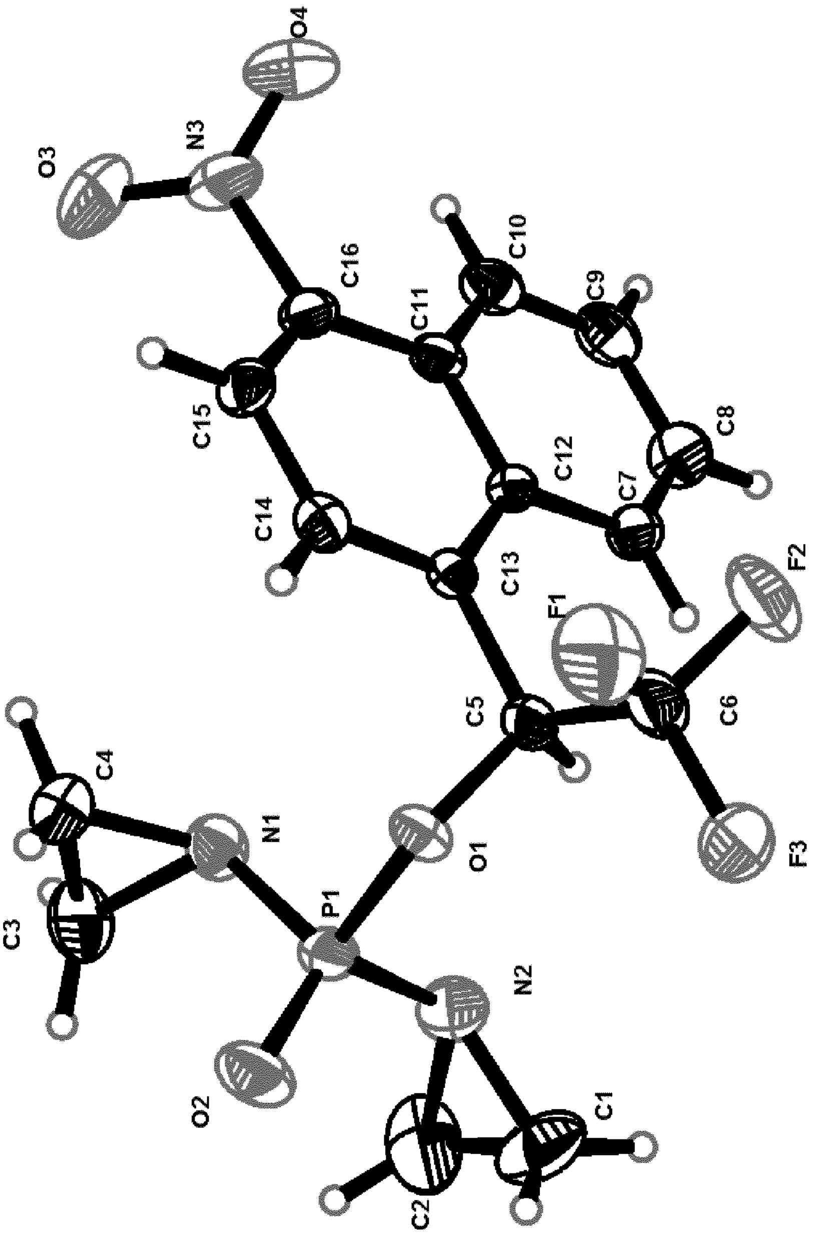

FIG. 14

**INTERNATIONAL SEARCH REPORT**

International application No.
**PCT/CN2020/114519**

**A. CLASSIFICATION OF SUBJECT MATTER**

C07F 9/564(2006.01)i; A61K 31/06(2006.01)i; A61K 31/664(2006.01)i; A61P 35/00(2006.01)i

According to International Patent Classification (IPC) or to both national classification and IPC

**B. FIELDS SEARCHED**

Minimum documentation searched (classification system followed by classification symbols)

C07F9; A61K31; A61P35

Documentation searched other than minimum documentation to the extent that such documents are included in the fields searched

Electronic data base consulted during the international search (name of data base and, where practicable, search terms used)

CNKI, CNABS, DWPI, SIPOABS, STN(CAPLUS, REGISTRY); 艾欣达伟医药, 李安蓉, 段建新, 孟繁英, 蔡晓宏, 硝基, 苯基, 膦, 磷, 膦酰胺, 癌, 瘤, nitro, phenyl, phosphoramide, phosphoric, cancer, tumour, tumor, search according to the structure of compound in claim 1

**C. DOCUMENTS CONSIDERED TO BE RELEVANT**

| Category* | Citation of document, with indication, where appropriate, of the relevant passages | Relevant to claim No. |
|---|---|---|
| X | CN 108024974 A (MOLECULAR TEMPLATES, INC.) 11 May 2018 (2018-05-11) entire document, in particular claims 1-22, description, paragraph 93, compound 2824, paragraphs 10-11, 33, 35, 48, 124 | 1-10, 12-17, 22-24 |
| A | CN 107530556 A (THRESHOLD PHARMACEUTICALS, INC.) 02 January 2018 (2018-01-02) entire document, in particular claims 1-24 | 1-10, 12-17, 22-24 |
| A | CN 107118231 A (PEKING UNIVERSITY) 01 September 2017 (2017-09-01) entire document, in particular claims 1-7 | 1-10, 12-17, 22-24 |
| A | WO 2019062919 A1 (OBI PHARMA INC et al.) 04 April 2019 (2019-04-04) entire document, in particular claim 1 | 1-10, 12-17, 22-24 |
| A | WO 0104130 A1 (PURDUE RESEARCH FOUNDATION et al.) 18 January 2001 (2001-01-18) entire document, in particular claims 1-33 | 1-10, 12-17, 22-24 |
| A | CN 108290911 A (THRESHOLD PHARMACEUTICALS, INC.) 17 July 2018 (2018-07-17) entire document, in particular claims 1-14 | 1-10, 12-17, 22-24 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 November 2020** | **17 November 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/114519**

| Patent document cited in search report | | | Publication date (day/month/year) | Patent family member(s) | | | Publication date (day/month/year) |
|---|---|---|---|---|---|---|---|
| CN | 108024974 | A | 11 May 2018 | JP | 2018527301 | A | 20 September 2018 |
| | | | | US | 2018169064 | A1 | 21 June 2018 |
| | | | | EP | 3313385 | A1 | 02 May 2018 |
| | | | | US | 10668047 | B2 | 02 June 2020 |
| | | | | CA | 2990665 | A1 | 29 December 2016 |
| | | | | EP | 3313385 | A4 | 23 January 2019 |
| | | | | TW | 201713646 | A | 16 April 2017 |
| | | | | US | 2020085786 | A1 | 19 March 2020 |
| | | | | WO | 2016210175 | A1 | 29 December 2016 |
| | | | | AU | 2016282785 | A1 | 15 February 2018 |
| CN | 107530556 | A | 02 January 2018 | SG | 11201707293V | A | 30 October 2017 |
| | | | | JP | 6704421 | B2 | 03 June 2020 |
| | | | | EP | 3277380 | A4 | 01 August 2018 |
| | | | | KR | 20190072688 | A | 25 June 2019 |
| | | | | KR | 102034618 | B1 | 21 October 2019 |
| | | | | AU | 2016229136 | B2 | 09 August 2018 |
| | | | | SG | 10201913462 X | A | 30 March 2020 |
| | | | | WO | 2016145092 | A1 | 15 September 2016 |
| | | | | CN | 107530556 | B | 10 April 2020 |
| | | | | KR | 20170128280 | A | 22 November 2017 |
| | | | | US | 10364261 | B2 | 30 July 2019 |
| | | | | IL | 254377 | D0 | 30 November 2017 |
| | | | | AU | 2016229136 | A1 | 05 October 2017 |
| | | | | KR | 20190072689 | A | 25 June 2019 |
| | | | | BR | 112017019287 | A2 | 02 May 2018 |
| | | | | JP | 2018513876 | A | 31 May 2018 |
| | | | | US | 2019225633 | A1 | 25 July 2019 |
| | | | | US | 2018044360 | A1 | 15 February 2018 |
| | | | | TW | 201706262 | A | 16 February 2017 |
| | | | | CA | 2979251 | A1 | 15 September 2016 |
| | | | | EP | 3277380 | A1 | 07 February 2018 |
| | | | | TW | I674258 | B | 11 October 2019 |
| CN | 107118231 | A | 01 September 2017 | CN | 107118231 | B | 07 June 2019 |
| WO | 2019062919 | A1 | 04 April 2019 | TW | 201919644 | A | 01 June 2019 |
| WO | 0104130 | A1 | 18 January 2001 | US | 7304046 | B2 | 04 December 2007 |
| | | | | US | 2003008850 | A1 | 09 January 2003 |
| | | | | US | 6656926 | B2 | 02 December 2003 |
| | | | | US | 2004176332 | A1 | 09 September 2004 |
| | | | | AU | 5935600 | A | 30 January 2001 |
| CN | 108290911 | A | 17 July 2018 | CN | 108290911 | B | 08 May 2020 |
| | | | | TW | 201726695 | A | 01 August 2017 |
| | | | | BR | 112017025778 | A2 | 14 August 2018 |
| | | | | EP | 3390415 | A4 | 22 May 2019 |
| | | | | CA | 2990696 | A1 | 26 May 2017 |
| | | | | KR | 101985778 | B1 | 04 June 2019 |
| | | | | IL | 256569 | D0 | 28 February 2018 |
| | | | | EP | 3390415 | A1 | 24 October 2018 |
| | | | | KR | 20170130615 | A | 28 November 2017 |
| | | | | WO | 2017087428 | A1 | 26 May 2017 |

Form PCT/ISA/210 (patent family annex) (January 2015)

**INTERNATIONAL SEARCH REPORT**
**Information on patent family members**

International application No.
**PCT/CN2020/114519**

| Patent document cited in search report | Publication date (day/month/year) | Patent family member(s) | | | Publication date (day/month/year) |
|---|---|---|---|---|---|
| | | JP | 2018517710 | A | 05 July 2018 |
| | | JP | 6695360 | B2 | 20 May 2020 |
| | | HK | 1250988 | A1 | 18 January 2019 |
| | | AU | 2016357728 | A1 | 30 November 2017 |
| | | EP | 3390415 | B1 | 26 February 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016210175 A1
- CN 108024974 A
- CN 107513057 A
- CN 107383136 B

### Non-patent literature cited in the description


- *Nature review cancer,* 2002, vol. 2, 38-47
- *Chinese Journal of Cancer,* 2014, vol. 33, 80-86
- *CHEMICAL ABSTRACTS,* 918633-87-1
- **MENG et al.** Molecular and Cellular Pharmacology of the Hypoxia-Activated Prodrug TH-302. *MCT,* 2012, (11), 740
- **VANHOEFER U ; CAO S ; HARSTRICK A ; SEEBER S ; RUSTUM YM.** Comparative antitumor efficacy of docetaxel and paclitaxel in nude mice bearing human tumor xenografts that overexpress the multidrug resistance protein (MRP). *Ann Oncol,* 1997, vol. 8, 1221-1228
- **BIGIONI M ; BENZO A ; IRRISSUTO C ; LOPEZ G ; CURATELLA B ; MAGGI CA ; MANZINI S ; CREA A ; CAROLI S ; CUBADDA F.** Antitumour effect of combination treatment with Sabarubicin (MEN 10755) and cis-platin (DDP) in human lung tumour xenograft. *Cancer Chemother Pharmacol,* 2008, vol. 62, 621-629
- **HUBER PE ; BISCHOF M ; JENNE J ; HEILAND S ; PESCHKE P ; SAFFRICH R ; GRONE HJ ; DEBUS J ; LIPSON KE ; ABDOLLAHI A.** Trimodal cancer treatment: beneficial effects of combined antiangiogenesis, radiation, and chemotherapy. *Cancer Res,* 2005, vol. 65, 3643-3655
- **HOUGHTON JA ; CHESHIRE PJ ; HALLMAN JD ; LUTZ L ; LUO X ; LI Y ; HOUGHTON PJ.** Evaluation of irinotecan in combination with 5-fluorouracil or etoposide in xenograft models of colon adenocarcinoma and rhabdomyosarcoma. *Clin Cancer Res,* 1996, vol. 2, 107-118
- **KRAUS-BERTHIER L ; GUILBAUD N ; JAN M ; SAINT-DIZIER D ; ROUILLON MH ; BURBRIDGE MF ; PIERRE A ; ATASSI G.** Experimental antitumour activity of S 16020-2 in a panel of human tumours. *Eur J Cancer,* 1997, vol. 33, 1881-1887
- **MERRIMAN RL ; HERTEL LW ; SCHULTZ RM ; HOUGHTON PJ ; HOUGHTON JA ; RUTHERFORD PG ; TANZER LR ; BODER GB ; GRINDEY GB.** Comparison of the antitumor activity of gemcitabine and ara-C in a panel of human breast, colon, lung and pancreatic xenograft models. *Invest New Drugs,* 1996, vol. 14, 243-247
- **TEICHER BA ; CHEN V ; SHIH C ; MENON K ; FORLER PA ; PHARES VG ; AMSRUD T.** Treatment regimens including the multitargeted antifolate LY231514 in human tumor xenografts. *Clin Cancer Res,* 2000, vol. 6, 1016-1023
- **MIDDLETON MR ; THATCHER N ; MCMURRY TB ; MCELHINNEY RS ; DONNELLY DJ ; MARGISON GP.** Effect of O6-(4-bromothenyl)guanine on different temozolomide schedules in a human melanoma xenograft model. *Int J Cancer,* 2002, vol. 100, 615-617
- **LIU et al.** TH-302, a hypoxia-activated prodrug with broad in vivo preclinical combination therapy efficacy: optimization of dosing regimens and schedules. *Cancer Chemother Pharmacol,* 2012, vol. 69, 1487-1498
- **CHANG et al.** Sorafenib (BAY43-9006) inhibits tumor growth and vascularization and induces tumor apoptosis and hypoxia in RCC xenograft models. *Cancer Chemother Pharmacol,* 2007, vol. 59 (5), 561-74
- **CHANG et al.** Sorafenib(BAY43-9006) inhibits tumor growth and vascularization and induces tumor apoptosis and hypoxia in RCC xenograft models. *Cancer Chemother Pharmacol,* 2007, vol. 59 (5), 561-74
- **SU D ; STAMATAKIS L ; SINGER EA ; SRINIVASAN R.** Renal cell carcinoma: molecular biology and targeted therapy. *Curr Opin Oncol,* 2014, vol. 26, 321-7

- **SUN et al.** Combination treatment with hypoxia-activated prodrug evofosfamide (TH-302) and mTOR inhibitors results in enhanced antitumor efficacy in preclinical renal cell carcinoma models. *Am J Cancer Res.,* 2015, vol. 5, 2139
- **MENG et al.** Enhancement of hypoxia-activated prodrug TH-302 anti-tumor activity by Chk1 inhibition. *BMC Cance,* 2015, vol. 15, 422
- **WANG F Z ; FEI H R ; CUI, Y J et al.** The checkpoint 1 kinase inhibitor LY2603618 induces cell cycle arrest, DNA damage response and autophagy in cancer cells. *Apoptosis,* 2014, vol. 19, 1389-1398
- **BALSINA et al.** Breaching the DNA damage checkpoint via PF-00477736, a novel small-molecule inhibitor of checkpoint kinase. *Mol Cancer Ther,* 2008, vol. 7 (8), 2394-404
- **MORGAN et al.** Mechanism of radiosensitization by the Chk1/2 inhibitor AZD7762 involves abrogation of the G2 checkpoint and inhibition of homologous recombinational DNA repair. *Cancer Researh,* 2010, vol. 70, 4972
- **JAYAPRAKASH et al.** Targeted hypoxia reduction restores T cell infiltration and sensitizes prostate cancer to immunotherapy. *JCI,* 2018, vol. 128, 5137
- **CURRAN et al.** PD-1 and CTLA-4 combination blockade expands infiltrating T cells and reduces regulatory T and myeloid cells within B16 melanoma tumors. *Proc Natl Acad Sci USA.,* 2010, vol. 107 (9), 4275-4280
- **NOGRADY.** Medicinal Chemistry A Biochemical Approach. Oxford University Press, 1985, 388-392
- Molecular and Cellular Pharmacology of the Hypoxia-Activated Prodrug TH-302. *Mol Cancer Ther,* 2012, vol. 11, 740-751
- **CHAWLA S P ; CRANMER L D ; VAN TINE B A et al.** Phase II Study of the Safety and Antitumor Activity of the Hypoxia-Activated Prodrug TH-302 in Combination With Doxorubicin in Patients With Advanced Soft Tissue Sarcoma[J. *Journal of Clinical Oncology,* 2014, vol. 32 (29), 3299-3306
- **WEISS G J ; INFANTE J R ; CHIOREAN E G et al.** Phase 1 Study of the Safety, Tolerability, and Pharmacokinetics of TH-302, a Hypoxia-Activated Prodrug, in Patients with Advanced Solid Malignancies[J. *Clinical Cancer Research,* 2011, vol. 17 (9), 2997-3004
- **BORAD M J ; REDDY S G ; BAHARY N et al.** Randomized Phase II Trial of Gemcitabine Plus TH-302 Versus Gemcitabine in Patients With Advanced Pancreatic Cancer [J. *Journal of Clinical Oncology,* 2015, vol. 33 (13), 1475-1481
- **BRENNER A ; REARDON D A ; WEN P Y et al.** ACTR-17. Evophosphamide (th-302) for recurrent gbm following bevacizumab failure, final results of a multicenter phase ii study [J]. *Neuro-oncology,* 2018
- **THOMPSON LH et al.** Repair of DNA adducts in asynchronous CHO cells and the role of repair in cell killing and mutation induction in synchronous cells treated with 7-bromomethylbenz[a]anthracene. *Somatic Cell Mol. Genet.,* 1984, vol. 10, 183-194
- **THOMPSON LH et al.** Genetic diversity of UV-sensitive DNA repair mutants of Chinese hamster ovary cells. *Proc. Natl. Acad. Sci. USA,* 1981, vol. 78, 3734-3737
- **HOY CA et al.** Defective DNA cross-link removal in Chinese hamster cell mutants hypersensitive to bifunctional alkylating agents. *Cancer Res,* 1985, vol. 45, 1737-1743
- **BUSCH D et al.** Summary of complementation groups of UV-sensitive CHO cell mutants isolated by large-scale screening. *Mutagenesis,* 1989, vol. 4, 349-354
- **BESSHO T et al.** Initiation of DNA interstrand cross-link repair in humans: the nucleotide excision repair system makes dual incisions 5'' to the cross-linked base and removes a 22- to 28-nucleotide-long damage-free strand. *Mol. Cell. Biol.,* 1997, vol. 17, 6822-6830
- **THOMPSON LH et al.** Hypersensitivity to mutation and sister-chromatid-exchange induction in CHO cell mutants defective in incising DNA containing UV lesions. *Somatic Cell Genet,* 1982, vol. 8, 759-773
- **JESSICA D. SUN ; QIAN LIU ; DHARMENDRA AHLUWALIA ; DAMIEN J. FERRARO ; YAN WANG ; DON JUNG ; MARK D. MATTEUCCI ; CHARLES P. HART.** Comparison of hypoxia-activated prodrug evofosfamide (TH-302) and ifosfamide in preclinical non-small cell lung cancer models [J. *Cancer Biology & Therapy,* 2016, vol. 17 (4), 371-380